# EUROPEAN PATENT APPLICATION

(11) **EP 4 537 854 A1**
(43) Date of publication of application: **16.04.2025**
(21) Application number: 24203965.9
(22) Date of filing: 01.10.2024
(51) Int. Cl.: A61K 47/68, A61P 35/00

(54) **COMPOUND FOR CONJUGATION, CONJUGATE AND PHARMACEUTICAL COMPOSITION COMPRISING THE SAME**

(30) Priority: 04.10.2023 US 202363587776 P
(71) Applicant: Formosa Laboratories, Inc., Taoyuan City 338002 (TW)
(72) Inventor: Chen, ChihHau, 338002 Taoyuan City (TW); Lin, WunHuei, 338002 Taoyuan City (TW); Hsieh, HaoYu, 338002 Taoyuan City (TW); Zhao, JianXun, 338002 Taoyuan City (TW); Hsu, HungYi, 338002 Taoyuan City (TW); Su, ShihHsun, 338002 Taoyuan City (TW); Tsai, ShuoEn, 338002 Taoyuan City (TW); Li, Yi-Shan, 338002 Taoyuan City (TW); Liao, TzuHan, 338002 Taoyuan City (TW); Wu, ChienHsun, 338002 Taoyuan City (TW); Huang, Pohui, 338002 Taoyuan City (TW); Juo, Bao Rong, 338002 Taoyuan City (TW); Juang, Yu-Min, 38002 Taoyuan City (TW); Li, Chao-Yi, 338002 Taoyuan City (TW); Chou, Yi-Shiuan, 338002 Taoyuan City (TW); Chung, Cheng-Yu, 338002 Taoyuan City (TW)
(74) Representative: Charrier Rapp & Liebau

(57) **Abstract**

Disclosed are antibody-drug conjugates, wherein the drug is, in particular, eribulin. Disclosed is a compound with one or more arm(s) for drug conjugation and a conjugate including the compound so as to provide high drug-to-moiety ratio and conjugate more drugs to the conjugate.

## Description

### CROSS-REFERENCE TO RELATED APPLICATIONS

The present disclosure claims priority to U.S. Provisional Application Serial No. 63/587,776 filed on October 04, 2023, the entirety of which is hereby incorporated by reference herein for all purposes.

### FIELD OF THE INVENTION

The present disclosure is directed to compounds for use in drug conjugation. Also described herein are conjugates with high drug-to-antibody ratio (DAR).

### BACKGROUND OF THE INVENTION

The use of antibody-drug conjugates (ADCs) for the local delivery of drugs, such as cytotoxic or cytostatic agents for killing or inhibiting tumor cells in the treatment of cancer, theoretically allows antibody-targeted delivery of the drug to tumors, and intracellular accumulation therein, while systemic administration of these unconjugated drugs may result in unacceptable levels of toxicity to normal cells as well as the tumor cells sought to be eliminated. Maximal efficacy with minimal toxicity is sought thereby.

A linker-drug combination is generally conjugated to the moieties, e.g., antibody, via the cysteine residues or lysine residues on the moieties. For example, eight out of thirteen FDA approved antibody-drug conjugates (ADCs) are conjugated via cysteine residues on the antibody, including Adcetris, Polivy, Padcev, Enhertu, Trodelvy, Blenrep , Zynlonta and Tivdak; and the rest ADCs are conjugated via lysine residues on the antibody, including Kadcyla, Mylotarg, Besponsa, Lumoxiti and Elahere. Normally, the ADCs conjugated via cysteine residues provide more homogeneous drug-to-antibody ratio (DAR), compared to those conjugated via lysine residues. Therefore, constructing the ADCs via cysteine residues is currently the main method for synthesizing the ADCs.

### SUMMARY OF THE INVENTION

Accordingly, to overcome the DAR-limitations mentioned above, the present disclosure provides a linker modified with one or more arm(s) so as to conjugate more drugs, thereby increasing the DAR and the activity of the conjugate described herein. In one embodiment, the present disclosure provides a linker with 1, 2, 3, 4, 5 or 6 arm(s).

In another aspect, as most of drugs are lipophilic, more drugs conjugated to linkers make the water solubility of the final linker-drug combination even lower. In order to increase the water solubility of the linker-drug combination, the present disclosure further provides hydrophilic spacer and/or hydrophilic chain to the compound and the conjugate described herein, thereby improving the characteristics of the compound and the conjugate described herein.

In one aspect, the present disclosure features a compound as represented by Formula (I), 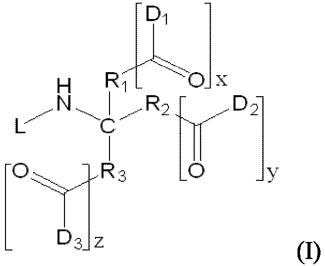 wherein,
L is a linker;
each of D₁, D₂, and D₃ independently is a drug;
each of x, y, and z independently is 0 or 1, with a provision that at least one of x, y, and z is 1;
each of R₁, R₂, and R₃ independently is a bond, hydrogen, 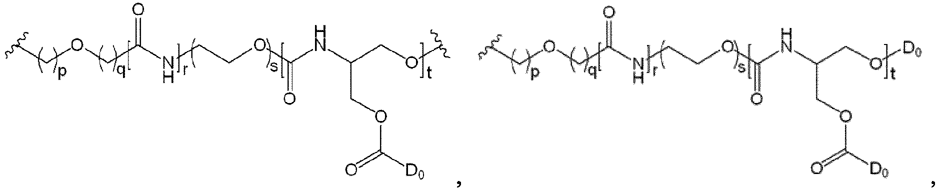 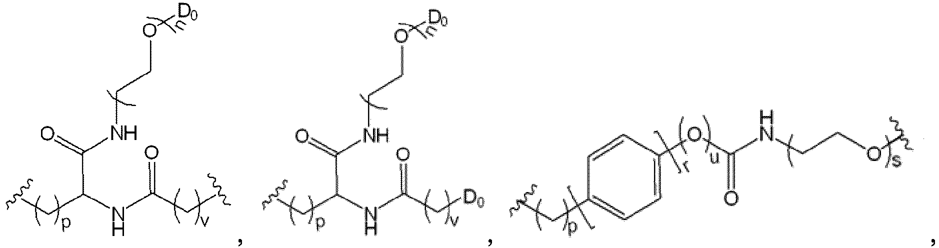 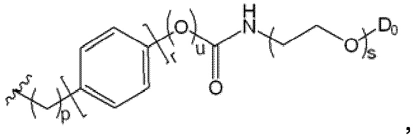 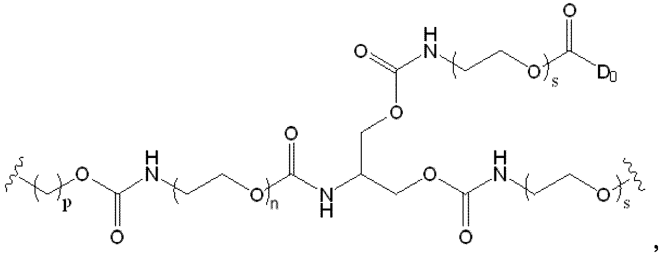 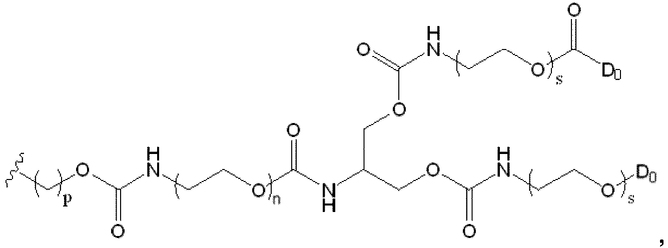 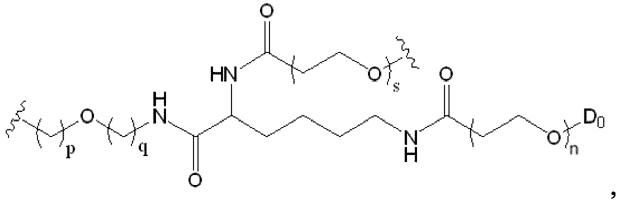 or 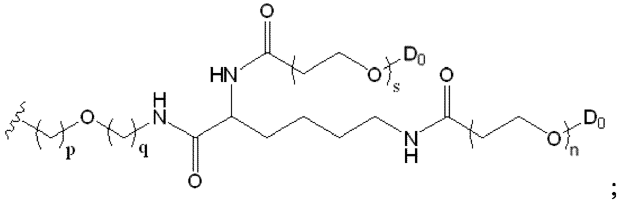 wherein p is 1, 2, or 3; q is 0, 1, or 2; r is 0 or 1; s is an integer between 0 and 37; t is 0 or 1; u is 0 or 1; v is 1, 2, or 3; and n is an integer between 1 and 37;or
x and y are 0 and R₁ and R₂ are together to form =O and R₃ is 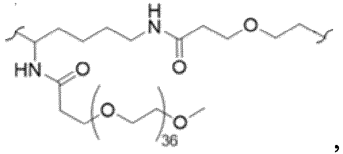
D₀ is methyl, propargyl, or a drug;
with a provision that at least one of R₁, R₂, and R₃ is not hydrogen;
if q, r, s, and t are 0, x+y+z>1;
if x is 0, R₁ is hydrogen, 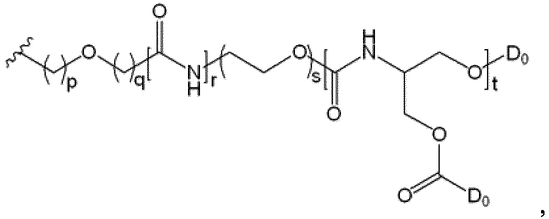 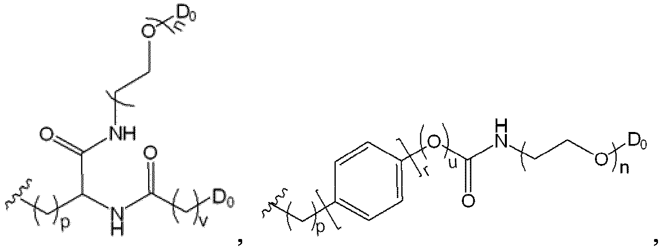 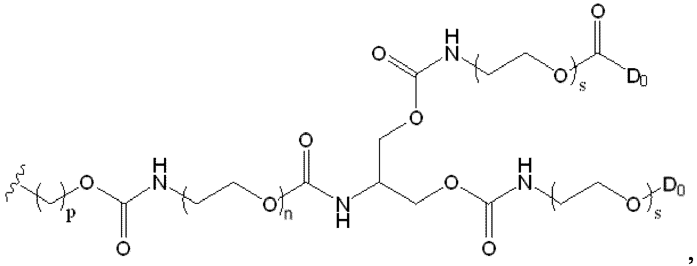 or 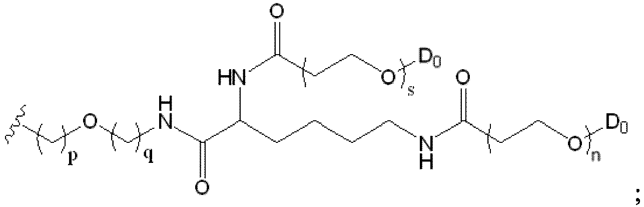
if y is 0, R₂ is hydrogen, 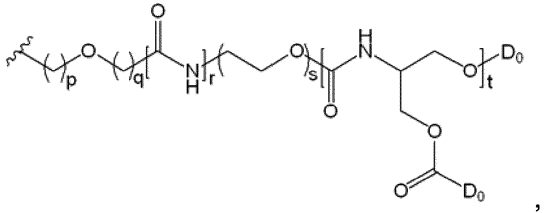 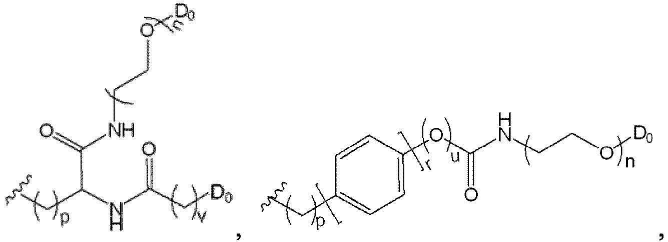 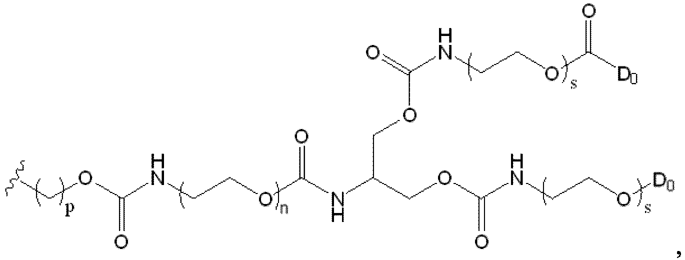 or 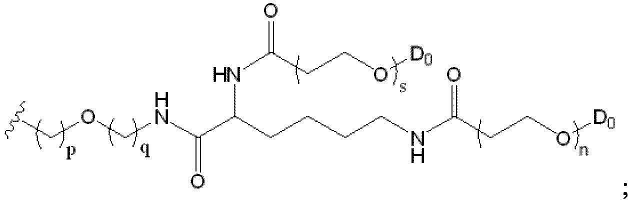
if z is 0, R₃ is hydrogen 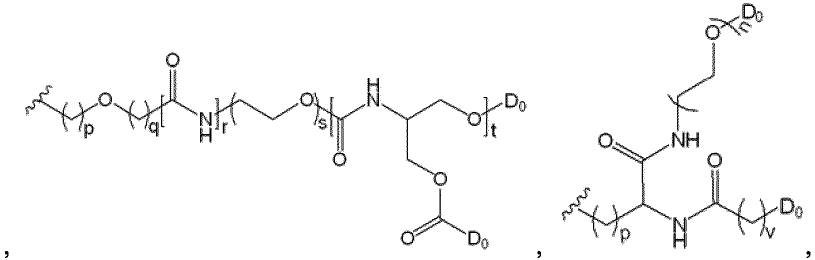 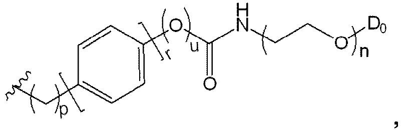 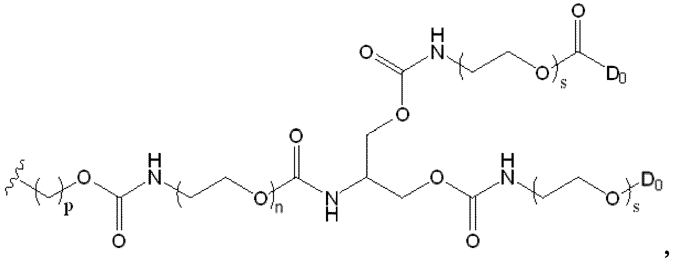 or 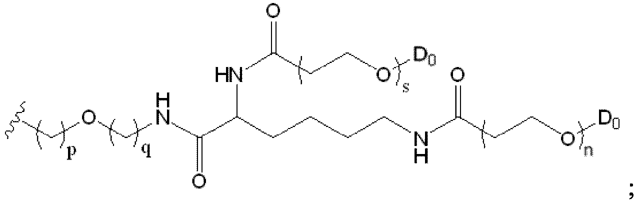
if x+y+z=1 and each of R₁, R₂, and R₃ independently is 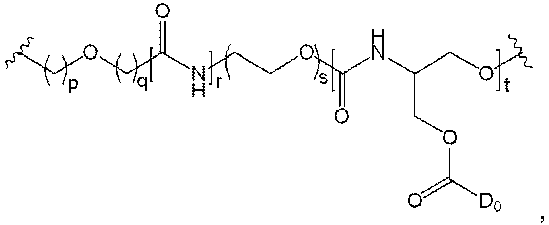 p is not 1;
if r, s, and t are 0 and each of R₁, R₂, and R₃ independently is 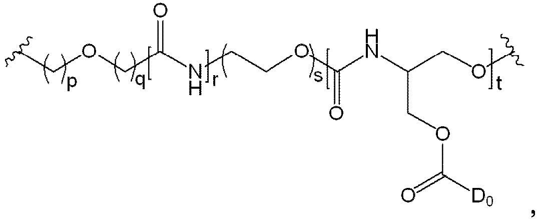 x+y+z>1.

In certain embodiments, L is selected from the group consisting of 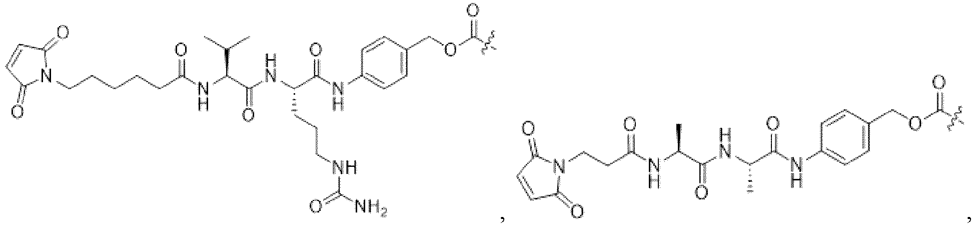 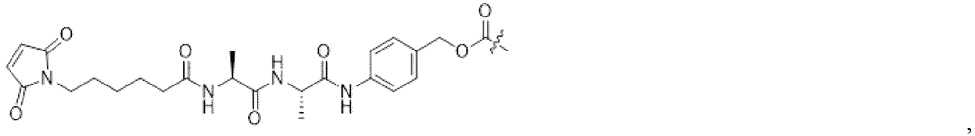 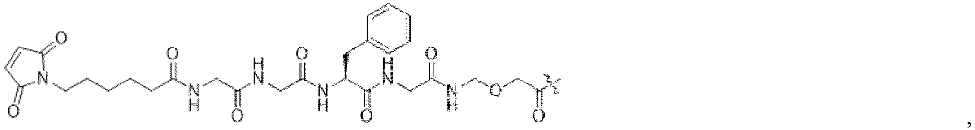 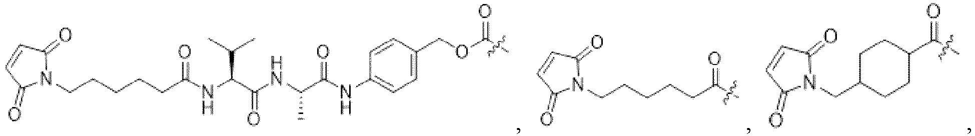 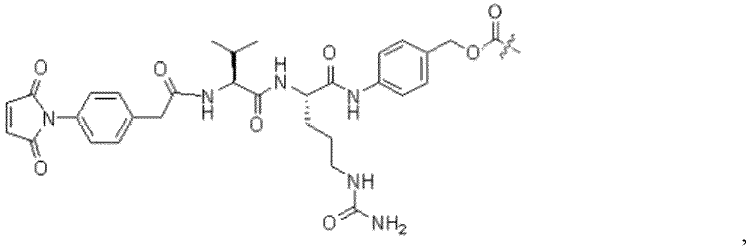 and

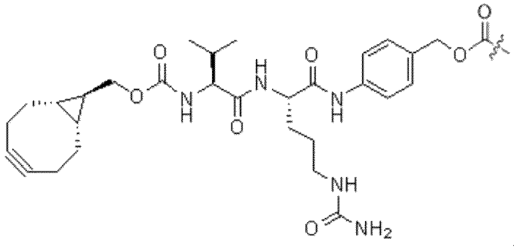

In another embodiments, n is any number between 1 and 37, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 or 37.

In certain embodiments, each of R₁, R₂, and R₃ independently is a bond, hydrogen, 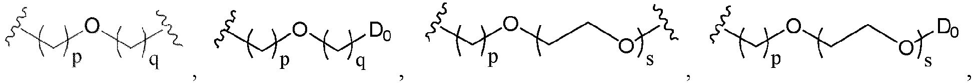 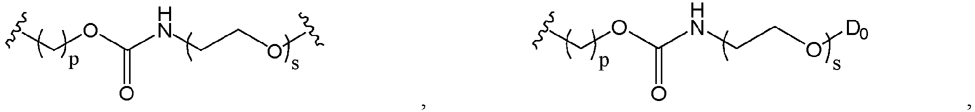 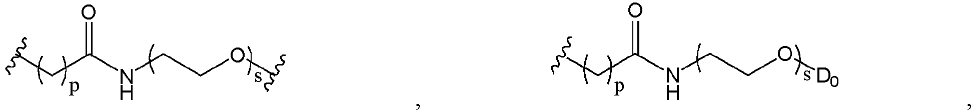 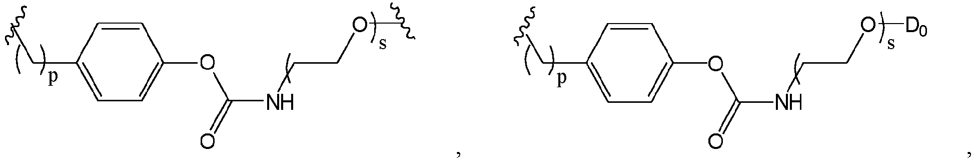 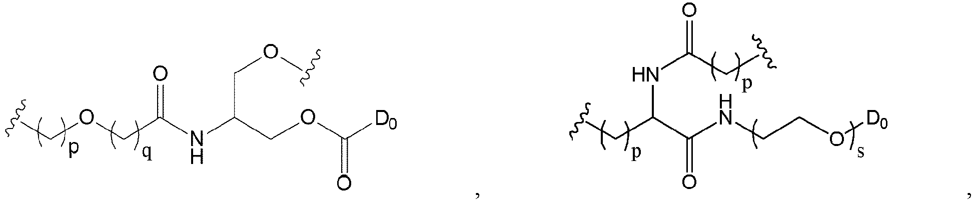 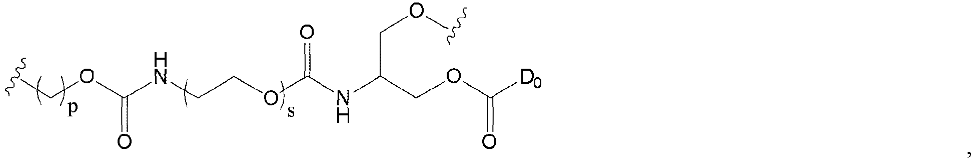 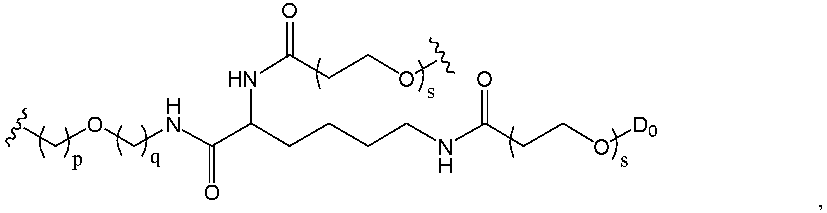 or

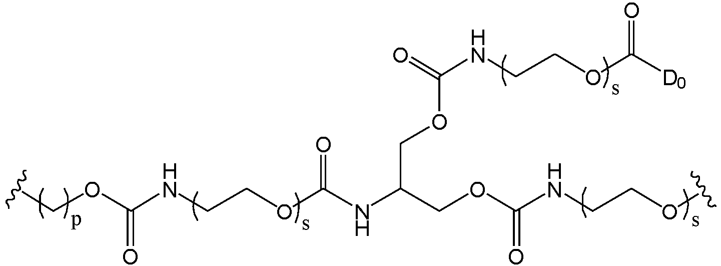

In certain embodiments, x+y+z=1. Both of R₂ and R₃ are H and R₁ is 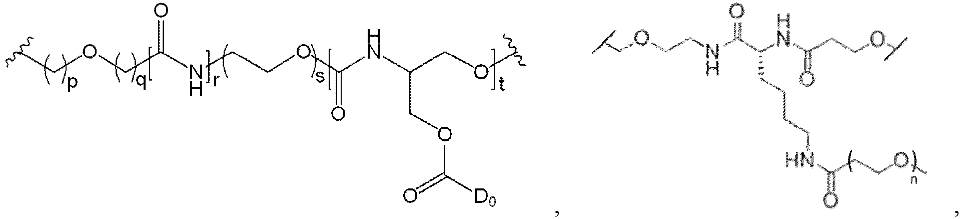 or

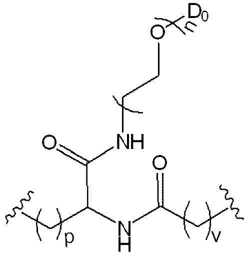

In certain embodiments, each of D₁, D₂, and D₃ independently is Monomethyl auristatin E (MMAE), Monomethyl auristatin F (MMAF), Exatecan, or Eribulin.

In certain embodiments, x+y+z=1 and R₁ is a bond; R₂ is 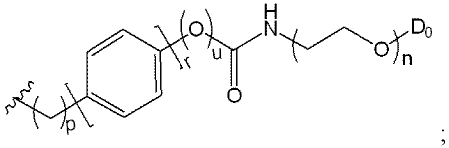 and R₃ is H.

In certain embodiments, x+y+z>1. In one embodiment, x+y+z=2; both of R₁ and R₂ are 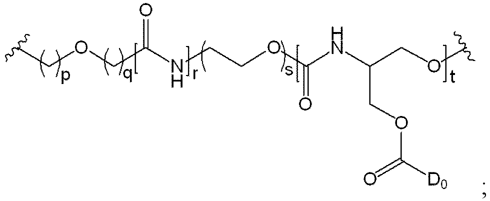 and R₃ is H. In another embodiment, x+y+z=3 and all of R₁, R₂, and R₃ are

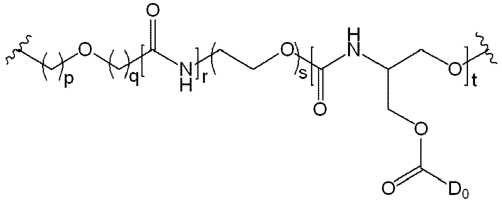

In certain embodiments, the compound is selected from the group consisting of: 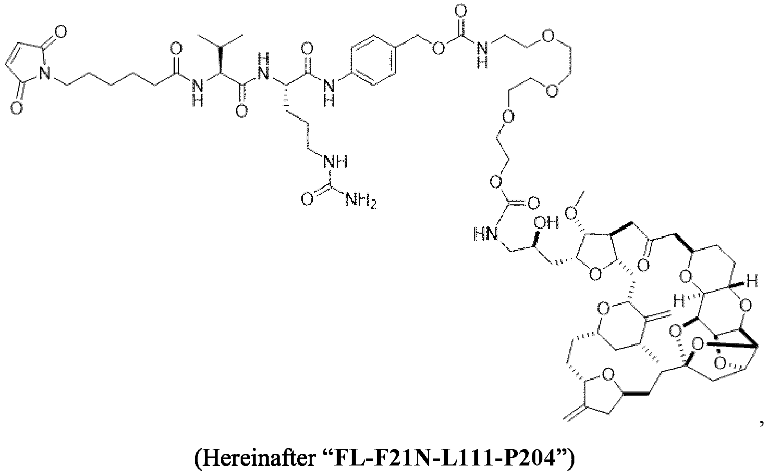 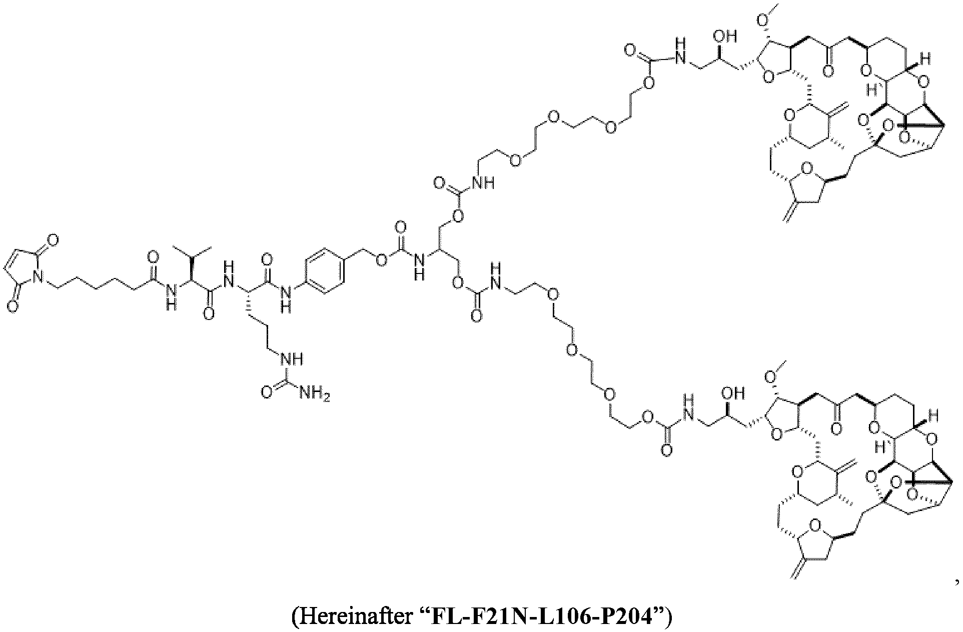 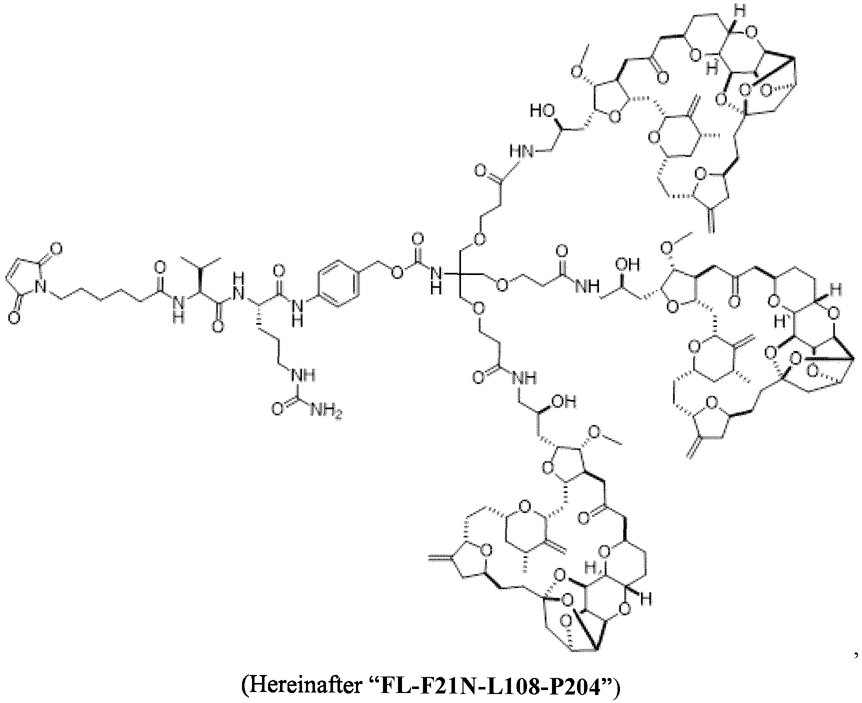 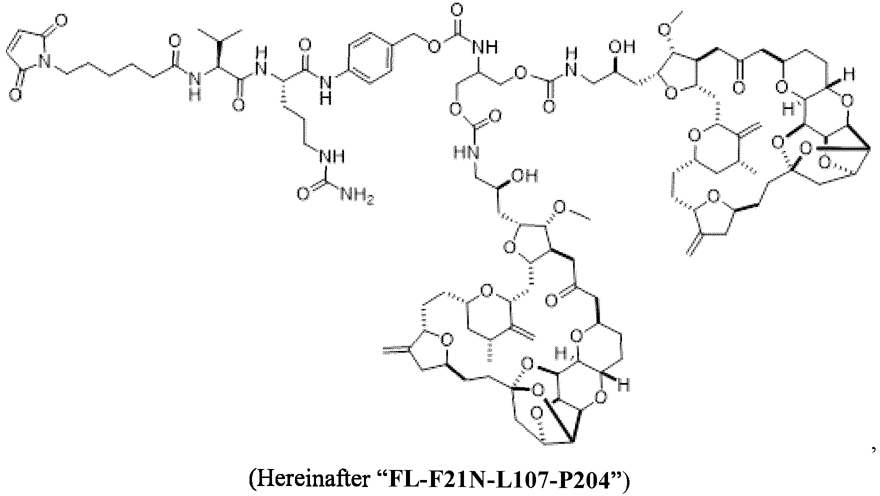 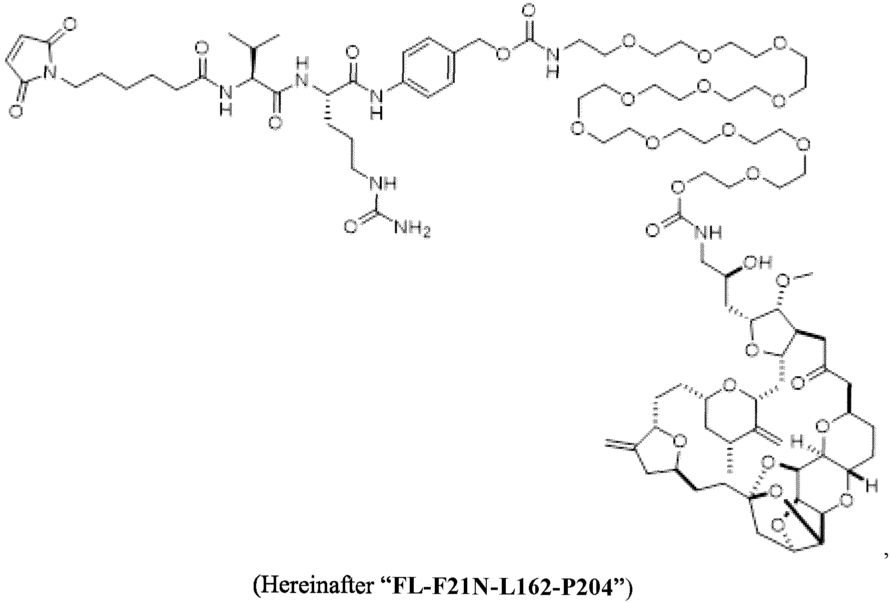 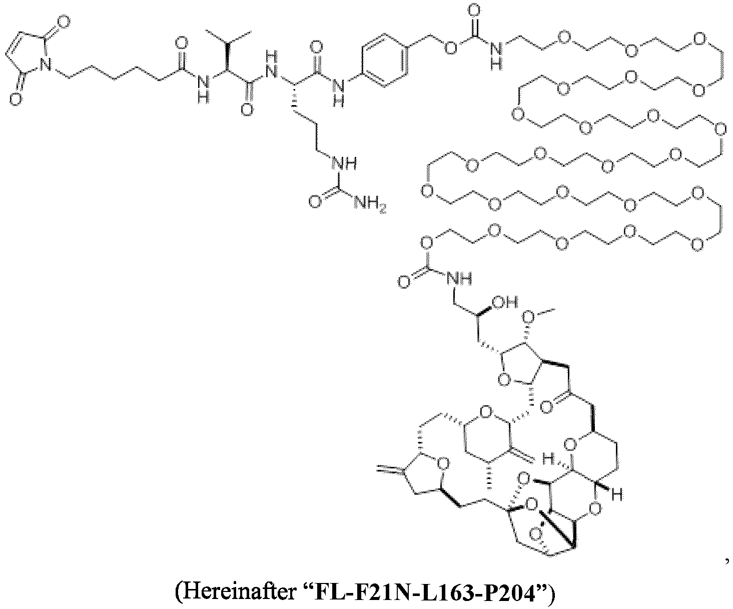 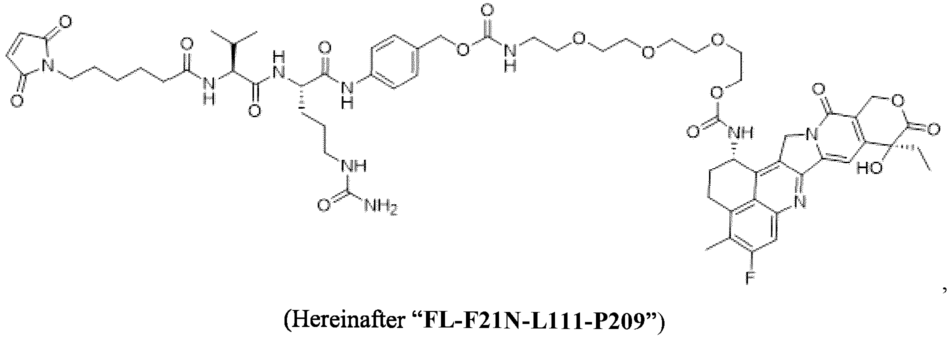 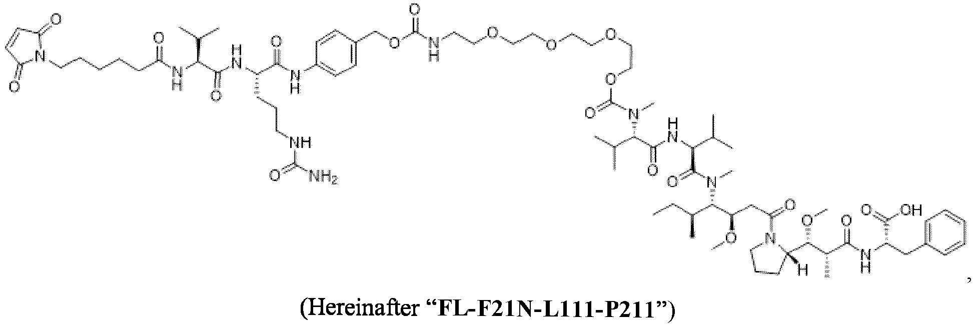 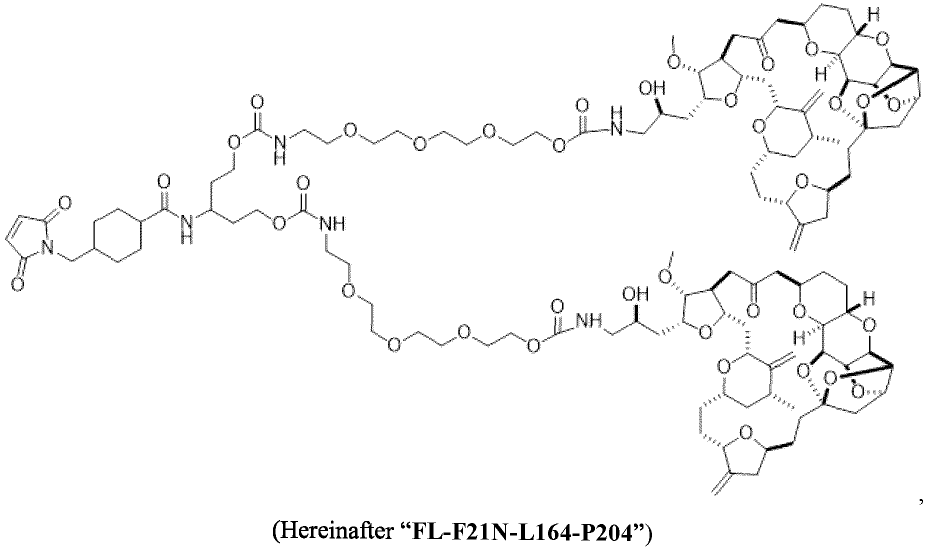 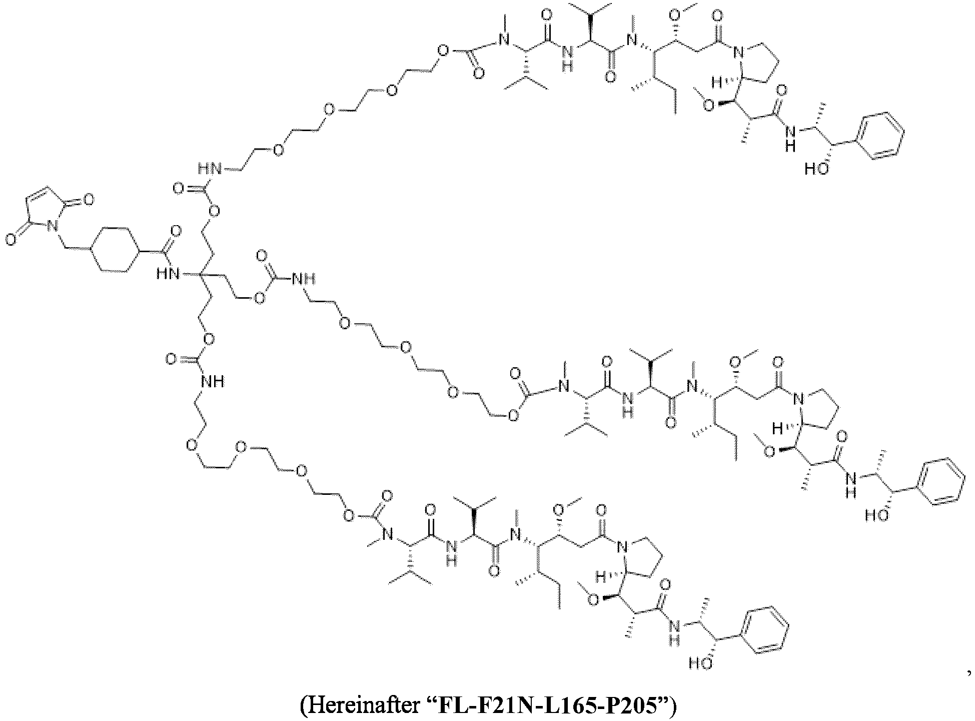 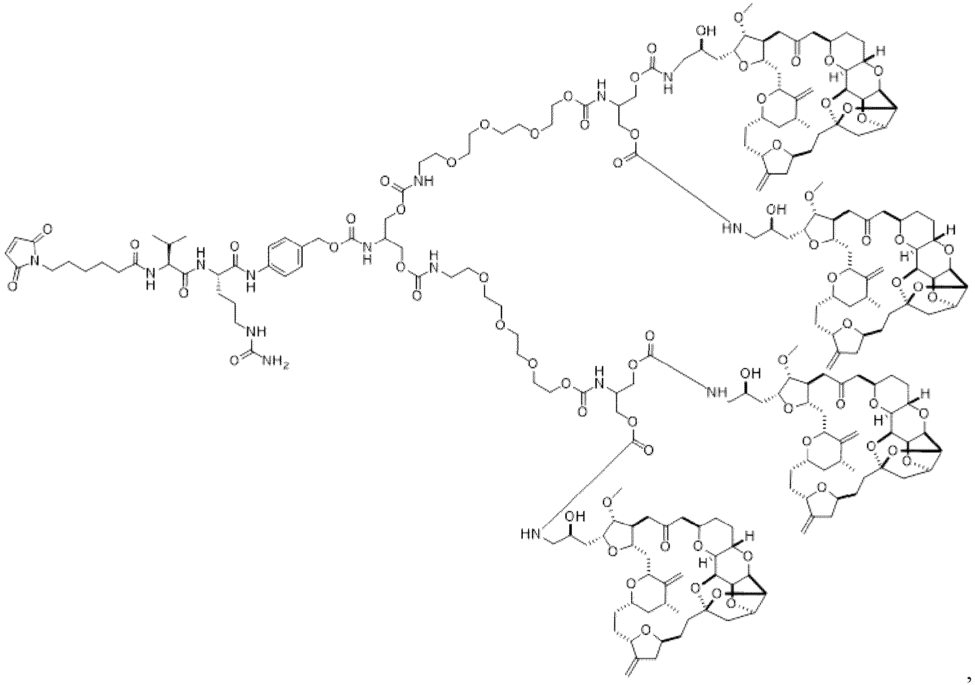 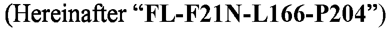 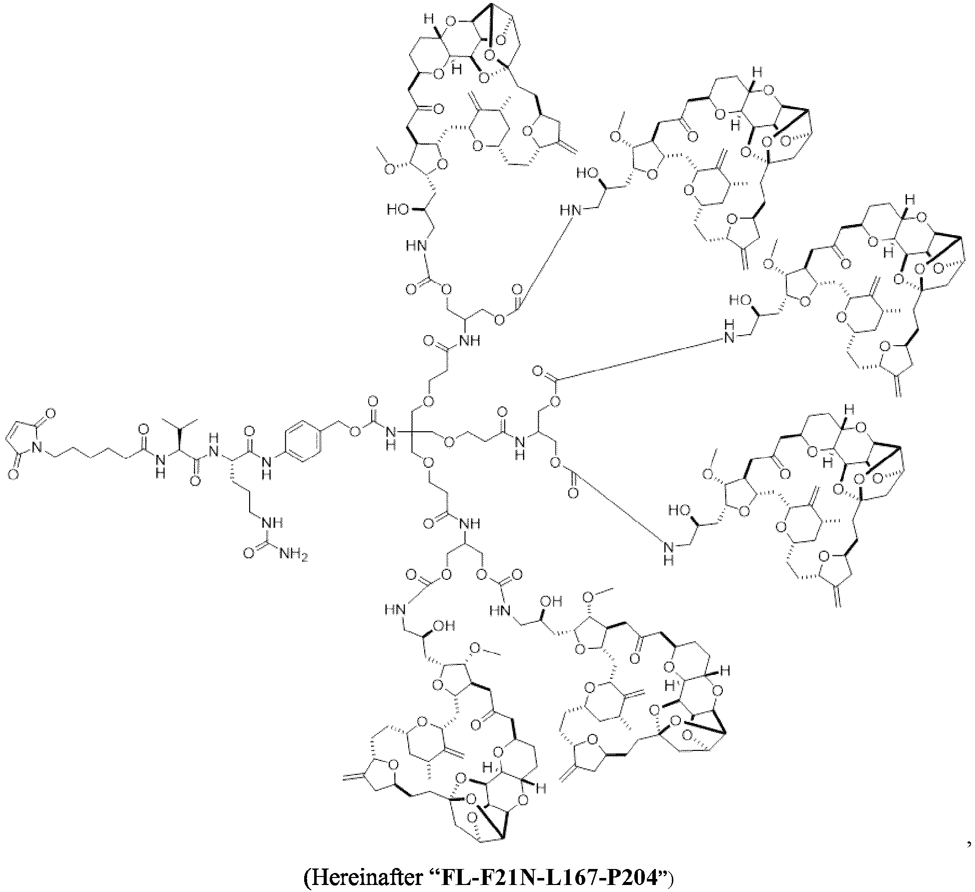 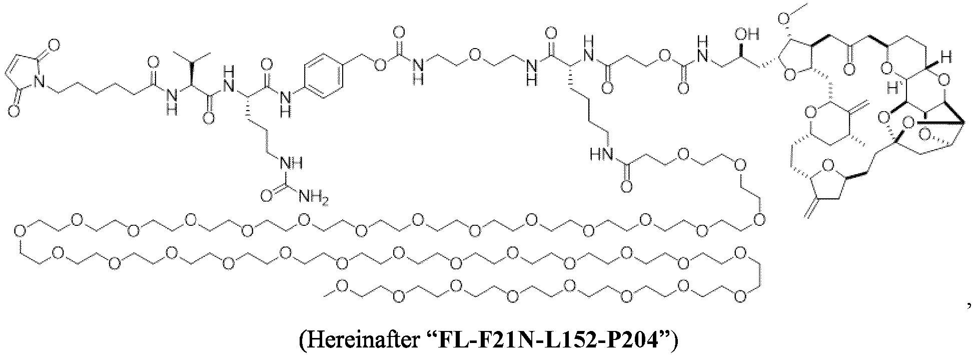 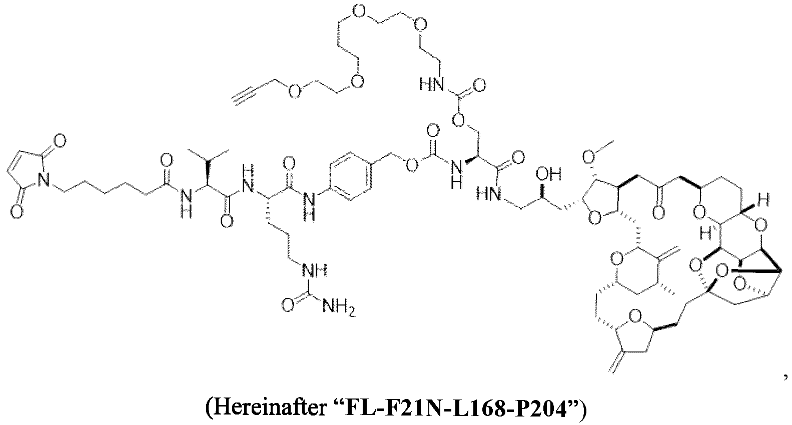 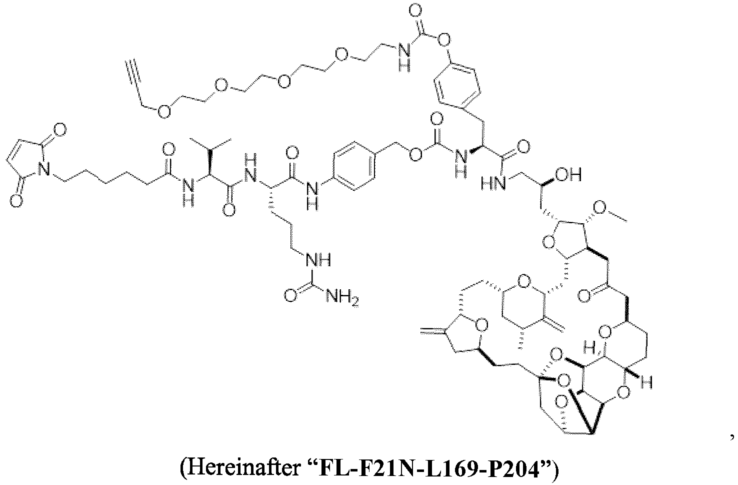 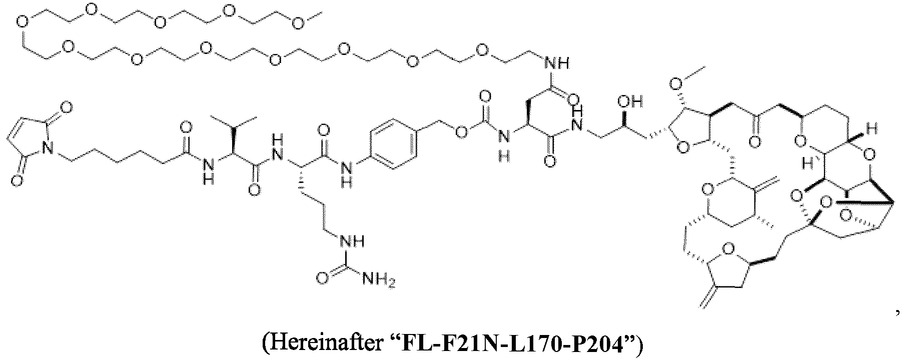 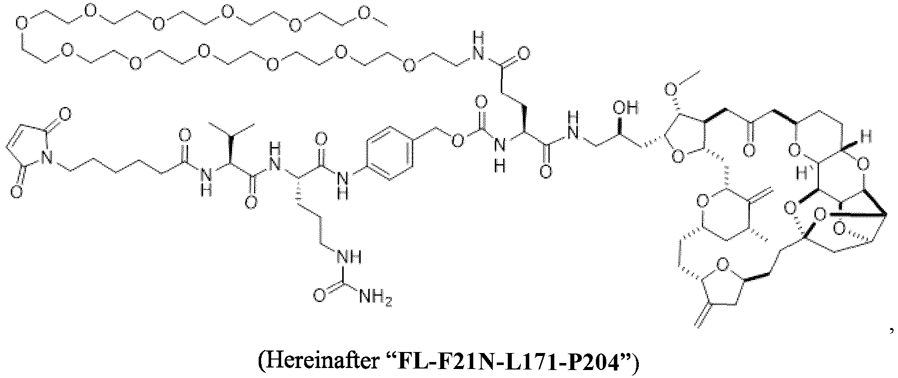 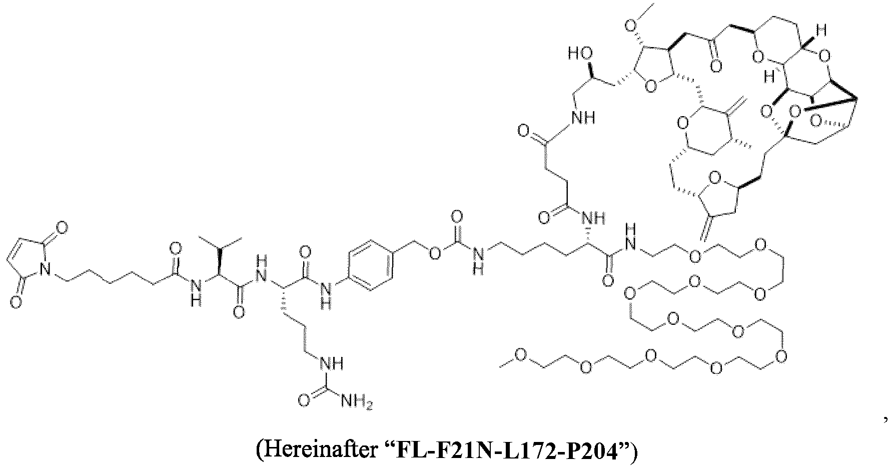 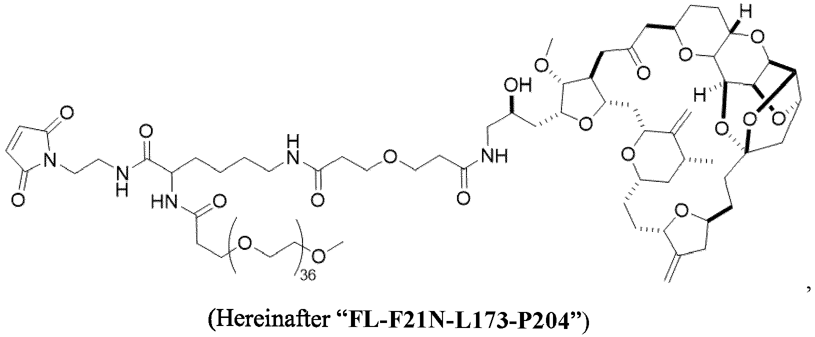 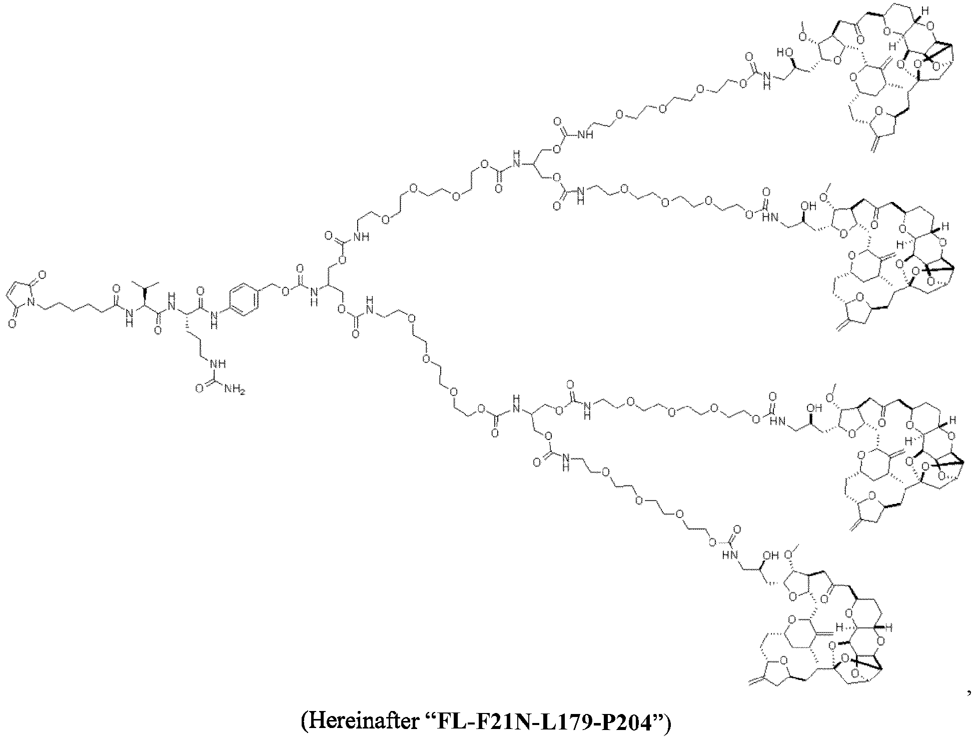 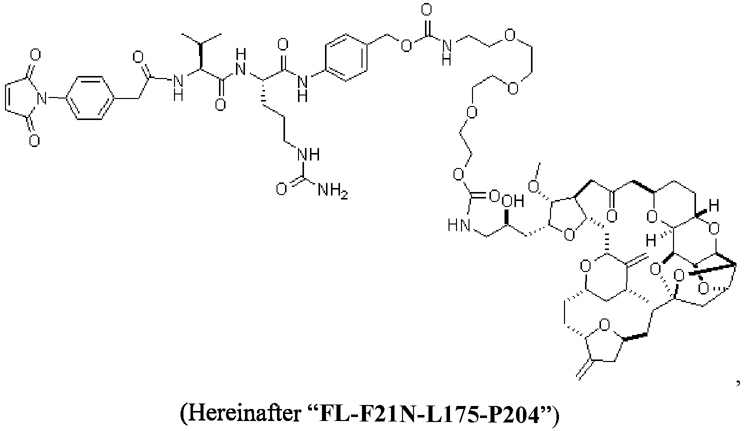 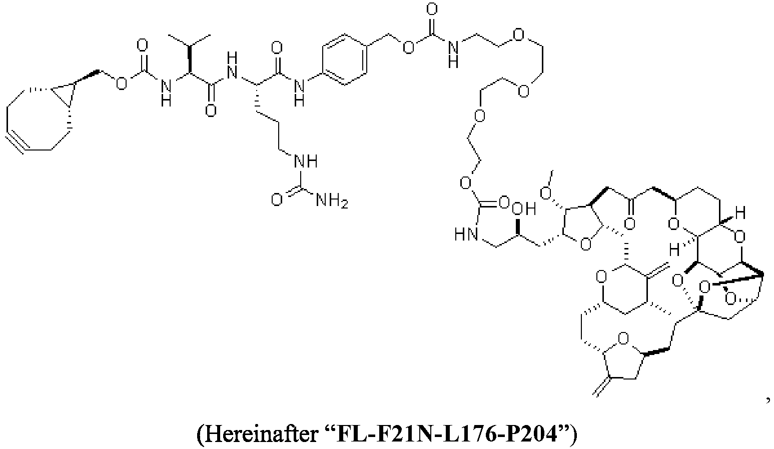 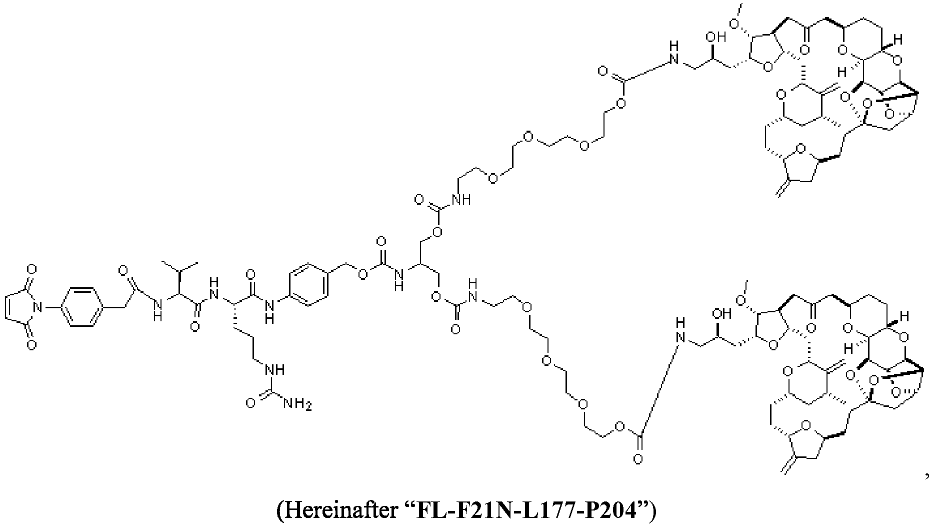 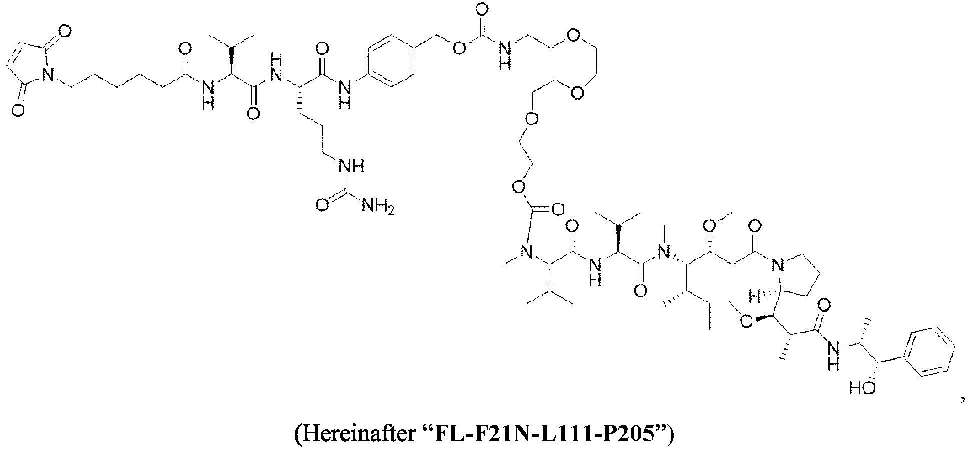 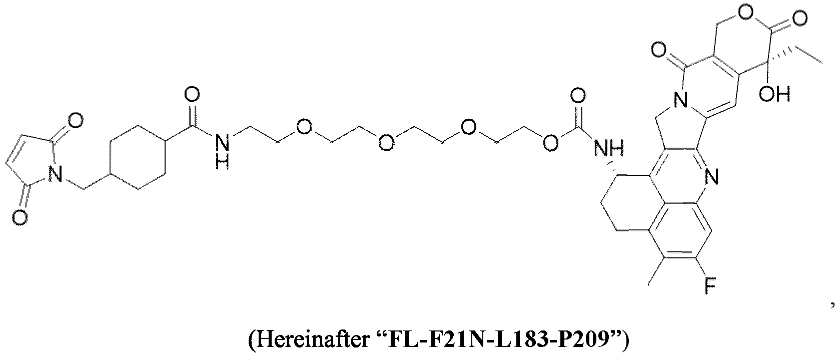 and

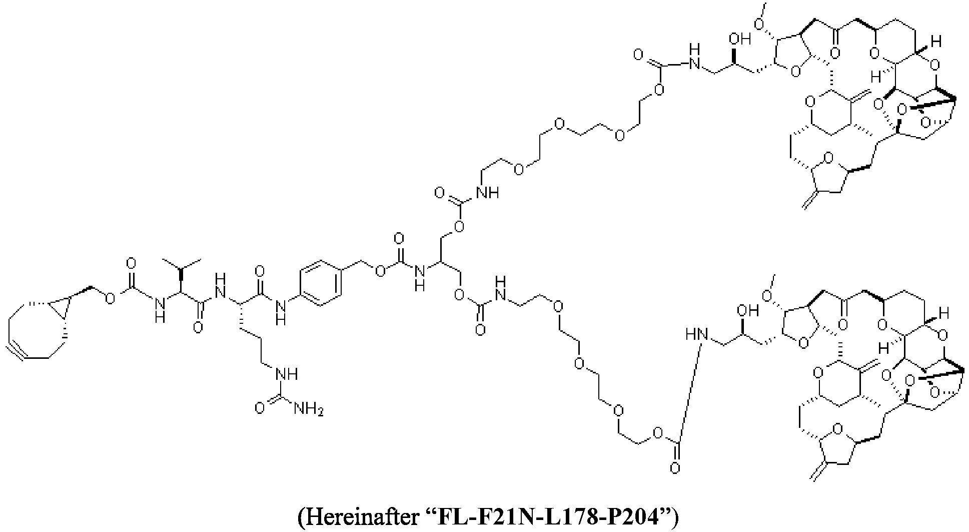

In one aspect, the present disclosure further provides a conjugate, comprising a moiety conjugated via a linker modified with one or more arms to a drug such as a cytotoxic agent, a chemotherapeutic agent, a growth inhibitory agent, a toxin (e.g., an enzymatically active toxin of bacterial, fungal, plant, or animal origin, or fragments thereof), or a radioactive isotope (i.e., a radio conjugate).

In one aspect, the present disclosure further provides a conjugate comprising as represented by Formula (II), 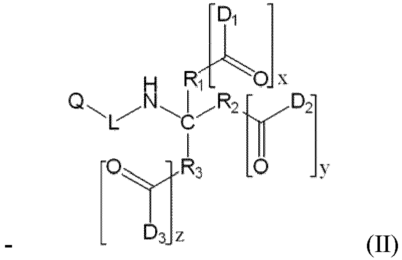 wherein
**Q** is a moiety selected from the group consisting of a peptide, a polypeptide and a protein that specifically binds to an antigen;
**L, D₁, D₂, D₃,** x, y, z, R₁, R₂, and R₃ are as defined above.

In certain embodiments, **L** is conjugated to **Q** via cysteine residues on **Q.**

In certain embodiments, **Q** is an antibody selected from a monoclonal antibody, an antigen-binding fragment, a chimeric antibody, and a humanized antibody. In another embodiments, the antigen-binding fragment is an Fab, F(ab')₂, Fv or scFv fragment.

In one aspect, the present disclosure provides a pharmaceutical composition comprising the conjugate described herein, or a pharmaceutically acceptable salt thereof; and a pharmaceutically acceptable diluent, carrier or excipient.

The details of one or more embodiments of the invention are set forth in the description below. Other features or advantages of the present invention will be apparent from the following drawings and detailed description of several embodiments, and also from the appending claims.

### BRIEF DESCRIPTION OF THE FIGURES

A more complete understanding of the invention may be obtained by reference to the accompanying drawings, when considered in conjunction with the subsequent detailed description. The embodiments illustrated in the drawings are intended only to exemplify the invention and should not be construed as limiting the invention to the illustrated embodiments.

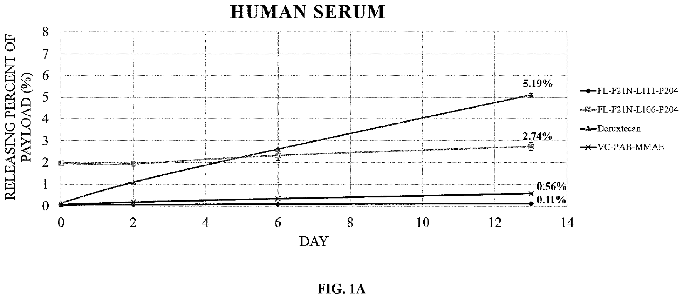

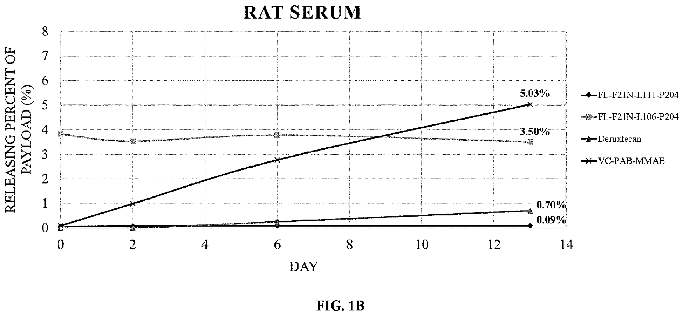

FIGs. 1A and 1B respectively illustrate the human and rat plasma stabilities of the compounds according to an embodiment of the present disclosure.

### DETAILED DESCRIPTION OF THE INVENTION

Accordingly, conjugates and compositions directed to the markers for use in diagnosing and treating a broad spectrum of cancers are provided. A conjugate comprising a drug conjugated to a moiety, e.g., an antibody or an antigen-binding fragment that binds to specific antigens, was developed and disclosed herein.

### General Definitions

In the present description, any concentration range, percentage range, ratio range, or integer range is to be understood to include the value of any integer within the recited range and, when appropriate, fractions thereof (such as one tenth and one hundredth of an integer), irrespective of whether a specific numerical value or specific sub-range is expressly stated. Also, any number range recited in the disclosure relating to any physical feature, such as polymer subunits, size, or thickness, are to be understood to include any integer within the recited range, unless otherwise indicated.

As used herein, the term "moiety" is defined as a structure that has a selective affinity for or selectively binds to a target molecule relative to other non-target molecules. A moiety may include, for example, an antibody, an antibody construct, a peptide, a polypeptide, a ligand, carbohydrate, a polynucleotide, an oligonucleotide, or a receptor or a binding portion thereof. The target molecule may be a biological receptor or other structure of a cell, such as a tumor antigen.

As used throughout this disclosure, "conjugate" means a linker-drug combination conjugated to a moiety.

As used throughout this disclosure, "linker-drug combination" means a linker group conjugated with a drug, i.e. the compound of the present application.

The term "monoclonal antibody" as used herein refers to an antibody obtained from a population of substantially homogeneous antibodies, i.e., the individual antibodies comprising the population are identical except for possible naturally occurring mutations that may be present in small amounts. Thus, the modifier "monoclonal" indicates the character of the antibody as not being a mixture of discrete antibodies. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity in vivo, to create a multi-specific antibody, etc., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparation is directed against a single determinant on an antigen. In addition to their specificity, the monoclonal antibody preparations are advantageous in that they are typically uncontaminated by other immunoglobulins.

Antibodies of the present invention also include chimerized or humanized monoclonal antibodies generated from antibodies of the present invention.

The antibodies can be full-length or can comprise a fragment (or fragments) of the antibody having an antigen-binding portion, including, but not limited to, Fab, F(ab')₂, Fab', F(ab)', Fv, single chain Fv (scFv), bivalent scFv (bi-scFv), trivalent scFv (tri-scFv), Fd, dAb fragment (e.g., Nature, 341 :544-546 (1989)), an CDR, diabodies, triabodies, tetrabodies, linear antibodies, single-chain antibody molecules, and multi-specific antibodies formed from antibody fragments. Single chain antibodies produced by joining antibody fragments using recombinant methods, or a synthetic linker, are also encompassed by the present invention. Science, 1988, 242:423-426. Proc. Natl. Acad. Sci. USA, 1988, 85:5879-5883.

The antibodies or antigen-binding portions thereof of the present invention may be monospecific, bi-specific or multi-specific.

Antibodies with a variable heavy chain region and a variable light chain region that are at least about 70%, at least about 75%, at least about 80%, at least about 81%, at least about 82%, at least about 83%, at least about 84%, at least about 85%, at least about 86%o, at least about 87%>, at least about 88%>, at least about 89%>, at least about 90%>, at least about 91 >, at least about 92%>, at least about 93%>, at least about 94%>, at least about 95%), at least about 96%>, at least about 97%>, at least about 98%>, at least about 99%> or about 100% homologous to the variable heavy chain region and variable light chain region of the antibody produced by the reference antibody, and can also bind to antigens. Homology can be present at either the amino acid or nucleotide sequence level.

The term "cytotoxic agent" as used herein refers to a substance that inhibits or prevents the function of cells and/or causes destruction of cells. The term is intended to include a chemical compound useful in the treatment of cancer. Examples of cytotoxic agents include Exatecan, Eribulin, Monomethyl auristatin E (MMAE), Monomethyl auristatin F (MMAF), mertansine (DM1), ravtansine (DM4), 7-ethyl-10-hydroxycamptothecin (SN-38), anthracycline, pyrrolobenzodiazepine, α-amanitin, tubulysin, benzodiazepine, erlotinib (TARCEVA^{®}, Genentech/OSI Pharm.), bortezomib (VELCADE^{®}, Millenium Pharm.), fulvestrant (FASLODEX^{®}, Astrazeneca), sunitinib (SUTENT^{®}, SU11248, Pfizer), letrozole (FEMARA^{®}, Novartis), imatinib mesylate (GLEEVEC^{®}, Novartis), PTK787/ZK 222584 (Novartis), oxaliplatin (ELOXATIN^{®}, Sanofi), leucovorin, rapamycin (Sirolimus, RAPAMUNE^{®}, Wyeth), lapatinib (TYKERB^{®}, GSK572016, GlaxoSmithKline), lonafarnib (SARASAR^{®}, SCH 66336), sorafenib (NEXAVAR^{®}, BAY43-9006, Bayer Labs.), and gefitinib (IRESSA^{®}, Astrazeneca), AG1478, AG1571 (SU 5271; Sugen), alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimustine; antibiotics such as the enediyne antibiotics (e.g., calicheamicin, especially calicheamicin gamma1I and calicheamicin omegaI1 (Angew Chem. Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} doxorubicin (including morpholinodoxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verrucarin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol-Myers Squibb Oncology, Princeton, N.J.), ABRAXANE^{™} Cremophor-free, albumin-engineered nanoparticle formulation of paclitaxel (American Pharmaceutical Partners, Schaumberg, Ill.), and TAXOTERE^{®} docetaxel (Rhône-Poulenc Rorer, Antony, France); chlorambucil; GEMZAR^{®} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeloda; ibandronate; CPT-11; topoisomerase inhibitor RFS 2000; difluoromethylomithine (DMFO); retinoids such as retinoic acid; capecitabine (XELODA^{®}, Roche); and pharmaceutically acceptable salts, acids or derivatives of any of the above.

The phrase "pharmaceutically acceptable salt," as used herein, refers to pharmaceutically acceptable organic or inorganic salts of an ADC. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counterions. The counterions may be any organic or inorganic ions that stabilize the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

In an embodiment, the compound may be represented by Formula (I), 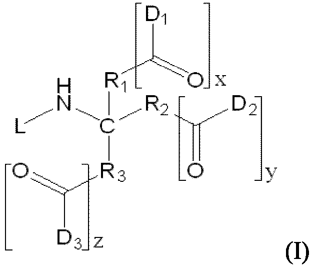 wherein,
L is a linker;
each of D₁, D₂, and D₃ independently is a drug;
each of x, y, and z independently is 0 or 1, with a provision that at least one of x, y, and z is 1;
each of R₁, R₂, and R₃ independently is a bond, hydrogen, 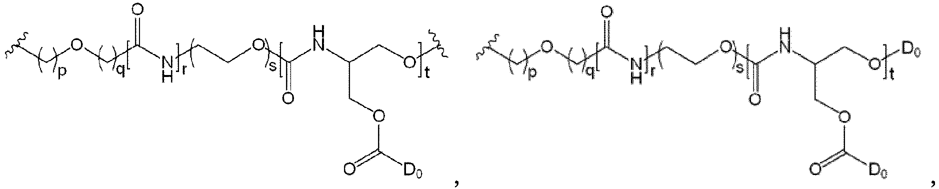 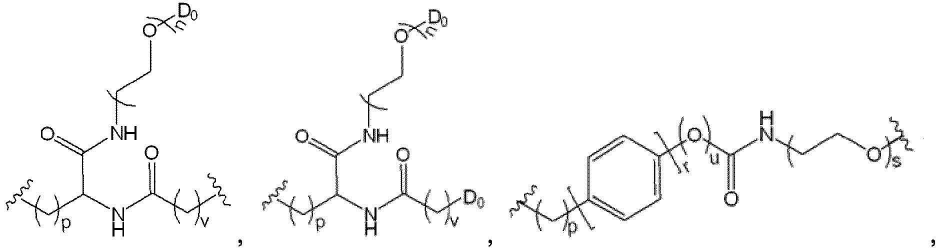 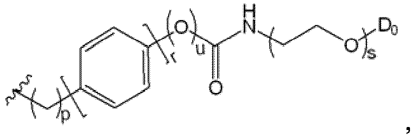 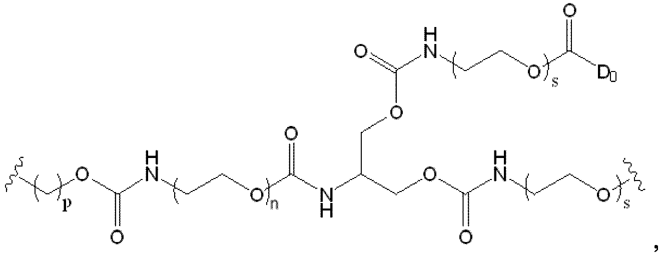 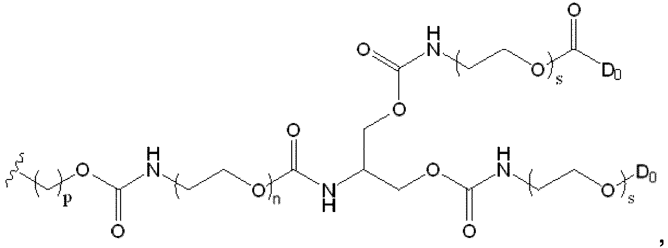 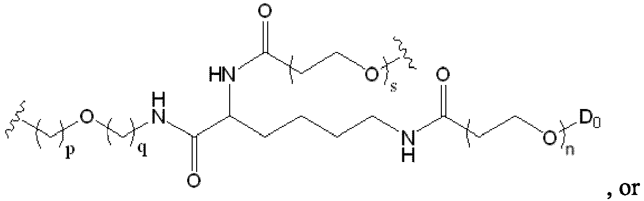 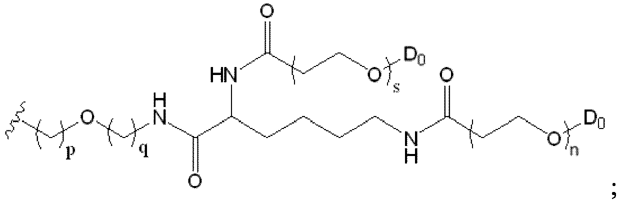 wherein p is 1, 2, or 3; q is 0, 1, or 2;
r is 0 or 1; s is an integer between 0 and 37; t is 0 or 1; u is 0 or 1; v is 1, 2, or 3; and n is an integer between 1 and 37; or
x and y are 0 and R₁ and R₂ are together to form =O and R₃ is 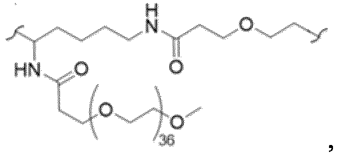
D₀ is methyl, propargyl, or a drug;
with a provision that at least one of R₁, R₂, and R₃ is not hydrogen;
if q, r, s, and t are 0, x+y+z>1;
if x is 0, R₁ is hydrogen, 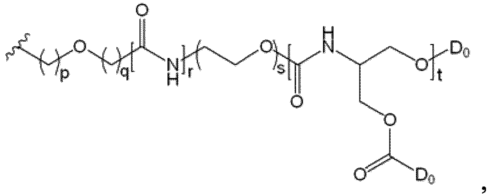 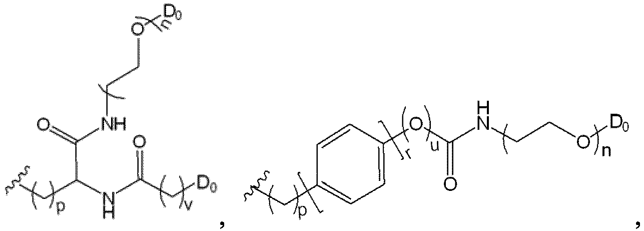 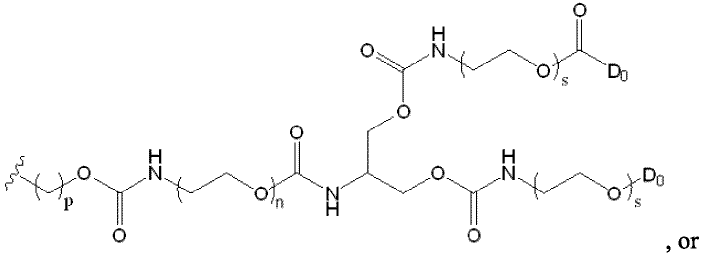 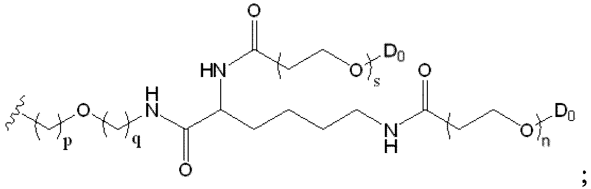
if y is 0, R₂ is hydrogen, 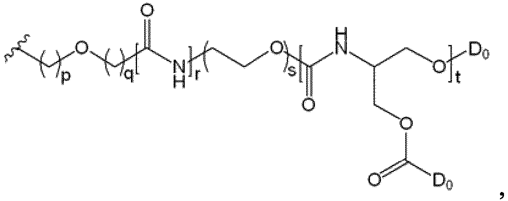 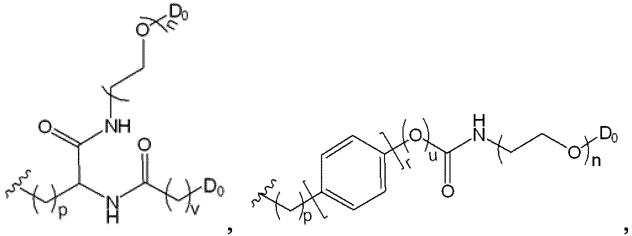 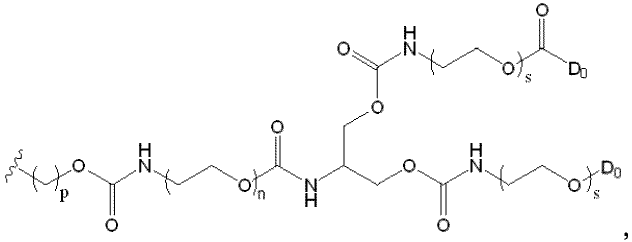 or 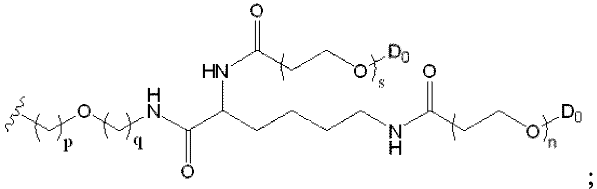
if z is 0, R₃ is hydrogen 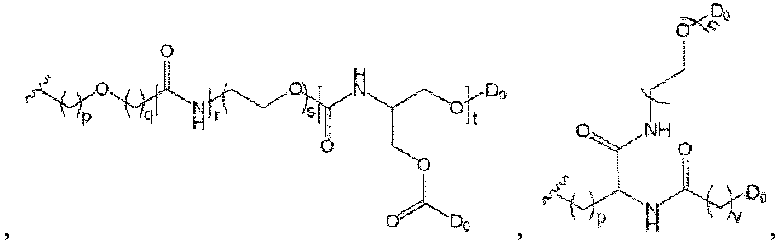 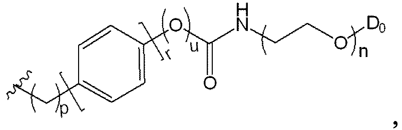 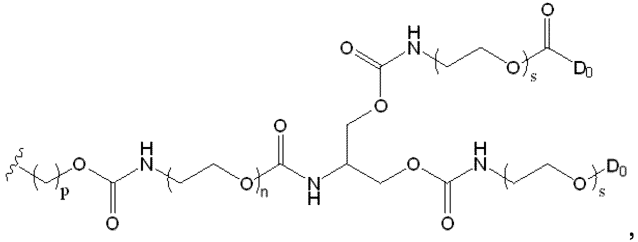 or 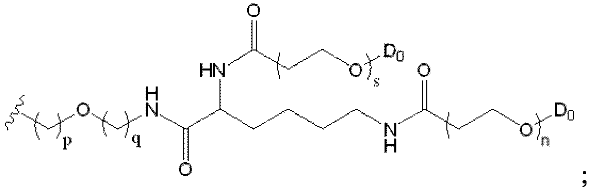
if x+y+z=1 and each of R₁, R₂, and R₃ independently is 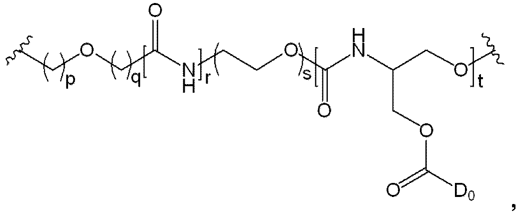 p is not 1;
if r, s, and t are 0 and each of R₁, R₂, and R₃ independently is 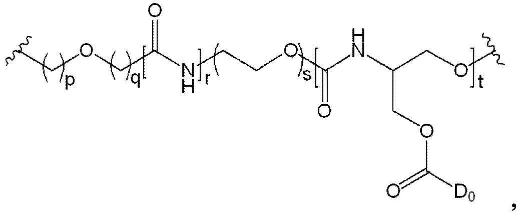 x+y+z>1.

In an embodiment, conjugates may be represented by Formula (II), 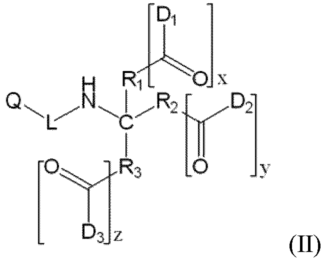 wherein
**Q** is a moiety selected from the group of peptide, polypeptide and protein that specifically binds to an antigen;
**L, D₁, D₂, D₃,** x, y, z, R₁, R₂, and R₃ are as defined as above.

In one embodiment, a substantial amount of the drug is not cleaved from the moiety until the conjugate enters a cell with a cell-surface receptor specific for the conjugate, and the drug is cleaved from the moiety when the moiety-drug conjugate does enter the cell.

In another embodiment, the moiety Q of Formula II is an antibody selected from a monoclonal antibody, an antigen-binding fragment, a chimeric antibody, and a humanized antibody. In another embodiment, the antigen-binding fragment is an Fab, F(ab')₂, Fv or scFv fragment.

Another aspect of the invention is a pharmaceutical composition including a Formula II conjugate, or a pharmaceutically acceptable salt thereof, and a pharmaceutically acceptable diluent, carrier, or excipient.

Another aspect includes kits, comprising an antibody-drug conjugate (ADC), a container, and a package insert or label indicating a treatment.

Another aspect includes methods of making, methods of preparing, methods of synthesis, methods of conjugation, and methods of purification of the conjugate, and the intermediates for the preparation, synthesis, and conjugation of the conjugate.

The linkers are preferably stable extracellularly. Before transport or delivery into a cell, the conjugate is preferably stable and remains intact, i.e. the moiety remains linked to the linker-drug combination. The linkers are stable outside the target cell and may be cleaved at some efficacious rate inside the cell. An effective linker will: (i) maintain the specific binding properties of the moiety; (ii) allow intracellular delivery of the conjugate or drug; (iii) remain stable and intact, i.e. not cleaved, until the conjugate has been delivered or transported to its targeted site; and (iv) maintain a cytotoxic (i.e. cell-killing) or cytostatic effect of the drug, e.g., maytansinoid. Stability of the conjugate may be measured by standard analytical techniques such as mass spectroscopy, HPLC, and the separation/analysis technique LC/MS.

Without further elaboration, it is believed that one skilled in the art can, based on the above description, utilize the present invention to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### EXAMPLES

### Syntheses of the Compounds in the Present Disclosure

### Example 1: Synthesis of FL-F21N-L111-P204 (Mal-Val-Cit-PAB-PEG4-Eribulin)

**Step 1-1:** Compound 48 (0.4 g, 0.54 mmol, 1.0 equiv.) was dissolved in dimethylacetamide (DMAc, 2.0 mL). 2-(2-(2-(2-aminoethoxy)ethoxy)ethoxy)ethan-1-ol (180.0 mg, 0.92 mmol, 1.7 equiv.) was also dissolved in DMAc (1.0 mL) with addition of N,N-diisopropylethylamine (DIPEA, 140.0 mg, 1.08 mmol, 2.0 equiv.). The 2-(2-(2-(2-aminoethoxy)ethoxy)ethoxy)ethan-1-ol solution was slowly added to the Compound **48** solution and stirred at 15-25°C for 16 hours. After the completion of the reaction was confirmed by ultra-performance liquid chromatography (UPLC), DMAc was removed by vacuum. The residue was further purified by hydrophilic-interaction chromatography (HILIC) so as to give FL-F21N-L111-P204-Int01 with a yield of 69%.

**Step 1-2:** FL-F21N-L111-P204-Int01 (180.0 mg, 0.22 mmol, 1.0 equiv.) was dissolved in DMAc (2.7 mL) and DIPEA (120.0 mg, 0.898 mmol, 4.0 equiv.) was added thereto. Bis(4-nitrophenyl) carbonate (B4NPC, 240.0 mg, 0.78 mmol, 3.5 equiv.) was added to the solution and stirred at 15-25°C for 2.5 hours. After the completion of the reaction was confirmed by UPLC, the solution was slowly added to ethyl acetate at 0-10°C under nitrogen atmosphere and stirred at the same temperature for 15 minutes, followed by 10 minutes at 15-25°C to precipitate crude FL-F21N-L111-P204-Int02. The crude was collected, rinsed with ethyl acetate repeatedly, filtered, and vacuum-dried to give FL-F21N-L111-P204-Int02 with a yield of 70%.

**Step 1-3:** Eribulin (40.0 mg, 0.05 mmol, 1.05 equiv.) was dissolved in DMAc (0.5 mL) and DIPEA (10.0 mg, 0.08 mmol, 1.5 equiv.) was added thereto. The solution was cooled down to 0-10°C and FL-F21N-L111-P204-Int02 (50.0 mg, 0.05 mmol, 1.0 equiv.) was added thereto. The solution was stirred at 15-25°C for 4.5 hours. After the completion of the reaction was confirmed by UPLC, the solution was purified by HILIC to afford FL-F21N-L111-P204 with a yield of 70%.

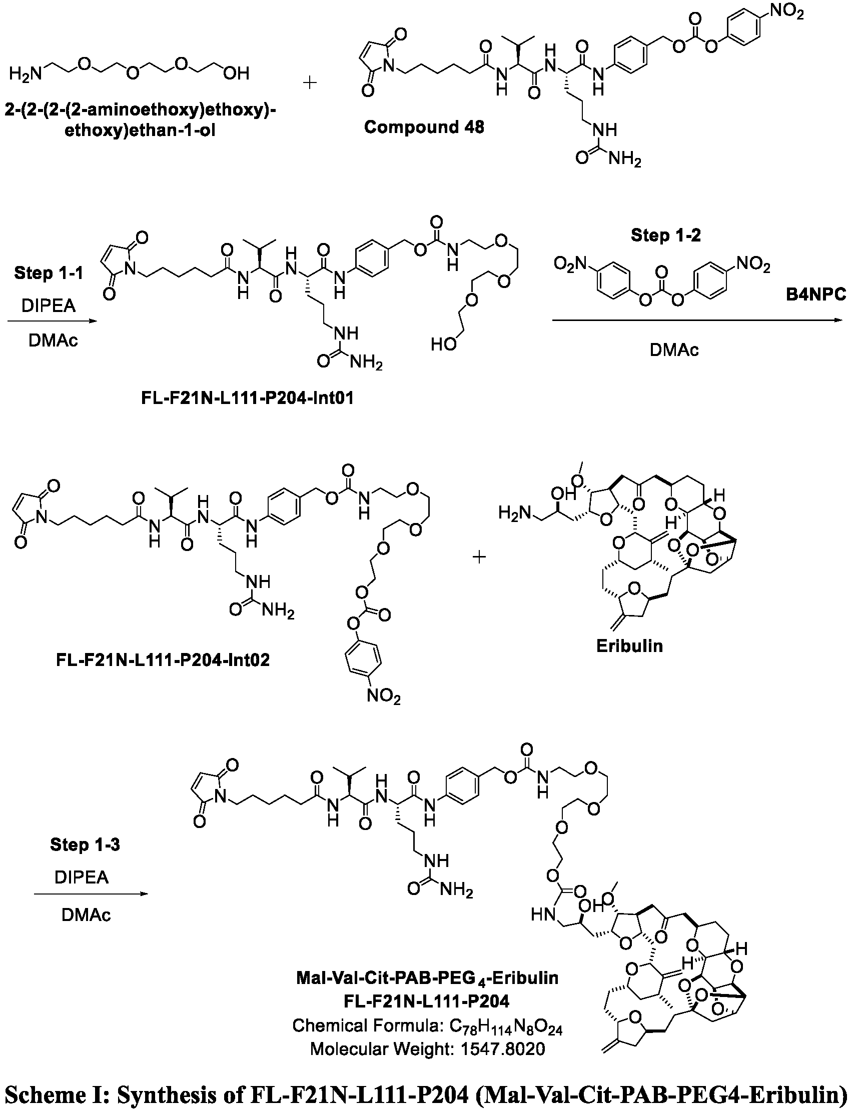

¹H-NMR (400MHz, DMSO-*d*₆) *δ* 9.96 (s, 1H), 8.05 (d, *J* = 7.6 Hz, 1H), 7.78 (d, *J* = 8.8 Hz, 1H), 7.58 (d, *J* = 8.8 Hz, 2H), 7.27 (d, *J* = 8.8 Hz, 2H), 7.19 (t, *J* = 4.9 Hz, 1H), 7.04 (t, *J* = 6.0 Hz, 1H), 6.99 (s, 1H), 5.96 (t, *J =* 5.4 Hz, 1H), 5.39 (s, 2H), 5.32 (t, *J =* 4.9 Hz, 1H), 5.05 (s, 1H), 5.00 (s, 1H), 4.93 (s, 2H), 4.83 (s, 1H), 4.75 (s, 1H), 4.63 (m, 1H), 4.55 (t, *J* = 4.0 Hz, 1H), 4.51 (d, *J* = 5.2 Hz, 1H), 4.38 (m, 1H), 4.33 (d, *J =* 4.0 Hz, 1H), 4.26 (m, 1H), 4.22-4.13 (m, 2H), 4.10 (s, 3H), 3.08-4.06 (m, 3H), 3.85-3.65 (m, 4H), 3.57-3.52 (m, 3H), 3.50 (s, 3H), 3.49 (s, 2H), 3.43-3.38 (m, 4H), 3.27-3.24 (m, 5H), 3.13 (m, 2H), 3.05-2.90 (m, 5H), 2.89-2.63 (m, 4H), 2.60-2.52 (m, 1H), 2.37-2.05 (m, 7H), 2.05-1.84 (m, 8H), 1.79-1.40 (m, 10H), 1.40-1.10 (m, 10H), 1.07-1.00 (m, 6H), 0.89-0.78 (m, 6H). ESI MS calculated [M+H]⁺: 1547.8, observed: 1547.7.

### Synthesis of FL-F21N-L167-P204 is similar to synthesis of Example 1.

### Example 2: Synthesis of FL-F21N-L106-P204 (Mal-Val-Cit-PAB-(PEG4-Eribulin)₂)

**Step 2-1:** Compound **48** (200.0 mg, 0.27 mmol, 1.0 equiv.) was dissolved in DMAc (1.2 mL). 2-aminopropane-1,3-diol (25.0 mg, 0.27 mmol, 1.0 equiv.) was also dissolved in DMAc (0.8 mL) with addition of DIPEA (32.0 mg, 0.27 mmol, 1.0 equiv.). The 2-aminopropane-1,3-diol solution was slowly added to the Compound **48** solution and stirred at 15-25°C for 3 hours. After the completion of the reaction was confirmed by UPLC, the solution was slowly added to ethyl acetate at 15-25°C and stirred for 1 hour to precipitate crude FL-F21N-L106-P204-Int01. The crude was collected, rinsed with ethyl acetate repeatedly, and purified by HILIC so as to give FL-F21N-L106-P204-Int01 with a yield of 60%.

**Step 2-2:** FL-F21N-L106-P204-Int01 (0.4 g, 0.58 mmol, 1.0 equiv.) and B4NPC (Bis(4-nitrophenyl) carbonate, 1.1 g, 3.50 mmol, 6.0 equiv.) were dissolved in DMAc (4.0 mL). The mixture was cooled down to 0-10°C and DIPEA (140.0 mg, 1.08 mmol, 2.0 equiv.) was added thereto. The reaction was performed at 15-25°C. After the completion of the reaction was confirmed by UPLC, n-heptane/ethyl acetate (1:1 v/v) solution was added in the mixture to precipitate crude FL-F21N-L106-P204-Int02. The crude was collected, rinsed with n-heptane/ethyl acetate (1:1 v/v) solution, filtered, and vacuum-dried to give FL-F21N-L111-P204-Int02 with a yield of 91%.

**Step 2-3:** FL-F21N-L106-P204-Int02 (200.0 mg, 0.20 mmol, 1.0 equiv.) was dissolved in DMAc (2.0 mL), and NaHCO₃ (33.0 mg, 0.39 mmol, 2.0 equiv.) was added thereto and the mixture was cooled down to 0-10°C. 2-(2-(2-(2-aminoethoxy)ethoxy)ethoxy)ethan-1-ol (103.0 mg, 0.53 mmol, 2.7 equiv.) was dissolved in DMAc (2.7 mL) and was slowly added to the FL-F21N-L106-P204-Int02 solution. The mixture was stirred at 15-25°C for 2 hours. After the completion of the reaction was confirmed by UPLC, n-heptane/ethyl acetate (1:1 v/v) solution was added in the mixture to precipitate crude FL-F21N-L106-P204-Int03. The crude was collected, rinsed with n-heptane/ethyl acetate (1:1 v/v) solution repeatedly, and purified by HILIC so as to give FL-F21N-L106-P204-Int03 with a yield of 36%.

**Step 2-4:** FL-F21N-L106-P204-Int03 (49.0 mg, 0.04 mmol, 1.0 equiv.) and B4NPC (106.0 mg, 0.35 mmol, 8.0 equiv.) were dissolved in DMAc (0.7 mL). The mixture was cooled down to 0-10°C and DIPEA (45.0 mg, 0.35 mmol, 8.0 equiv.) was added thereto. The mixture was stirred at 15-25°C for 2 hours. After the completion of the reaction was confirmed by UPLC, n-heptane/ethyl acetate (1:1 v/v) solution was added in the mixture to precipitate crude FL-F21N-L106-P204-Int04. The crude was collected, rinsed with n-heptane/ethyl acetate (1:1 v/v) solution repeatedly, filtered and vacuum-dried to give FL-F21N-L106-P204-Int04 with a yield of 71%.

**Step 2-5:** FL-F21N-L106-P204-Int04 (49.0 mg, 0.03 mmol, 1.0 equiv.) was dissolved in DMAc (2.0 mL). Eribulin (57.0 mg, 0.08 mmol, 2.30 equiv.) was dissolved in DMAc (0.5 mL) and DIPEA (14.0 mg, 0.11 mmol, 3.3 equiv.) was added thereto. The Eribulin solution was slowly added to the FL-F21N-L106-P204-Int04 solution and stirred at 15-25°C for 1 hour. After the completion of the reaction was confirmed by UPLC, ethyl acetate was added in the mixture to precipitate crude FL-F21N-L106-P204. The crude was collected, rinsed with ethyl acetate repeatedly, and purified by HILIC so as to give FL-F21N-L106-P204 with a yield of 25%.

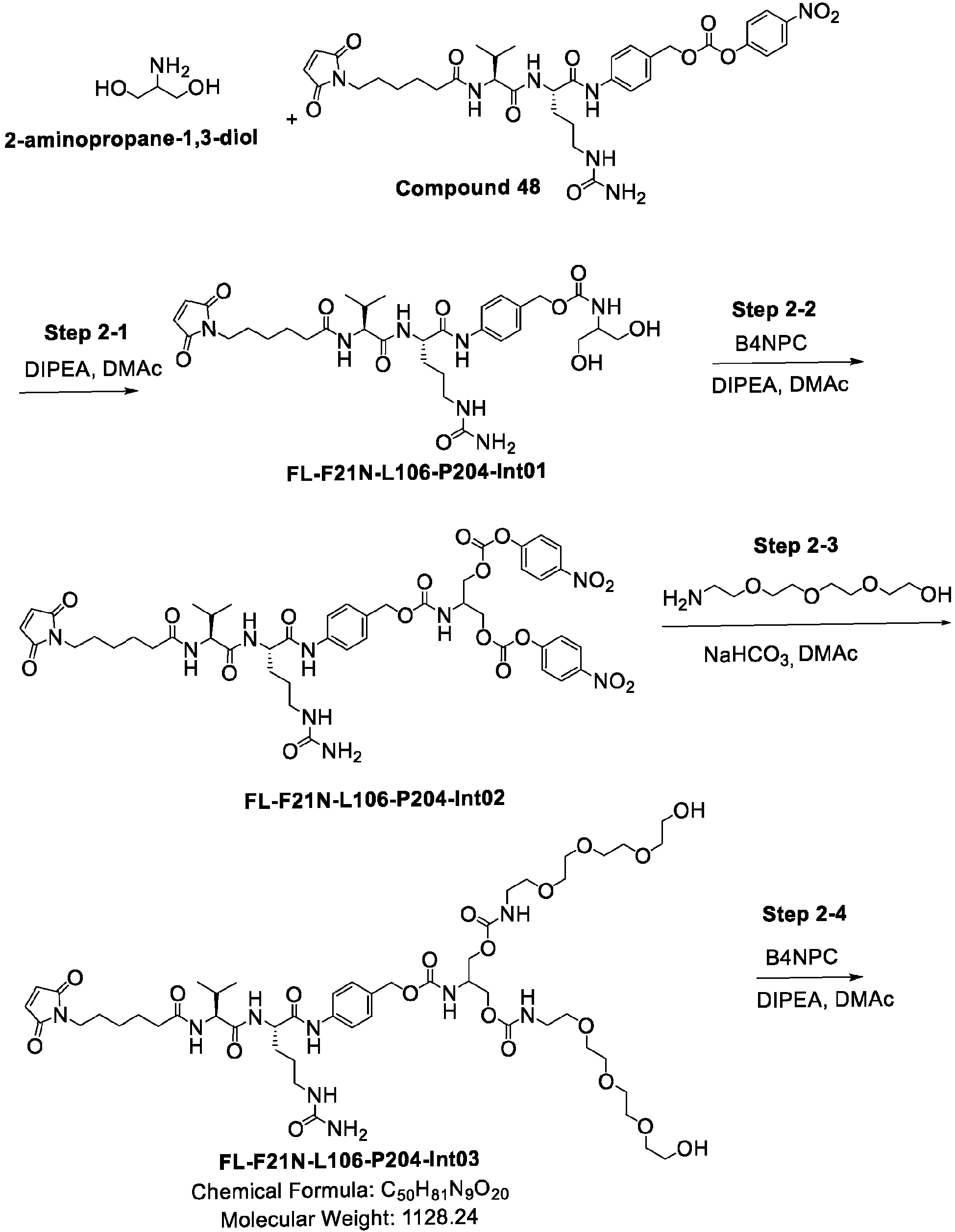

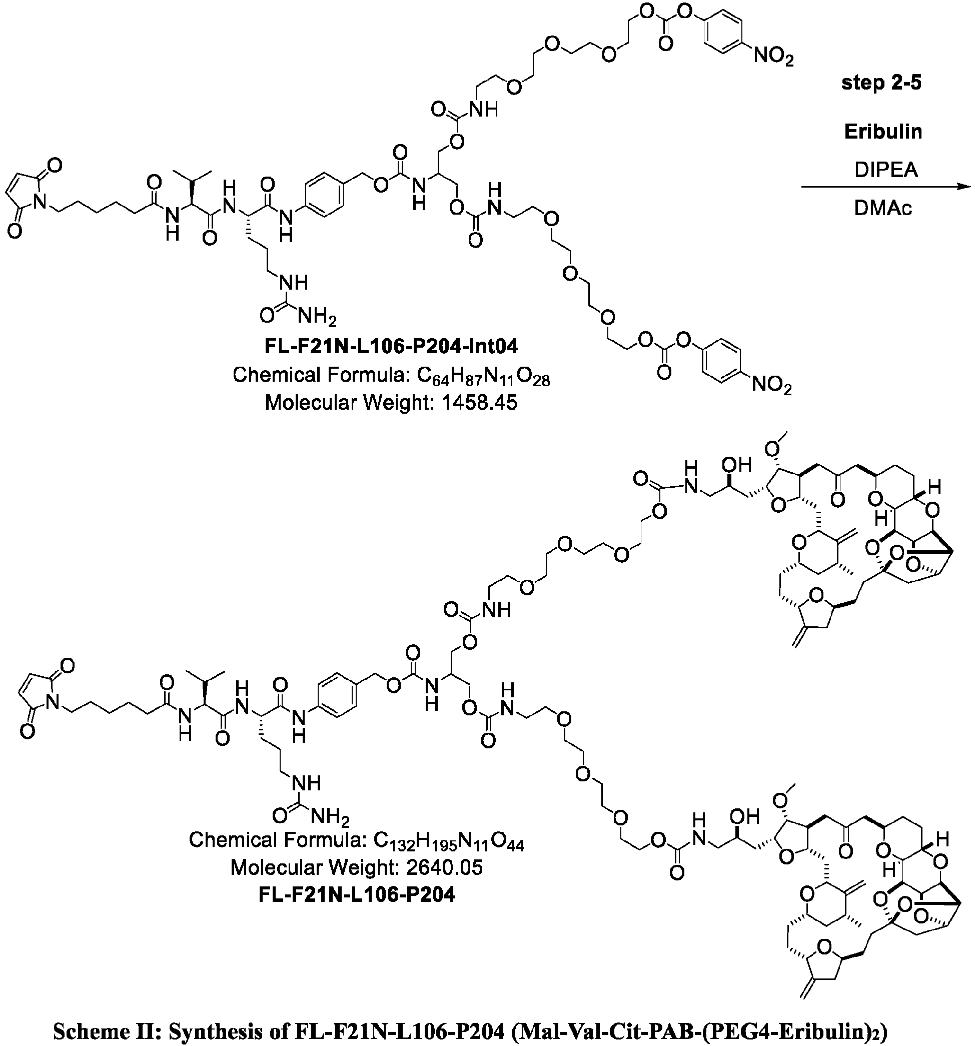

¹H-NMR (400MHz, DMSO-d₆) *δ* 9.96 (s, 1H), 8.04 (d, *J* = 7.6 Hz, 1H), 7.77 (d, *J* = 8.8 Hz, 1H), 7.59 (d, *J =* 8.4 Hz, 2H), 7.28 (d, *J* = 8.4 Hz, 2H), 7.16-7.08 (m, 2H), 7.02 (t, *J* = 5.6 Hz, 2H), 6.99 (s, 2H), 5.95 (t, *J =* 5.2 Hz, 1H), 5.38 (s, 2H), 5.08-5.02 (m, 2H), 5.02-4.97 (m, 2H), 4.97-4.92 (m, 2H), 4.86-4.80 (m, 2H), 4.78-4.72 (m, 2H), 4.66-4.60 (m, 2H), 4.55 (t, *J =* 4.0 Hz, 2H), 4.53-4.46 (m, 2H), 4.38 (m, 1H), 4.34-4.22 (m, 3H), 4.22-4.13 (m, 4H), 4.13-3.94 (m, 4H), 3.94-3.64 (m, 9H), 3.59-3.45 (m, 18H), 3.42-3.34 (m, 6H), 3.28-3.23 (m, 10H), 3.15-3.06 (m, 5H), 3.06-2.88 (m, 6H), 2.87-2.52 (m, 10H), 2.37-1.84 (m, 32H), 1.78-1.12 (m, 43H), 1.04 (d, *J =* 6 Hz, 6H), 0.89-0.78 (m, 6H). ESI MS calculated [M+2H]²⁺: 1320.2, observed: 1320.9.

### Example 3: Synthesis of FL-F21N-L107-P204 (Mal-Val-Cit-PAB-(Eribulin)₂)

**Step 3-1:** Compound **48** (1.0 g, 1.35 mmol, 1.0 equiv.) was dissolved in DMAc (40.0 mL). 2-aminopropane-1,3-diol (123.0 mg, 1.35 mmol, 1.0 equiv.) was also dissolved in DMAc (10.0 mL) with addition of DIPEA (175.0 mg, 1.35 mmol, 1.0 equiv.). The 2-aminopropane-1,3-diol solution was slowly added to the Compound **48** solution and stirred at 15-25°C for 7 hours. After the completion of the reaction was confirmed by UPLC, the mixture was slowly added to ethyl acetate at 15-25°C and stirred for 1 hour to precipitate crude FL-F21N-L107-P204-Int01. The crude was collected, rinsed with ethyl acetate repeatedly, and purified by HILIC so as to give FL-F21N-L107-P204-Int01 with a yield of 75%.

**Step 3-2:** FL-F21N-L107-P204-Int01 (504.0 mg, 0.73 mmol, 1.0 equiv.) and B4NPC (1.3 g, 4.4 mmol, 6.0 equiv.) were dissolved in DMAc (5.0 mL). The mixture was cooled down to 0-10°C and DIPEA (566.0 mg, 4.4 mmol, 6.0 equiv.) was added thereto. After the completion of the reaction was confirmed by UPLC, ethyl acetate was added in the mixture to precipitate crude FL-F21N-L107-P204-Int02. The crude was collected, rinsed with ethyl acetate, filtered, and vacuum-dried to give FL-F21N-L107-P204-Int02 with a yield of 91%

**Step 3-3:** FL-F21N-L107-P204-Int02 (100.0 mg, 0.10 mmol, 1.0 equiv.) was dissolved in DMAc (4.0 mL). Eribulin (201.0 mg, 0.28 mmol, 2.80 equiv.) was dissolved in DMAc (1.0 mL) and DIPEA (35.0 mg, 0.28 mmol, 2.8 equiv.) was added thereto. The Eribulin solution was slowly added to the FL-F21N-L107-P204-Int02 solution and stirred at 15-25°C for 3 hours. After the completion of the reaction was confirmed by UPLC, ethyl acetate was added in the solution to precipitate crude FL-F21N-L107-P204. The crude was collected, rinsed with ethyl acetate repeatedly, and further purified by HILIC so as to give FL-F21N-L106-P204 with a yield of 32%

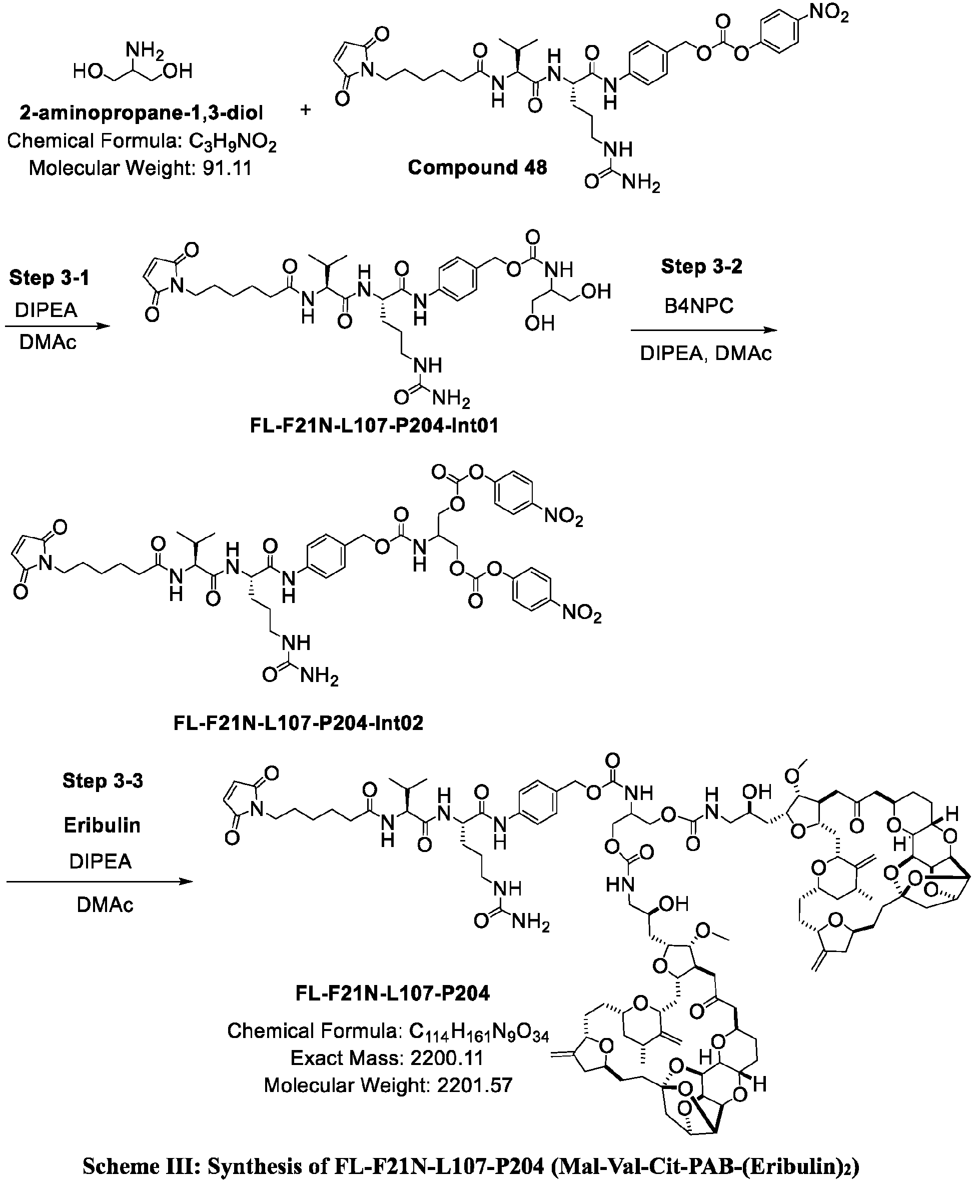

¹H-NMR (400MHz, DMSO-*d*₆) *δ* 9.95 (s, 1H), 8.03 (d, *J* = 7.6 Hz, 1H), 7.76 (d, *J* = 8.8 Hz, 1H), 7.59 (d, *J* = 8.4 Hz, 2H), 7.28 (d, *J* = 8.4 Hz, 2H), 6.99 (s, 2H), 6.99-6.94 (m, 1H), 5.95 (t, *J* = 5.6 Hz, 1H), 5.08-5.02 (m, 2H), 5.02-4.97 (m, 2H), 4.97-4.92 (m, 2H), 4.86-4.80 (m, 2H), 4.78-4.72 (m, 2H), 4.66-4.60 (m, 2H), 4.55 (t, *J =* 4.0 Hz, 2H), 4.52-4.47 (m, 2H), 4.38 (m, 1H), 4.31 (d, *J =* 4.0 Hz, 1H), 4.30-4.22 (m, 2H), 4.22-4.13 (m, 3H), 4.13-4.06 (m, 6H), 4.06-3.93 (m, 4H), 3.93-3.65 (m, 11H), 3.58-3.45 (m, 6H), 3.37 (t, *J =* 6.8 Hz, 2H), 3.28-3.22 (m, 6H), 3.09-2.88 (m, 7H), 2.84 (d, *J =* Hz, 2H), 2.80-2.64 (m, 5H), 2.61-2.52 (m, 2H), 2.36-2.06 (m, 14H), 2.06-1.85 (m, 15H), 1.77-1.11 (m, 35H), 1.08-1.00 (m, 6H), 0.88-0.79 (m, 6H). ESIMS calculated [M+H+NH₄]²⁺: 1109.6, observed: 1110.2.

### Example 4: Synthesis pf FL-F21N-L108-P204 (Mal-Val-Cit-PAB-(Eribulin)₃)

**Step 4-1:** 3,3'-((2-amino-2-((2-carboxyethoxy)methyl)propane-1,3-diyl)bis(oxy)) dipropionic acid (1.3 g, 3.85 mmol, 1.0 equiv.) was dissolved in DMAc (40.0 mL) and cooled down to 0-10°C. HOBt (3.1 g, 23.10 mmol, 6.0 equiv.), DIPEA (4.5 g, 34.65 mmol, 9.0 equiv.) and Compound **48** (2.8 g, 1.35 mmol, 1.0 equiv.) were added and the mixture was stirred at 15-25°C for 5 hours. After the completion of the reaction was confirmed by UPLC, ethyl acetate was added in the mixture to precipitate crude FL-F21N-L108-P204-Int01. The crude was collected and further purified by preparative HPLC so as to give FL-F21N-L108-P204-Int01 with a yield of 7%.

**Step 4-2:** FL-F21N-L108-P204-Int01 (228.0 mg, 0.24 mmol, 1.0 equiv.) was dissolved in DMAc (2.3 mL). HOAt (157.0 mg, 1.15 mmol, 4.8 equiv.), HATU (329.0 mg, 0.86 mmol, 3.6 equiv.) and DMAP (176.0 mg, 1.44 mmol, 6.0 equiv.) were added and cooled down to 0-10°C. Eribulin (674.0 mg, 0.92 mmol, 3.85 equiv.) was added and the mixture was stirred at 0-10°C for 3 hours. After the completion of the reaction was confirmed by HPLC, ethyl acetate was added in the mixture to precipitate crude FL-F21N-L108-P204. The crude was collected and further purified by preparative HPLC so as to give FL-F21N-L108-P204 with a yield of 11%.

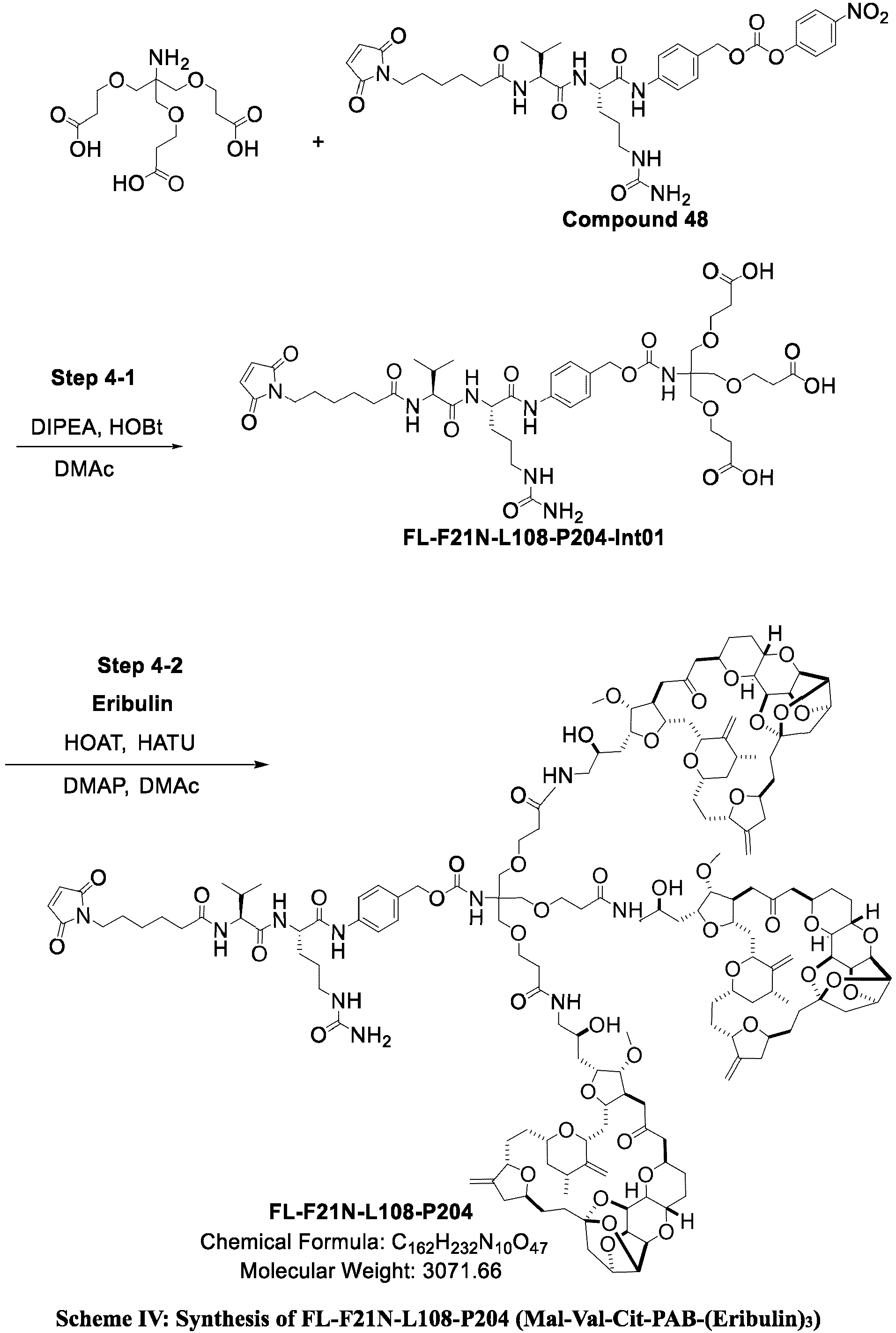

¹H-NMR (400MHz, DMSO-*d*₆) *δ* 11.94 (br, 3H), 9.95 (s, 1H), 8.09 (s, 1H), 8.04 (d, *J =* 7.5 Hz, 1H), 7.77 (m, 3H), 7.59 (d, *J* = 8.4 Hz, 2H), 7.27 (d, *J* = 8.4 Hz, 2H), 7.18 (s, 2H), 6.99 (s, 2H), 6.64 (s, 2H), 6.45 (s, 2H), 5.96 (t, *J =* 5.8 Hz, 1H), 5.39 (s, 2H), 5.32 (t, *J =* 4.8 Hz, 3H), 5.05 (s, 3H), 4.98 (s, 3H), 4.90 (s, 2H), 4.82 (s, 3H), 4.74 (s, 3H), 4.63 (m, 3H), 4.58-4.54 (m, 5H), 4.39-4.35 (m, 1H), 4.27-4.23 (m, 3H), 4.20-4.14 (m, 3H), 4.10 (s, 6H), 4.04-3.99 (m, 2H), 3.83-3.68 (m, 9H), 3.57-3.48 (m, 15H), 3.26 (s, 9H), 3.11-3.0 (m, 6H), 2.84 (d, 3H), 2.76-2.65 (m, 6H), 2.33-2.14 (m, 23H), 2.01-1.92 (m, 22H), 1.73-1.61 (m, 17H), 1.51-1.44 (m, 13H), 1.35-1.29 (m, 15H), 1.19-1.13 (m, 6H), 1.03 (d, *J =* 6.4 Hz, 9H), 0.87-0.81 (m, 15H); MALDI-TOF MS calculated [M+Na]⁺: 3092.5964, observed: 3092.4241.

### Example 5: Synthesis of FL-F21N-L162-P204 (Mal-Val-Cit-PEG12-Eribulin)

**Step 5-1:** Compound **48** (1.0 g, 1.36 mmol, 1.0 equiv.) was dissolved in DMAc (5.0 mL). NH₂-PEG12-OH (888.0 mg, 1.63 mmol, 1.2 equiv.) was also dissolved in DMAc (5.0 mL). The NH₂-PEG12-OH solution was slowly added to the Compound **48** solution and stirred at 15-25°C for 1 hour. After the completion of the reaction was confirmed by HPLC, the mixture was slowly added to the n-heptane/ethyl acetate (3/10, 65mL) solution to precipitate crude FL-F21N-L162-P204-Int01. The crude was collected, rinsed with the n-heptane/ethyl acetate (3/10) solution, filtered, and vacuum-dried to give FL-F21N-L162-P204-Int01 with a yield of 91%.

**Step 5-2:** FL-F21N-L162-P204-Int01 (500.0 mg, 0.44 mmol, 1.0 equiv.) was dissolved in DMAc (5.0 mL) and DIPEA (169.0 mg, 1.32 mmol, 3.0 equiv.) was added thereto. The mixture was cooled down to 5-10°C, and B4NPC (399.0 mg, 1.32 mmol, 3.0 equiv.) was added thereto. The mixture was stirred at 5-10°C for 30 minutes and followed by stirring at 15-25°C for an additional 2.5 hours. After the completion of the reaction was confirmed by HPLC, n-heptane/ethyl acetate (1:2, v/v) solution was added under nitrogen atmosphere, and the mixture was stirred at 0-10°C for 15 minutes, followed by stirring at 15-25°C for an additional 10 minutes to precipitate crude FL-F21N-L162-P204-Int01. The crude was collected, rinsed with n-heptane/ethyl acetate (1:2, v/v) solution, filtered, and vacuum-dried to give FL-F21N-L162-P204-Int02 with a yield of 57%.

**Step 5-3:** Eribulin (134.0 mg, 0.18 mmol, 1.2 equiv.) was dissolved in DMAc (0.5 mL) and cooled to 0-10°C. FL-F21N-L162-P204-Int02 (200.0 mg, 0.15 mmol, 1.0 equiv.) was added thereto. The solution was stirred at 15-25°C for 4.5 hours. After the completion of the reaction was confirmed by HPLC, the solution was purified by HILIC to afford FL-F21N-L162-P204 with a yield of 28%.

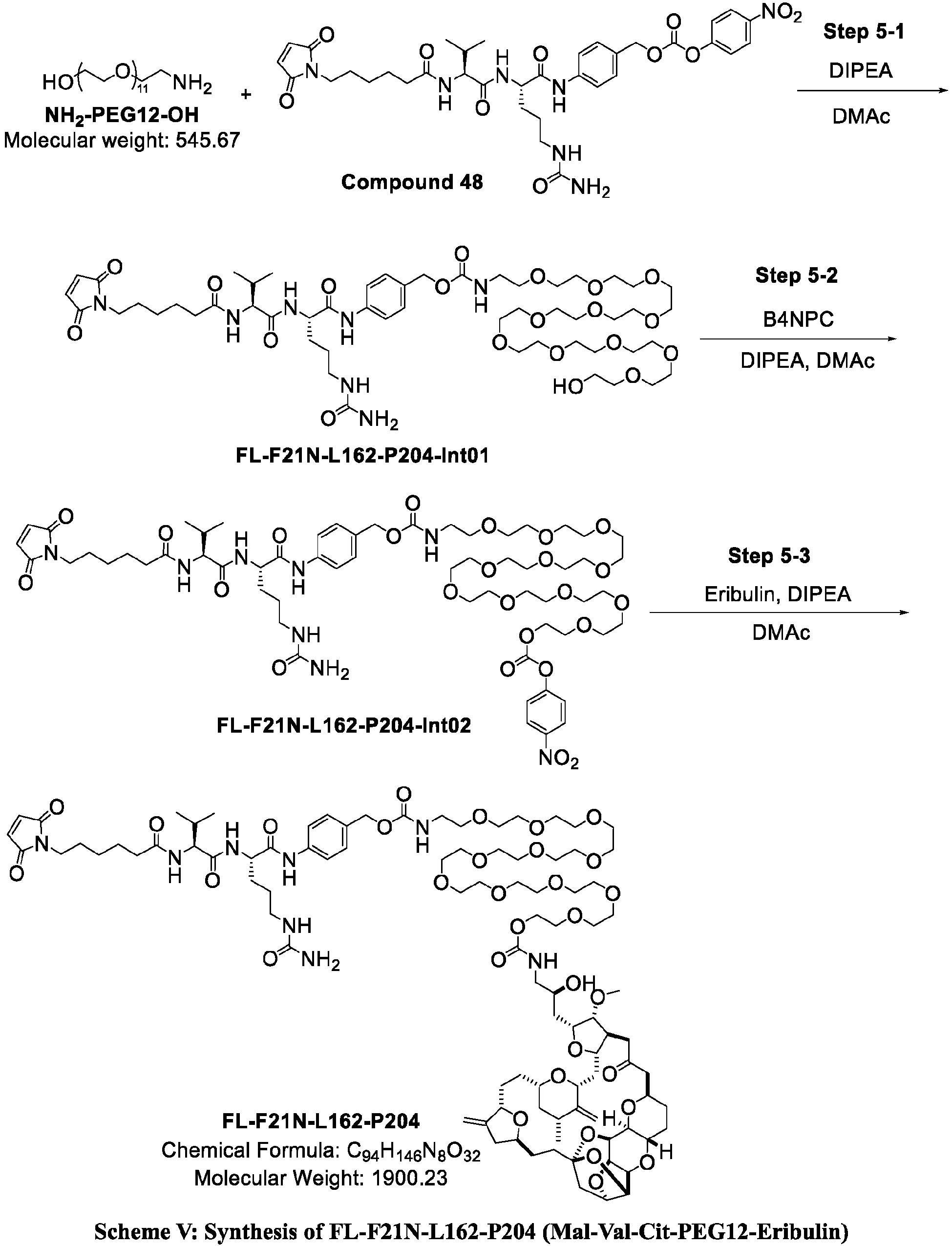

¹H NMR (600 MHz, DMSO-*d*₆) *δ* 9.95 (s, 1H), 8.05 (d, *J =* 7.5 Hz, 1H), 7.78 (d, *J =* 8.7 Hz, 1H), 7.56 (d, *J* = 8.2 Hz, 2H), 7.25 (d, *J* = 8.2 Hz, 2H), 7.18 (t, *J* = 5.7 Hz, 1H), 7.04 (t, *J* = 6.0 Hz, 1H), 6.98 (s, 2H), 5.95 (t, *J* = 5.9 Hz, 1H), 5.38 (s, 2H), 5.04 (s, 1H), 4.98 (s, 1H), 4.91 (s, 2H), 4.81 (s, 1H), 4.73 (s, 1H), 4.63-4.60 (m, 1H), 4.53 (t, *J =* 4.3 Hz, 1H), 4.50 (d, *J =* 5.2 Hz, 1H), 4.34-4.38 (m, 1H), 4.24 (d, *J* = 11.0 Hz, 1H), 4.19-4.12 (m, 2H), 4.10-4.06 (m, 3H), 4.01 (t, *J* = 4.9 Hz, 3H), 3.80-3.77 (m, 1H), 3.75-3.72 (m, 1H), 3.68-3.65 (m, 1H), 3.53 (t, *J =* 4.8 Hz, 2H), 3.48 (s, 34H), 3.38 (t, *J* = 6.0 Hz, 2H), 3.35 (t, *J* = 7.1 Hz, 2H), 3.24 (s, 3H), 3.10-3.13 (m, 2H), 3.02-2.90 (m, 5H), 2.82 (d, *J* = 10.2 Hz, 1H), 2.76-2.66 (m, 3H), 2.60-2.52 (m, 2H), 2.32-2.07 (m, 10H), 2.01-1.86 (m, 7H), 1.75-1.57 (m, 7H), 1.53-1.42 (m, 7H), 1.38-1.10 (m, 12H), 1.02 (d, *J =* 6.4 Hz, 3H), 0.93-0.99 (m, 2H), 0.83 (d, *J =* 6.7Hz, 3H), 0.80 (d, *J =* 6.7Hz, 3H). ESI MS calculated [M+H]⁺: 1899.0, observed: 1900.4.

### Example 6: Synthesis of FL-F21N-L111-P209 (Mal-Val-Cit-PEG4-Exatecan)

**Step 6-1:** Compound **48** (0.50g, 0.68 mmol, 1.0 equiv.) was dissolved in dimethylacetamide (DMAc, 2.0 mL). 2-(2-(2-(2-aminoethoxy)ethoxy)ethoxy)ethan-1-ol (327.0 mg, 1.69 mmol, 2.5 equiv.) was also dissolved in DMAc (2.0 mL). The 2-(2-(2-(2-aminoethoxy)ethoxy)ethoxy)ethan-1-ol solution was slowly added to the Compound **48** solution and stirred at 15-25°C for 30 minutes. After the completion of the reaction was confirmed by UPLC, the solution was slowly added to ethyl acetate (25mL) at 15-25°C and stirred at the same temperature for 30 minutes. The mixture was filtered, vacuum-dried, and purified by HILIC so as to give FL-F21N-L111-P209-Int01 with a yield of 34%.

**Step 6-2:** FL-F21N-L111-P209-Int01 (180.0 mg, 0.22 mmol, 1.0 equiv.) was dissolved in DMAc (3.0 mL) and DIPEA (176.0 mg, 1.32 mmol, 6.0 equiv.) was added thereto. B4NPC (240.0 mg, 0.78 mmol, 3.5 equiv.) was added to the solution and stirred at 15-25°C for 1 hour. After the completion of the reaction was confirmed by UPLC, the solution was slowly added to ethyl acetate at 0-10°C under nitrogen atmosphere and stirred at the same temperature for 20 minutes to precipitate crude FL-F21N-L111-P209-Int02. The crude was collected, rinsed with ethyl acetate repeatedly, filtered, and vacuum-dried to give FL-F21N-L111-P209-Int02 with a yield of 82%.

**Step 6-3:** FL-F21N-L111-P209-Int02 (90.8 mg, 0.10 mmole, 1.0 equiv.) was dissolved in DMAc (4.5 mL) and exatecan mesylate (55 mg, 0.11 mmole, 1.1 equiv.) was added thereto. The mixture was cooled down to 5-10°C and DIPEA (2.45 mg, 0.2 equiv.) was added in 6 times respectively with 60-min intervals. The addition amount of DIPEA was 13.5 mg (1.1 equiv) in total. The mixture was stirred at 5-10°C for 96 hours. After the completion of the reaction was confirmed by UPLC, the mixture was slowly added to ethyl acetate (20 mL) at 5-15°C and stirred between -10°C and -5°C for 60 minutes. After centrifugation (4000 rpm, 15°C, 5 minutes), the supernatant was removed. The pellet was rinsed with ethyl acetate (20mL), vacuum-dried, and purified by HILIC to give FL-F21N-L111-P209 with a yield of 11.2%.

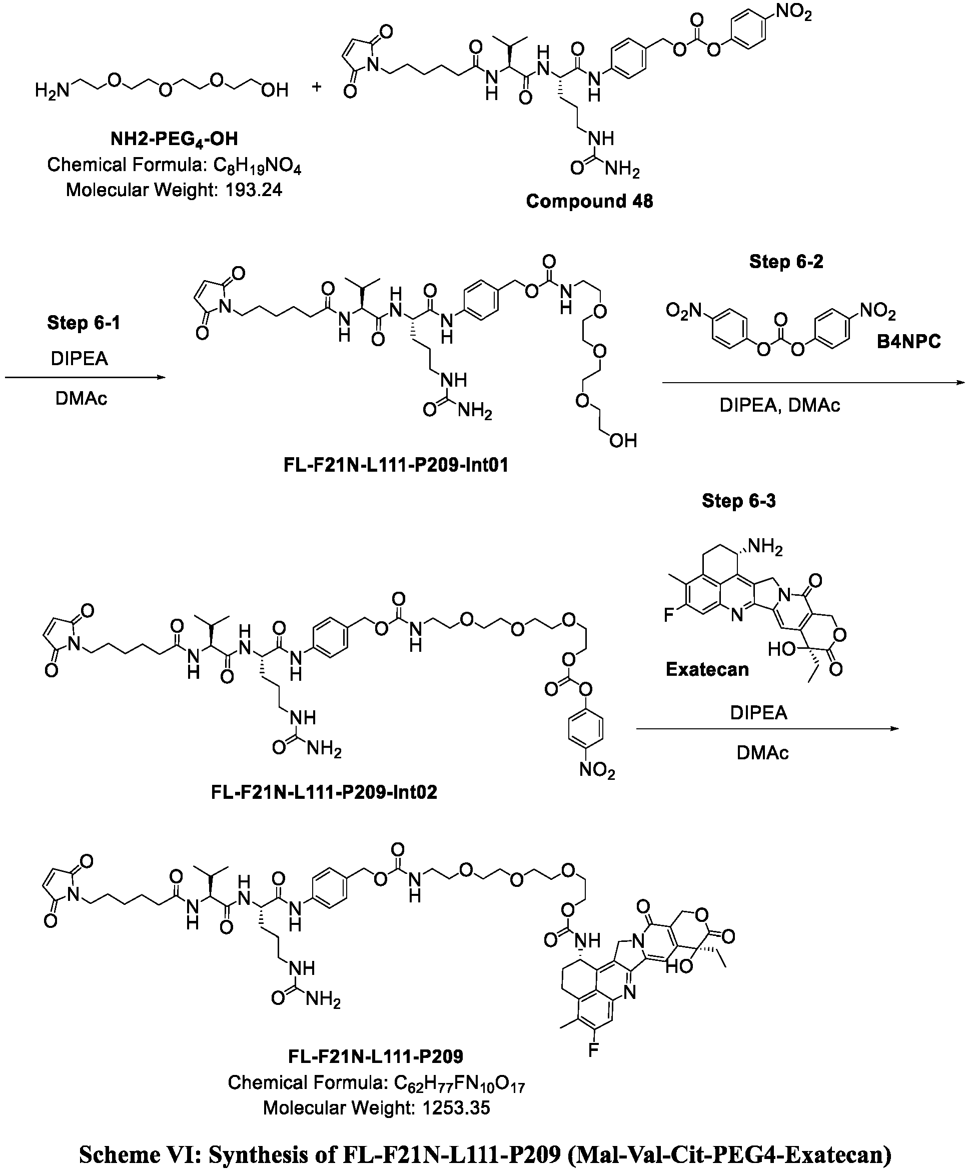

¹H NMR (400 MHz, DMSO-*d*₆) *δ* 9.95 (s, 1H), 8.05-8.01 (m, 2H), 7.79-7.53 (m, 2H), 7.57 (d, *J* = 8.5 Hz, 2H), 7.31 (s, 1H), 7.25 (d, *J* = 8.5 Hz, 2H), 7.17 (m, 1H), 6.99 (s, 1H), 6.49 (s, 1H), 5.95 (t, *J =* 5.8 Hz, 1H), 5.42 (s, 2H), 5.38 (s, 2H), 5.24 (s, 2H), 5.24 (m, 1H), 4.91 (s, 2H), 4.37 (m, 1H), 4.21-4.16 (m, 3H), 3.64-3.60 (m, 2H), 3.57-3.41 (m, 6H), 3.40-3.32 (m, 4H), 3.12-3.04 (m, 3H), 3.04-2.89 (m, 2H), 2.38 (s, 3H), 2.25-1.70 (m, 9H), 1.29-1.13 (m, 12H), 0.90-0.80 (m, 9H). ESIMS calculated [M+H]⁺: 1253.5, observed: 1253.4

### Example 7: Synthesis of FL-F21N-L164-P204 (MC-(PEG4-Eribulin)₂)

**Step 7-1:** 4-(N-Maleimidomethyl)cyclohexaneformic acid N-hydroxysuccinimide ester (500.0 mg, 1.50 mmol, 1.0 equiv.) was dissolved in DMAc (10 mL), and 3-aminopentane-1,5-diol (244.9 mg, 2.06 mmol, 1.4 equiv.) was slowly added thereto. The mixture was stirred at 15-25°C for 3 hours. After the completion of the reaction was confirmed by UPLC, the mixture was concentrated under reduced pressure to remove most of the DMAc, mixed with n-heptane/ethyl acetate (2:1, v/v) in ice bath, and stirred for 30 minutes to precipitate crude FL-F21N-L164-P204-Int01. The crude was collected and rinsed with n-heptane/ethyl acetate (2:1, v/v) to give FL-F21N-L164-P204-Int01 with a crude yield of 125%.

**Step 7-2:** FL-F21N-L164-P204-Int01 (441.0 mg, 1.30 mmol, 1.0 equiv.) was dissolved in DMAc (4.4 mL). DIPEA (1.0 mL, 5.87 mmol, 4.5 equiv.) and B4NPC (1.8 g, 5.87 mmol, 4.5 equiv.) were added. After the completion of the reaction was confirmed by UPLC, the mixture was concentrated under reduced pressure to remove most of the DMAc and mixed with n-heptane/ethyl acetate (3:1, v/v) in ice bath to precipitate crude FL-F21N-L164-P204-Int02. The crude was collected, rinsed with n-heptane/ethyl acetate (9:1, v/v), filtered, and vacuum-dried to afford FL-F21N-L164-P204-Int02 with a yield of 83%.

**Step 7-3:** FL-F21N-L164-P204-Int02 (429.5 mg, 0.64 mmol, 1.0 equiv.) was dissolved in DMAc (17.2 mL), and NaHCO₃ (135.0 mg, 1.61 mmol, 2.5 equiv.) was added thereto. 2-(2-(2-(2-aminoethoxy)ethoxy)ethoxy)ethan-1-ol (323.1 mg, 0.53 mmol, 2.6 equiv.) was slowly added to the FL-F21N-L164-P204-Int02 solution. The mixture was stirred at 15-25°C for 2 hours. After the completion of the reaction was confirmed by UPLC, n-heptane/ethyl acetate (9:1 v/v) solution was added in the mixture to precipitate crude FL-F21N-L164-P204-Int03. The crude was collected, rinsed with n-heptane/ethyl acetate (9:1 v/v) solution repeatedly, filtered, vacuum-dried, and purified by HILIC so as to give FL-F21N-L164-P204-Int03 with a yield of 38%.

**Step 7-4:** FL-F21N-L164-P204-Int03 (177.1 mg, 0.23 mmol, 1.0 equiv.) was dissolved in DMAc (1.8 mL) and DIPEA (0.1 mL, 0.57 mmol, 2.5 equiv.) and B4NPC (208.0 mg, 0.68 mmol, 3.0 equiv.) were added thereto. After the completion of the reaction was confirmed by UPLC, the mixture was concentrated under reduced pressure to remove most of the DMAc and mixed with n-heptane/ethyl acetate (9:1, v/v) in ice bath to precipitate crude FL-F21N-L164-P204-Int04. The crude was collected, rinsed with n-heptane/ethyl acetate (9:1, v/v), filtered, and vacuum-dried to afford FL-F21N-L164-P204-Int04 with a yield of 74%.

**Step 7-5:** FL-F21N-L164-P204-Int04 (177.6 mg, 0.16 mmol, 1.0 equiv.) was dissolved in DMAc (7.1 mL). Eribulin (234.1 mg, 0.32 mmol, 2.0 equiv.) was dissolved in DMAc (1.8 mL) and DIPEA (14.0 mg, 0.11 mmol, 3.3 equiv.) was added thereto. The Eribulin solution was slowly added to the FL-F21N-L164-P204-Int04 solution and stirred at 15-25°C for 4 hours. After the completion of the reaction was confirmed by UPLC, n-heptane/ethyl acetate (3:1, v/v) was added in the mixture to precipitate crude FL-F21N-L164-P204. The crude was collected, rinsed with n-heptane/ethyl acetate (3:1 v/v) solution repeatedly, filtered, and vacuum-dried to afford FL-F21N-L164-P204 with a yield of 72%.

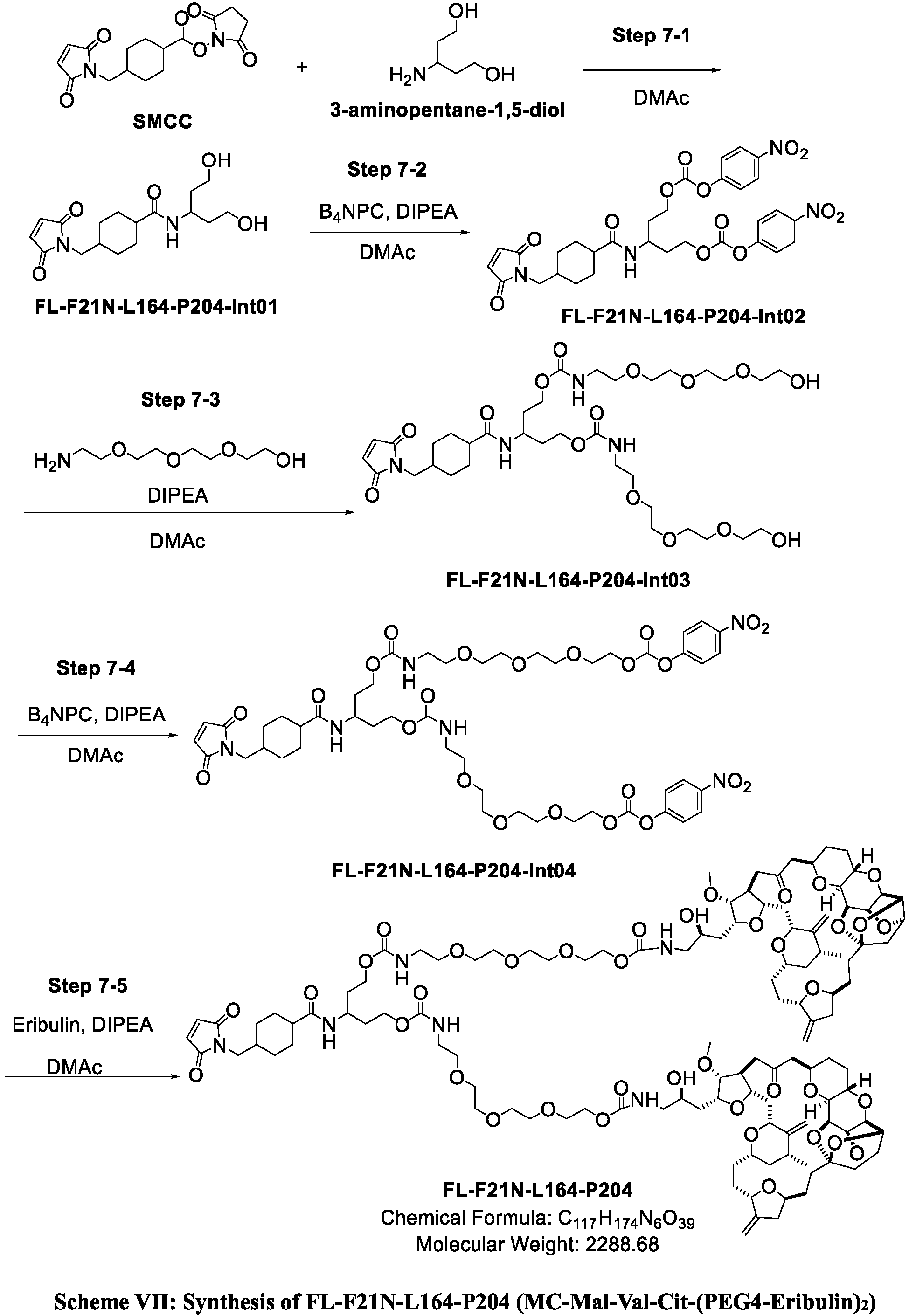

¹H-NMR (400MHz, DMSO-*d*₆) *δ* 6.99 (s, 2H), 5.05 (s, 2H), 4.99 (s, 2H), 4.83 (s, 2H), 4.75 (s, 2H), 4.63 (m, 2H), 4.55 (t, *J* = 4.0 Hz, 2H), 4.49 (d, *J* = 4.8 Hz, 2H), 4.26 (d, *J* = 10.4 Hz, 2H), 4.20-4.14 (m, 3H), 4.10 (s, 6H), 4.04-4.02 (m, 6H), 3.89-3.73 (m, 11H), 3.68-3.67 (m, 3H), 3.56-3.54 (m, 6H), 3.51 (s, 9H), 3.49 (s, 9H), 3.38 (t, *J =* 6.0 Hz, 6H), 3.26 (m, 8H), 3.23 (s, 1H), 3.12-3.07 (m, 4H), 2.95 (t, *J* = 5.7 Hz, 4H), 2.84 (d, *J* = 7.2 Hz, 2H), 2.79-2.66 (m, 5H), 2.60-2.53 (m, 3H), 2.34-2.13 (m, 12H), 2.04-1.87 (m, 12H), 1.76-1.60 (m, 16H), 1.56-1.42 (m, 7H), 1.33-1.16 (m, 12H), 1.04 (d, *J =* 6.4 Hz, 6H), 0.99-0.84 (m, 4H). ESIMS calculated [M+H]⁺: 2288.2, observed: 2287.8.

### Example 8: Synthesis of FL-F21N-L111-P211 (Mal-Val-Cit-PAB-PEG4-MMAF)

**Step 8-1:** Compound **48** (1.2 g, 1.627 mmol, 1.0 equiv.) was dissolved in dimethylacetamide (DMAc, 4.8 mL). 2-(2-(2-(2-aminoethoxy)ethoxy)ethoxy)ethan-1-ol (0.787 g, 4.066 mmol, 2.5 equiv.) was also dissolved in DMAc (4.8 mL). The 2-(2-(2-(2-aminoethoxy)ethoxy)ethoxy)ethan-1-ol solution was slowly added to the Compound **48** solution and stirred at 15-25°C for 30 minutes. After the completion of the reaction was confirmed by UPLC, the mixture was slowly added into ethyl acetate (60 mL) to precipitate crude FL-F21N-L111-P211-Int01. After centrifugation (5000 rpm, 15°C, 5 minutes), the supernatant was removed. The pellet was rinsed with ethyl acetate, vacuum-dried, and purified by HILIC to give FL-F21N-L111-P211-Int01 with a yield of 34%.

**Step 8-2:** FL-F21N-L111-P211-Int01 (188.2 mg, 0.237 mmol, 1.0 equiv.) was dissolved in DMAc (3.8 mL) and DIPEA (93.0 mg, 0.713 mmol, 3.0 equiv.) was added thereto. B4NPC (217.0 mg, 0.713 mmol, 3 equiv.) was added into the solution and stirred at 20-25°C for 5 hour. After the completion of the reaction was confirmed by UPLC, the solution was slowly added into ethyl acetate at 0-10°C under nitrogen atmosphere and stirred at the same temperature for 20 minutes. After centrifugation (5000 rpm, 15°C, 5 minutes), the supernatant was removed. The pellet was rinsed with ethyl acetate and vacuum-dried to give FL-F21N-L111-P204-Int02 with a yield of 76%.

**Step 8-3:** FL-F21N-L111-P211-Int02 (171.6 mg, 0.179 mmol, 1.0 equiv.) was dissolved in DMAc (6.9 mL), mixed with HOAt (19.5 mg, 0.108 mmol, 0.8 equiv.), MMAF (144.1 mg, 0.1969 mmol, 1.1 equiv.) and DIPEA (104.1 mg, 0.81 mmol, 4.5 equiv.), and stirred at 21°C for 46 hours. After the completion of the reaction was confirmed by UPLC, the mixture was evaporated in vacuum to remove DMAc, mixed with ethyl acetate (35 mL), and stirred at 0-4°C for 20 minutes to precipitate FL-F21N-L111-P211. After centrifugation (4000 rpm, 15°C, 5 minutes), the supernatant was removed. The pellet was rinsed with n-heptane/ethyl acetate (1:1, v/v), vacuum-dried, and purified by RP-18 chromatography to afford FL-F21N-L111-P211 with a yield of 22%.

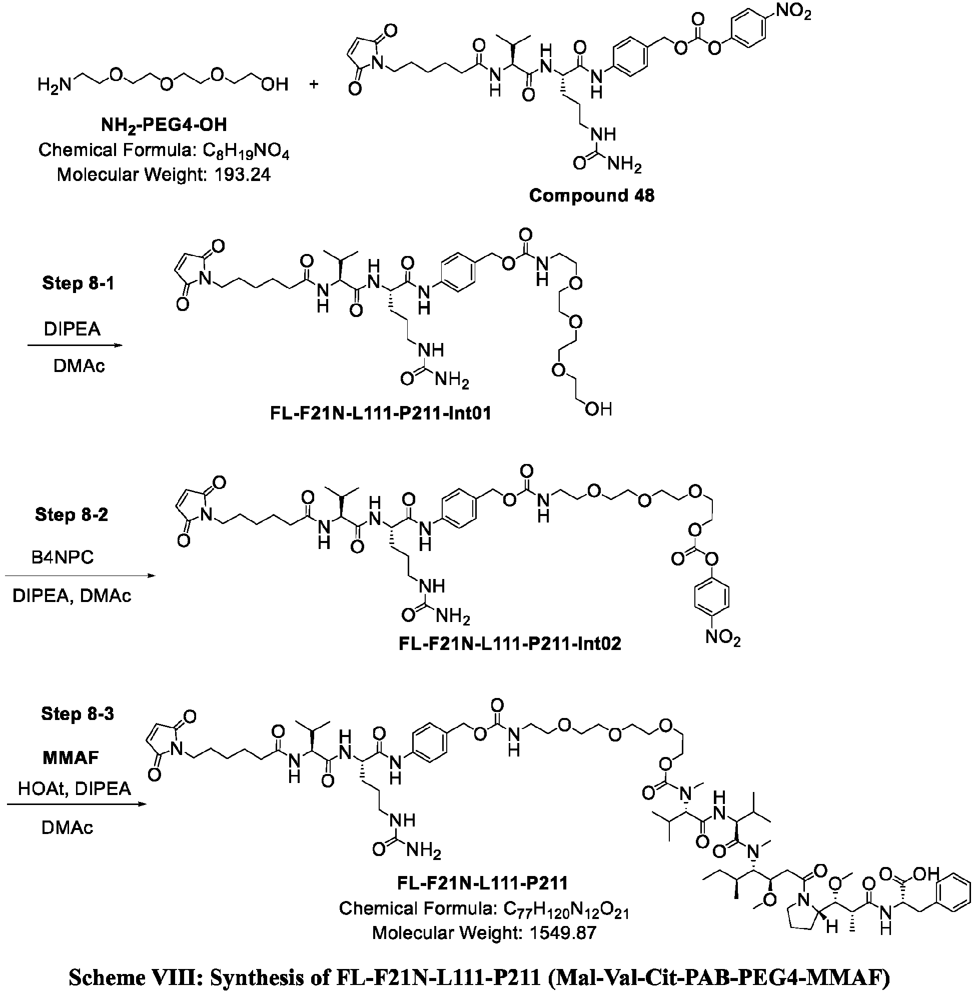

¹H-NMR (400MHz, DMSO-*d*₆) *δ* 9.98 (s, 1H), 8.09 (d, *J* = 7.5 Hz, 1H), 7.81 (d, *J* = 8.6 Hz, 1H), 7.59 (d, *J* = 8.4 Hz, 2H), 7.27 (d, *J* = 8.4, 2H), 7.21-7.12 (m, 5H), 6.99 (s, 2H), 6.05-5.99 (m, 1H), 5.41 (d, *J =* 6.9 Hz, 2H), 4.93 (s, 2H), 4.53 (t, *J =* 8.6 Hz, 1H), 4.39-4.33 (m, 2H), 4.19 (t, *J =* 7.7 Hz, 2H), 4.12 (s, 1H), 3.99-3.96 (m, 1H), 3.79-3.74 (m, 1H), 3.60 (m, 2H), 3.51-3.49 (m, 10H), 3.41-3.36 (m, 4H), 3.24 (s, 3H), 3.19 (s, 3H), 3.16-3.11 (m, 4H), 3.07-3.04 (m, 2H), 3.03-2.93 (m, 4H), 2.88-2.84 (m, 4H), 2.28-2.06 (m, 5H), 2.03-1.95 (m, 2H), 1.77-1.71 (m, 6H), 1.64-1.43 (m, 6H), 1.36-1.30 (m, 3H), 1.27-1.25 (m, 2H), 1.20-1.15 (m, 4H), 1.05-1.01 (m, 2H), 0.91-0.74 (m, 26H). ESIMS calculated [M+Na]⁺: 1571.9, observed: 1571.6.

Synthesis of FL-F21N-L183-P209 is similar to synthesis of Example 8, wherein the linker (L) is N-hydroxysuccinimide ester (SMCC), and the drug is Exatecan.

### Example 9: Synthesis of FL-F21N-L163-P204 (Mal-Val-Cit-PEG24-Eribulin)

**Step 9-1:** Compound **48** (0.33 g, 0.45 mmol, 1.0 equiv.) was dissolved in DMAc (3.3 mL). NH₂-PEG24-OH (577.0 mg, 0.54 mmol, 1.2 equiv.) was added to the Compound **48** solution and stirred at 5-10°C for 1 hour. After the completion of the reaction was confirmed by HPLC, the mixture was slowly added to n-heptane/ethyl acetate (5/8, 42 mL) solution to precipitate FL-F21N-L163-P204-Int01. The crude was collected and vacuum-dried to give FL-F21N-L163-P204-Int01 with a yield of 95%.

**Step 9-2:** FL-F21N-L163-P204-Int01 (700.0 mg, 0.42 mmol, 1.0 equiv.) was dissolved in DMAc (10.5 mL) and DIPEA (270.0 mg, 2.10 mmol, 5.0 equiv.) was added thereto. The mixture was cooled down to 5-10°C, and B4NPC (509.0 mg, 1.68 mmol, 4.0 equiv.) was added thereto. The mixture was stirred at 5-10°C for 30 minutes and followed by stirring at 15-25°C for 3 hours. After the completion of the reaction was confirmed by HPLC, n-heptane/ethyl acetate (7/4, 110mL) solution was added to the mixture and the mixture was stirred at 15-25°C for 1 hour to precipitate crude FL-F21N-L163-P204-Int01. The crude was collected and vacuum-dried to give FL-F21N-L163-P204-Int02 with a yield of 64%.

**Step 9-3:** FL-F21N-L163-P204-Int02 (490.0 mg, 0.27 mmole, 1.0 equiv.) was dissolved in DMAc (5.0 mL) and Eribulin (243.0 mg, 0.33 mmol, 1.25 equiv.) was added thereto. The solution was stirred at 0-5°C for 2 hours. After the completion of the reaction was confirmed by HPLC, n-heptane/ethyl acetate ((1/1, 49mL) solution was added in the solution to precipitate crude FL-F21N-L163-P204. The crude was collected and purified by HILIC to afford FL-F21N-L163-P204 with a yield of 19%.

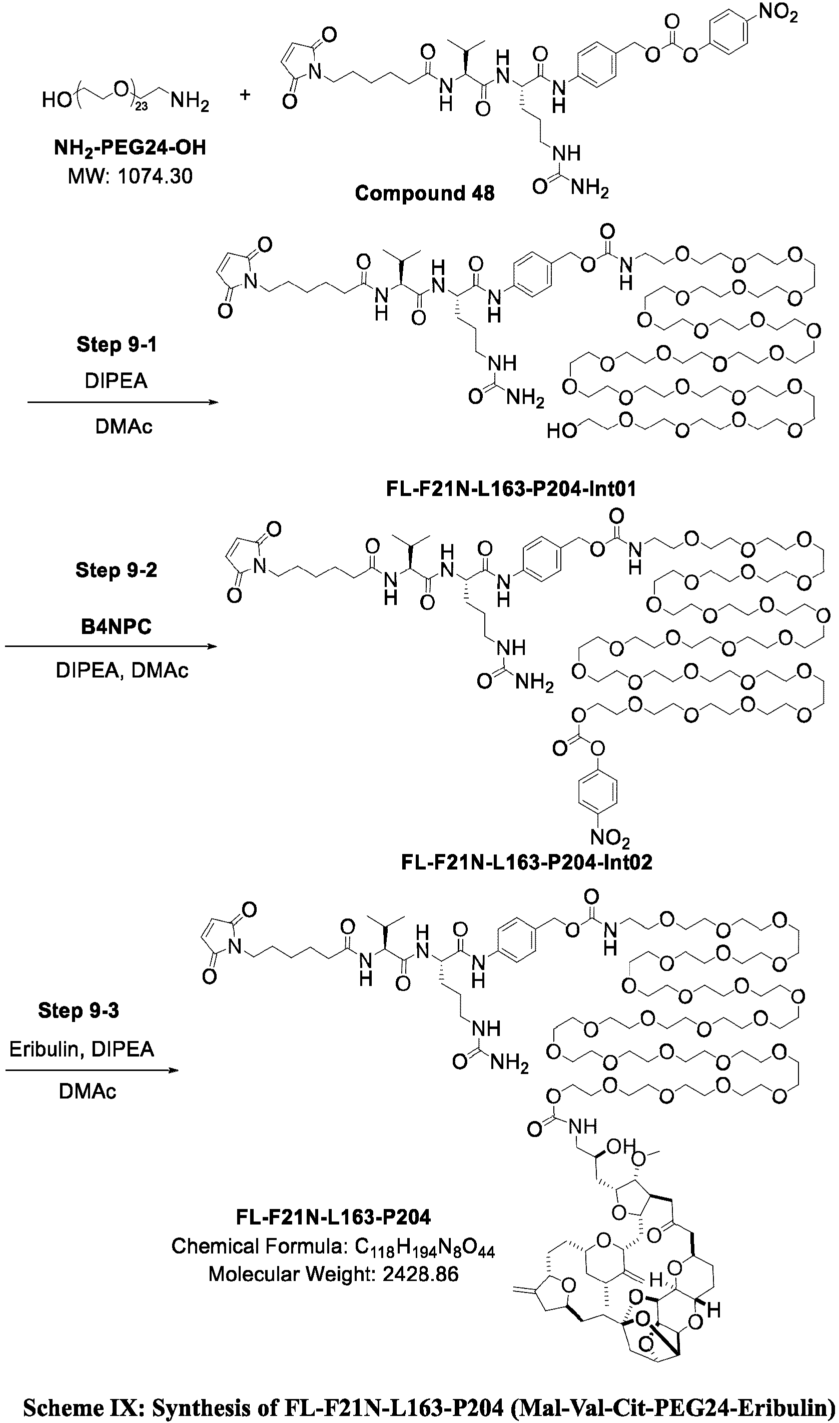

¹H NMR (600 MHz, DMSO-*d*₆) *δ* 9.97 (s, 1H), 8.06 (d, *J* = 7.5 Hz, 1H), 7.79 (d, *J =* 8.7 Hz, 1H), 7.58 (d, *J* = 8.3 Hz, 2H), 7.27 (d, *J* = 8.3 Hz, 2H), 7.20 (t, *J* = 5.8 Hz, 1H), 7.05 (t, *J* = 5.8 Hz, 1H), 7.00 (s, 2H), 5.96 (t, *J* = 5.8 Hz, 1H), 5.40 (s, 2H), 5.05 (s, 1H), 5.00 (s, 1H), 4.93 (s, 2H), 4.83 (s, 1H), 4.75 (s, 1H), 4.64-4.62 (m, 1H), 4.55 (t, *J =* 4.3 Hz, 1H), 4.51 (d, *J =* 5.2 Hz, 1H), 4.40-4.36 (m, 1H), 4.26 (d, *J* =10.8 Hz, 1H), 4.20-4.15 (m, 2H), 4.11-4.08 (m, 3H), 4.05-4.01 (m, 3H), 3.82-3.78 (m, 1H), 3.77-3.74 (m, 1H), 3.70-3.67 (m, 1H), 3.63-3.61 (m, 1H), 3.55 (t, *J =* 4.8 Hz, 2H), 3.50 (s, 82H), 3.40 (t, *J =* 6.0 Hz, 2H), 3.36 (t, *J* = 7.1 Hz, 2H), 3.26 (s, 3H), 3.14-3.12 (m, 2H), 3.04-2.92 (m, 5H), 2.84 (d, *J* = 9.8 Hz, 1H), 2.78-2.67 (m, 3H), 2.61-2.54 (m, 2H), 2.38-2.09 (m, 10H), 2.03-1.92 (m, 7H), 1.72-1.58 (m, 7H), 1.53-1.43 (m, 7H), 1.34-1.15 (m, 11H), 1.03 (d, *J =* 6.4 Hz, 3H), 1.01-0.95 (m, 2H), 0.85 (d, *J* = 6.7 Hz, 3H), 0.82 (d, *J* = 6.7 Hz, 3H). ESI MS calculated [M+H]⁺: 2428.86, observed: 2429.57.

### Example 10: Synthesis of FL-F21N-L165-P205 (SMCC-(PEG4-MMAE)₃)

**Step 10-1:** TBSCl (332 mg, 2.2 mmol) and imidazole (313 mg, 4.6 mmol) were dissolved in DMF (2.5 mL), and 3-amino-3-(2-hydroxyethyl)pentane-1,5-diol (100 mg, 0.6 mmol) was added thereto. The mixture was stirred at room temperature for 1.5 hours. After the reaction was complete, the mixture was added with water and extracted three times with dichloromethane. The organic layers were combined, dehydrated with anhydrous sodium sulfate, filtered, vacuum-dried and purified by silica gel to give FL-F21N-L165-P205-Int01 with a yield of 78%.

**Step 10-2:** 4-(N-Maleimidomethyl)cyclohexanecarboxylic acid N-hydroxysuccinimide ester (SMCC) (1.0 g, 4.2 mmol) and HATU (1.92 g, 5.0 mmol) were dissolved in DMF (15 mL). DIPEA (1.47 ml, 8.4 mmol) and FL-F21N-L165-P205-Int01 (2.1 g, 4.2 mmol) were added thereto and the mixture was stirred at room temperature for 22 hours. After the reaction was complete, the mixture was added with a saturated ammonium chloride solution (50 mL) and extracted three times with ethyl acetate (100 mL*3). The organic layers were combined, extracted with saturated saline, dehydrated with anhydrous sodium sulfate, filtered, concentrated, and finally purified by silica gel to give F21N-L165-P205-Int02 with a yield of 59%.

**Step 10-3:** F21N-L165-P205-Int02 (1.8 g, 2.5 mmol) was dissolved in acetic acid/tetrahydrofuran/water (3:2:2, 126 mL) solution and stirred at room temperature for 14 hours. After the reaction was complete, the reaction solution was dried under vacuum and then purified by silica gel to give F21N-L165-P205-Int03 with a yield of 42%.

**Step 10-4:** F21N-L165-P205-Int03 (400 mg, 1.05 mmol) and B4NPC(1.91 g, 6.28 mmol) were dissolved in DMF (17 mL). DIPEA (1.1 mL, 6.31 mmol) was added thereto and the mixture was stirred at room temperature for 13 hours. After the reaction was complete, the reaction solution was dried under vacuum and then purified by silica gel to give F21N-L165-P205-Int04 with a yield of 87%.

**Step 10-5:** 2-(2-(2-(2-aminoethoxy)ethoxy)ethoxy)ethan-1-ol (538 mg, 2.78 mmol) and F21N-L165-P205-Int04 (800 mg, 0.91 mmol) were dissolved in dichloromethane (47 mL). Triethylamin (NEts) (0.77 mL, 5.52 mmol) was added thereto and the mixture was stirred at room temperature for 17 hours. After the reaction was complete, the reaction solution was dried under vacuum and then purified by silica gel to give F21N-L165-P205-Int05 with a yield of 44%.

**Step 10-6:** F21N-L165-P205-Int05 (420 mg, 0.40 mmol) and B4NPC (738 mg, 2.42 mmol) were dissolved in DMF (7 mL). DIPEA (0.42 mL, 2.42 mmol) was added thereto and the mixture was stirred at room temperature for 11 hours. After the reaction was complete, the reaction solution was dried under vacuum and then purified by silica gel to give F21N-L165-P205-Int06 with a yield of 60%.

**Step 10-7:** MMAE (566 mg, 0.79 mmol), FL-F21N-L165-P205-Int06 (370 mg, 0.24 mmol) and HOBT (106 mg, 0.79mmol) were dissolved in DMF (20 mL). DIPEA (0.28 mL, 1.61 mmol) was added thereto and the mixture was stirred at room temperature for 39 hours. After the reaction was complete, the reaction solution was dried under vacuum and then purified by pre-HPLC to give FL-F21N-L165-P205 with a yield of 38%.

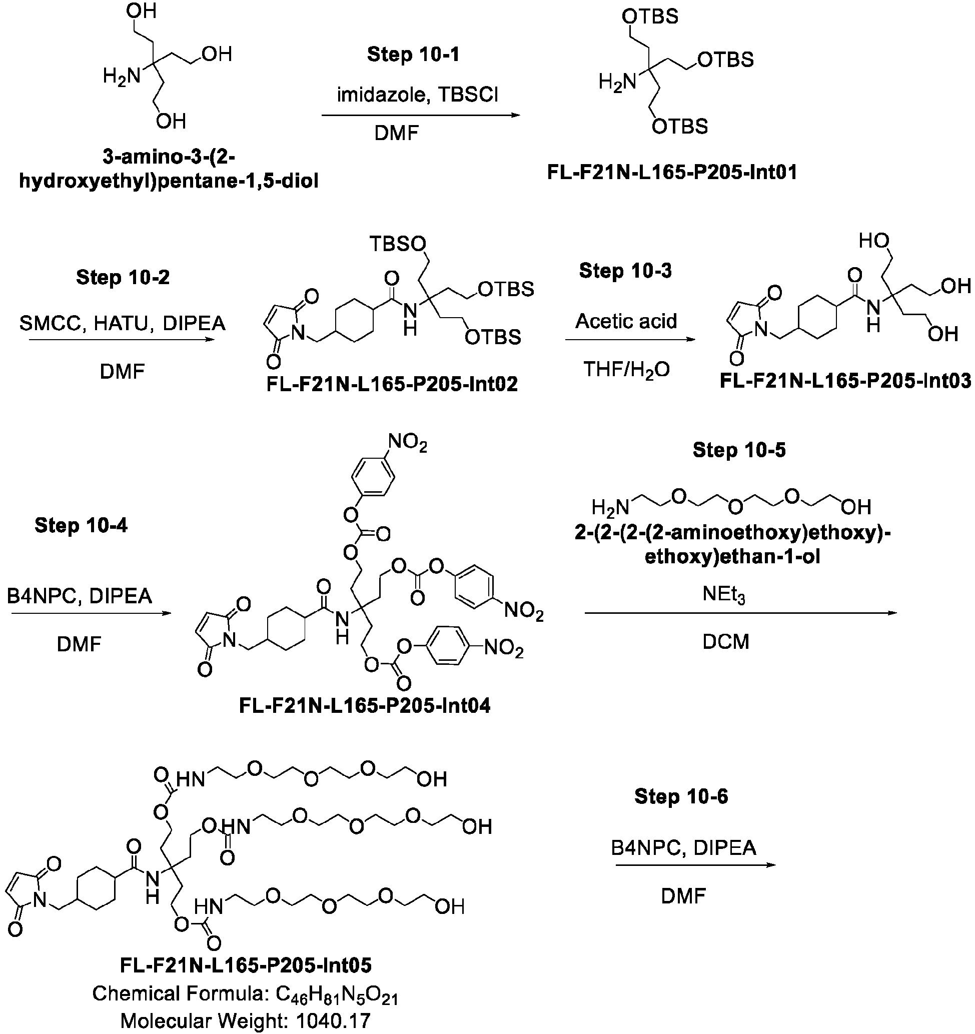

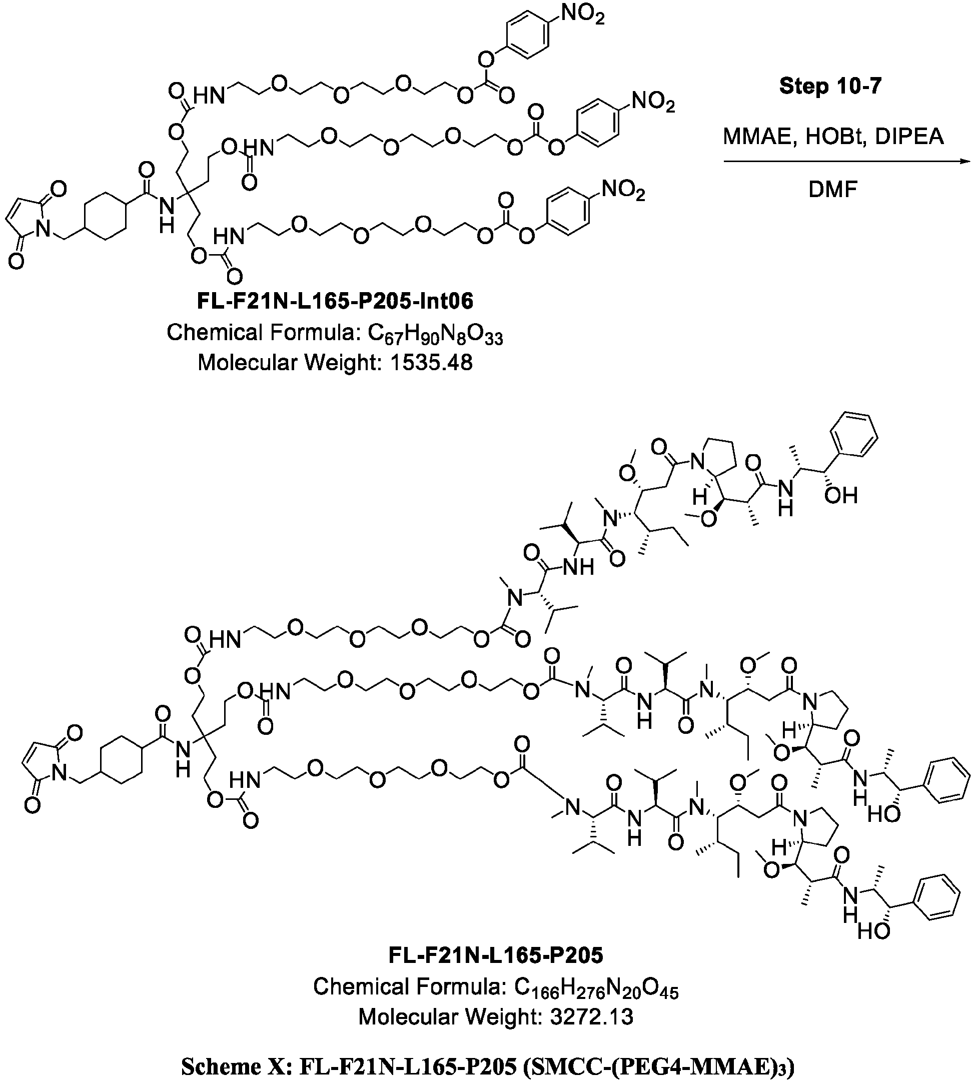

¹H NMR (600 MHz, *CDCl₃*) *δ* 7.43 - 7.29 (m, 12H), 7.24 - 7.23 (m, 3H), 6.83 (br, 2H), 6.69 (s, 2H), 6.54 (br, 2H), 5.91 (br, 1H), 5.72 (br, 1H), 5.56 (br, 2H), 5.05 (br, 1H), 4.94 (s, 2H), 4.85 (br, 1H), 4.78 - 4.63 (m, 5H), 4.31 - 4.08 (m, 26H), 3.85 (d, *J =* 8.0 Hz, 2H), 3.75 - 3.57 (m, 30H), 3.52 - 3.48 (m, 10H), 3.42 - 3.37 (m, 12H), 3.33 - 3.31 (m, *J =* 14.3 Hz, 16H), 3.12 (br, 2H), 3.01 (br, 6H), 2.92 - 2.87 (m, 7H), 2.84 - 2.80 (m, 1H), 2.75 (br, 1H), 2.61 (br, 1H), 2.49 - 2.43 (m, 3H), 2.40 - 2.33 (m, 4H), 2.27 - 1.98 (m, 21H), 1.83 - 1.79 (m, 7H), 1.71 - 1.66 (m, 6H), 1.48 - 1.36 (m, 6H), 1.28 -1.21 (m, 10H), 1.09 - 0.76(m, 71H). ESI-MS calculated [M+2Na]²⁺: 1658, observed: 1659.

### Example 11: Synthesis of FL-F21N-L152-P204 (Mal-Val-Cit-(PEG37)(Eribulin))

**Step 11-1:** Fmoc-L-Lys(Boc)-OH (2.0 g, 4.2 mmol) was dissolved in DMF (22 mL) and potassium carbonate (1.8 g, 12.8 mmol) was added thereto. The reaction solution was cooled down to 0°C and then potassium iodide (0.8 mL, 12.8 mmol) was slowly added thereto. After the addition was complete, the reaction temperature was returned to room temperature and the solution was stirred for 2 hours. Once the reaction was complete, the reaction was quenched by adding saturated saline (100 mL). The mixture was extracted with ethyl acetate (100 mL*3). The organic layers were combined, extracted with a saturated sodium bicarbonate solution, dehydrated with anhydrous sodium sulfate, and then filtered and vacuum-dried. The vacuum-dried residue was dissolved in in DMF (10 mL), mixed with piperidine (2.5 mL), and stirred at room temperature for 0.5 hours. Finally, the reaction solution was dried under vacuum and purified by silica gel to give Compound A with a yield of 90%.

**Step 11-2:** Compound **A** (910 mg, 3.5 mmol), sodium 4-hydroxybutanoate (537 mg, 4.2 mmol) and HATU (1.61 g, 4.2 mmol) were dissolved in DMF (16 mL). DIPEA (0.73 mL, 4.2 mmol) was added thereto and the mixture was stirred at room temperature for 0.5 hours. After the reaction was complete, the mixture was added with saturated saline (50 mL) and extracted with ethyl acetate (100 mL*3). The organic layers were combined, dehydrated with anhydrous sodium sulfate, filtered and concentrated to afford crude Compound **B.** The crude was purified by silica gel so as to give Compound **B** with a yield of 81%.

**Step 11-3:** Compound **B** (980 mg, 2.8 mmol) was dissolved in tetrahydrofuran/water (THF/H₂O 1: 1, 26 mL). LiOH (204 mg, 8.5 mmol) was added thereto and the mixture was stirred at room temperature for 19 hours. After the reaction was complete, the mixture was evaporated to remove tetrahydrofuran, and extracted with dichloromethane (50 mL*2). Next, the water layer was acidified with 1N HCl, and then extracted with ethyl acetate (100 mL*3). The organic layer was collected, dehydrated with anhydrous sodium sulfate, and then filtered and concentrated so as to give Compound **C** with a yield of 90%.

**Step 11-4:** 2,2'-oxybis(ethan-1-amine) (716 mg, 6.9 mmol) was dissolved in dichloromethane (6 mL) and cooled down to 0°C. Boc₂O (500 mg, 2.3 mmol) was dissolved in dichloromethane (2 mL) and then was then added dropwise into the reaction solution. After the dropwise addition was complete, the reaction solution was stirred at room temperature for 30 hours. After the reaction was complete, the reaction solution was extracted with a saturated sodium bicarbonate solution (20 mL) and dichloromethane (30 mL*3). The organic layer was combined, dehydrated with anhydrous sodium sulfate, filtered, and concentrated to give crude FL-F21N-L152-P204-Int01. The crude was purified by silica gel to give FL-F21N-L152-P204-Int01 with a yield of 53%.

**Step 11-5:** FL-F21N-L152-P204-Int01 and DIPEA (0.94 mL, 5.4 mmol) were dissolved in DMF (26 mL). Compound **48** (2.0 g, 2.7 mmol) was added thereto and the mixture was stirred at room temperature for 18 hours. After the reaction was complete, the reaction solution was dried under vacuum and then purified by silica gel so as to give FL-F21N-L152-P204-Int02 with a yield of 88%.

**Step 11-6:** FL-F21N-L152-P204-Int02 (600 mg, 0.75 mmol) was dissolved in dichloromethane (4.5 mL). TFA (1.5 mL) was added thereto and the mixture was stirred at room temperature for 0.5 hours. After the reaction was complete, toluene/ethyl acetate (1:1, 10 mL × 3) was added thereto and the mixture was concentrated to give a TFA salt of FL-F21N-L152-P204-Int02. Compound C (248 mg, 0.75 mmol), DIPEA (0.65 mL, 3.73 mmol) and HATU (423 mg, 1.11 mmol) were dissolved in DMF (8 mL). Further, the TFA salt of FL-F21N-L152-P204-Int02 was dissolved in DMF (7 mL) and added into the mixture of Compound C with stirring at room temperature for 0.5 hours. After the reaction was complete, ethyl acetate was added in the mixture to precipitate crude FL-F21N-L152-P204-Int03. The crude was collected and purified using silica gel and pre-HPLC so as to give FL-F21N-L152-P204-Int03 with a yield of 32%.

**Step 11-7:** FL-F21N-L152-P204-Int03 (225 mg, 0.22 mmol) was dissolved in dichloromethane (18 mL) and mixed with TFA (2.0 mL). The reaction solution was cooled down to 0°C with stirring for 45 minutes. After the reaction was complete, ethyl acetate (10 mL × 3) was added thereto and the mixture was concentrated to give a TFA salt of FL-F21N-L152-P204-Int03. m-PEG37-acid (378 mg, 0.22 mmol), DIPEA (0.39 mL, 2.2 mmol) and HATU (85 mg, 0.22 mmol) were dissolved in DMF (12 mL). Further, the FL-F21N-L152-P204-Int03 TFA salt was dissolved in DMF (10 mL) and added into the mixture of m-PEG37-acid with stirring at room temperature for 18 hours. After the reaction was complete, the reaction solution was concentrated and purified using pre-HPLC so as to give FL-F21N-L152-P204-Int04 with a yield of 49%.

**Step 11-8:** FL-F21N-L152-P204-Int04 (250 mg, 0.096 mmol) and B4NPC (105 mg, 0.345 mmol) were dissolved in DMF (2.5 mL). DIPEA (0.19 mL, 1.076 mmol) was added thereto and the mixture was stirred at room temperature for 24 hours. Next, Eribulin (70 mg, 0.096 mmol) was added thereto and the mixture was continuously stirred at room temperature. After 1.5 hours, Eribulin (70 mg, 0.096 mmol) and DIPEA (0.063 mL, 0.36 mmol) were added thereto. After 3 hours, Eribulin (52 mg, 0.071 mmol) was added thereto and the mixture was stirred at room temperature for 2 hours. After the reaction was complete, the reaction solution was dried under vacuum and then purified by pre-HPLC so as to give FL-F21N-L152-P204 with a yield of 64%.

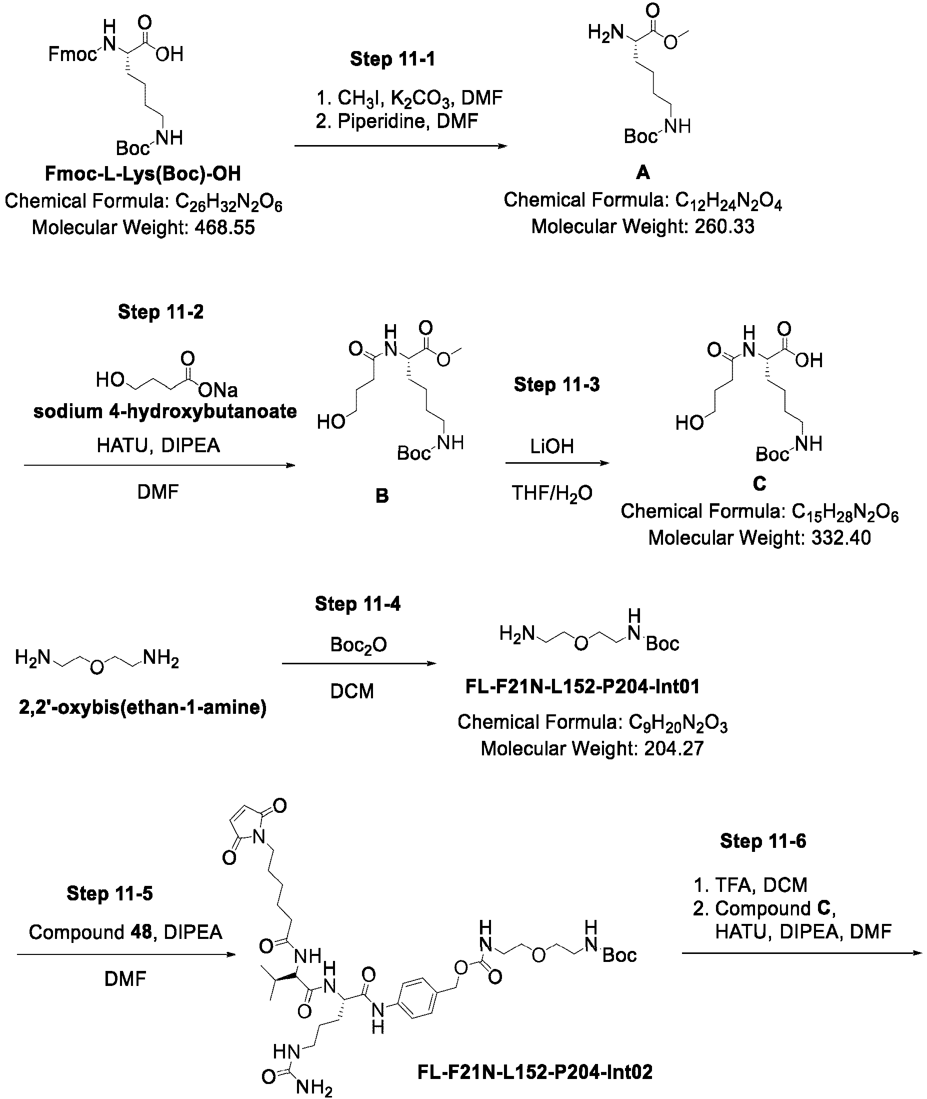

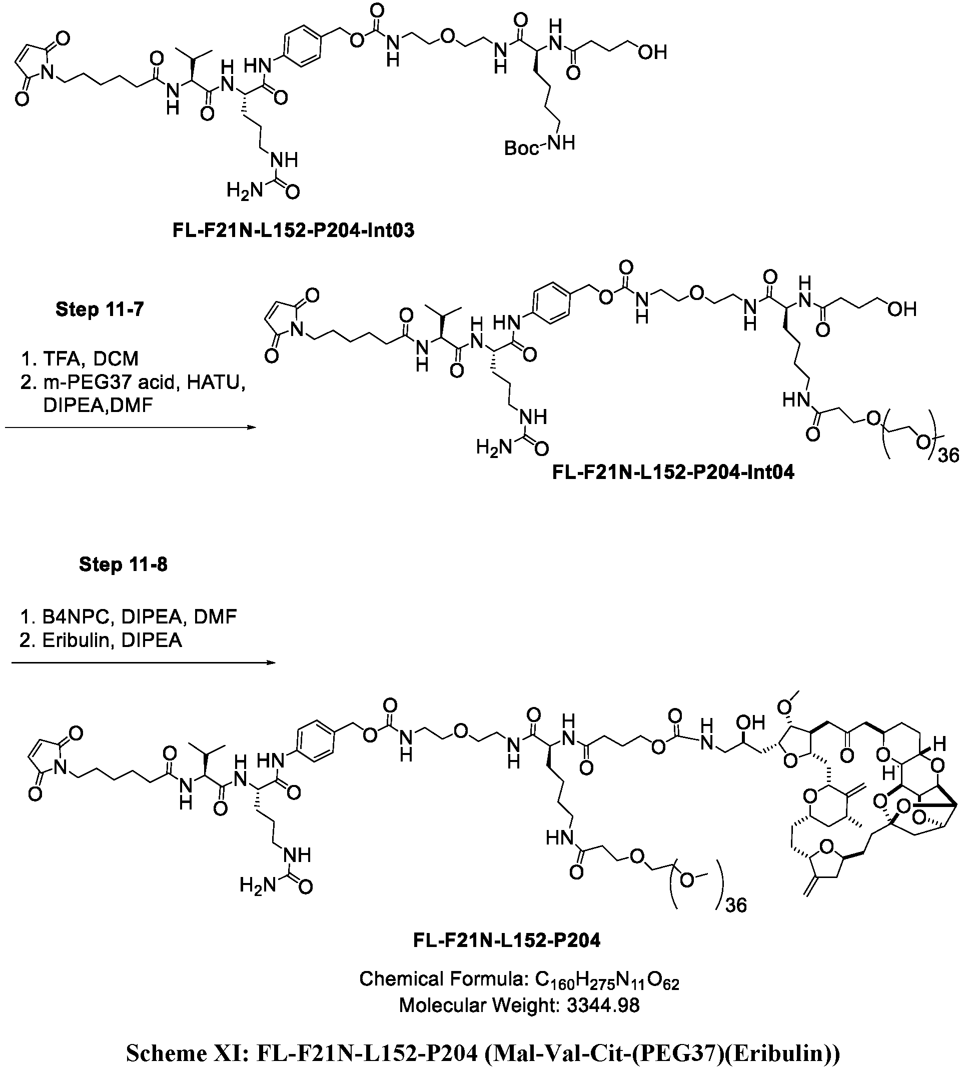

¹H-NMR (600 MHz, Methanol-*d₄*) *δ* 7.58 (d, *J =* 8.3 Hz, 2H), 7.31 (d, *J =* 8.3 Hz, 2H), 6.80 (s, 2H), 5.16-5.12 (m, 1H), 5.09-4.99 (m, 3H), 4.87 (s, 1H), 4.82 (s, 1H), 4.71 (t, *J* = 4.3 Hz, 1H), 4.61 (t, *J* = 4.3 Hz, 1H), 4.51 (dd, *J* = 8.7 Hz, *J* = 5.0 Hz, 1H), 4.47 (d, *J =* 10.8 Hz, 1H), 4.33-4.23 (m, 3H), 4.20-4.16 (m, 2H), 4.14-4.04 (m, 3H), 4.03-3.95 (m, 2H), 3.90-3.82 (m, 3H), 3.76-3.69 (m, 6H), 3.69-3.57 (m, 144 H), 3.55-3.46 (m, 6H), 3.40 (s, 3H), 3.38-3.32 (m, 5H), 3.30-3.26 (m, 2H), 3.25-3.08 (m, 6H), 2.95-2.84 (m, 2H), 2.76-2.64 (m, 2H), 2.48-2.40 (m, 4H), 2.40-2.25 (m, 7H), 2.24-2.15 (m, 2H), 2.14-1.83 (m, 6H), 1.95-1.83 (m, 5H), 1.82-1.68 (m, 6H), 1.67-1.28 (m, 19H), 1.10 (d, *J=* 6.3 Hz, 3H), 1.06-0.99 (m, 1H), 0.99-0.95 (m, 6H). ESI-MS calculated [M+2Na]²⁺: 1696, observed: 1696.

Synthesis of FL-F21N-L168-P204, FL-F21N-L169-P204, FL-F21N-L170-P204, FL-F21N-L171-P204 and FL-F21N-L172-P204 are similar to synthesis of Example 11.

The synthesis pathway for FL-F21N-L168-P204 is planned as follows:

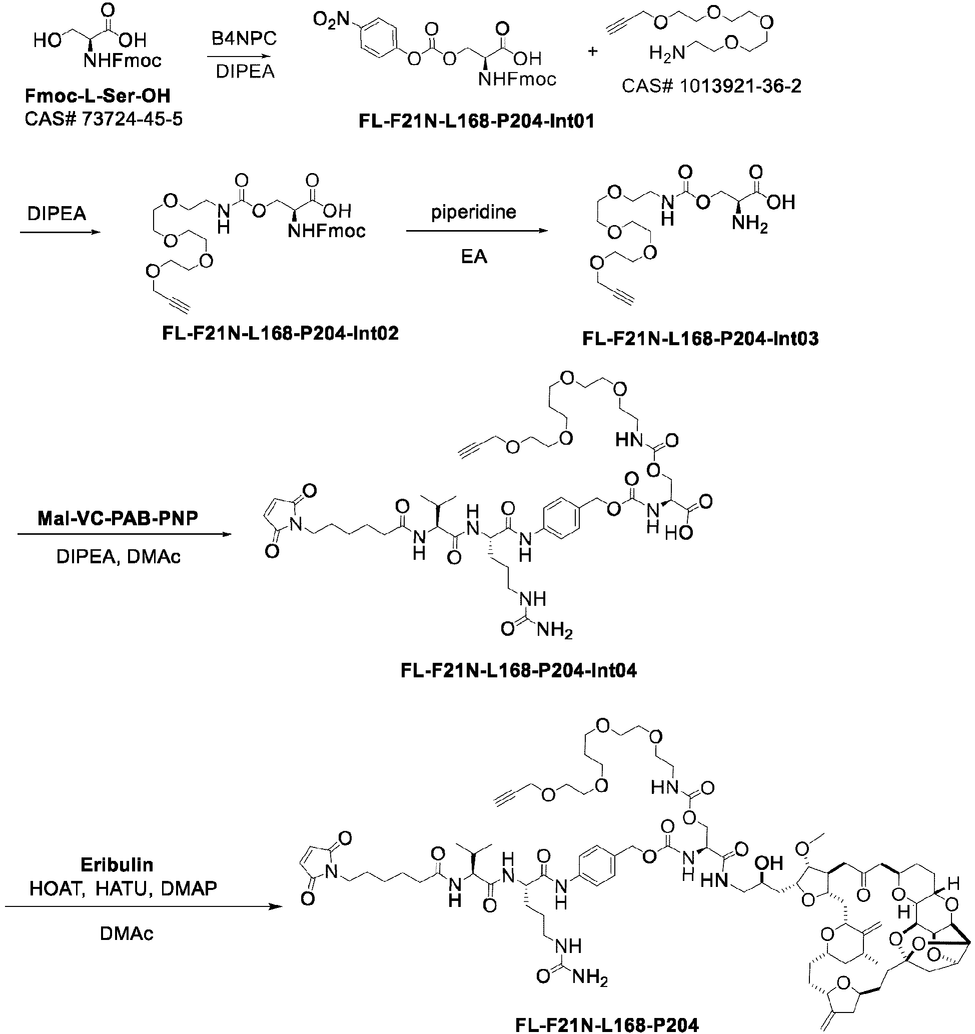

The synthesis pathway for FL-F21N-L169-P204 is planned as follows:

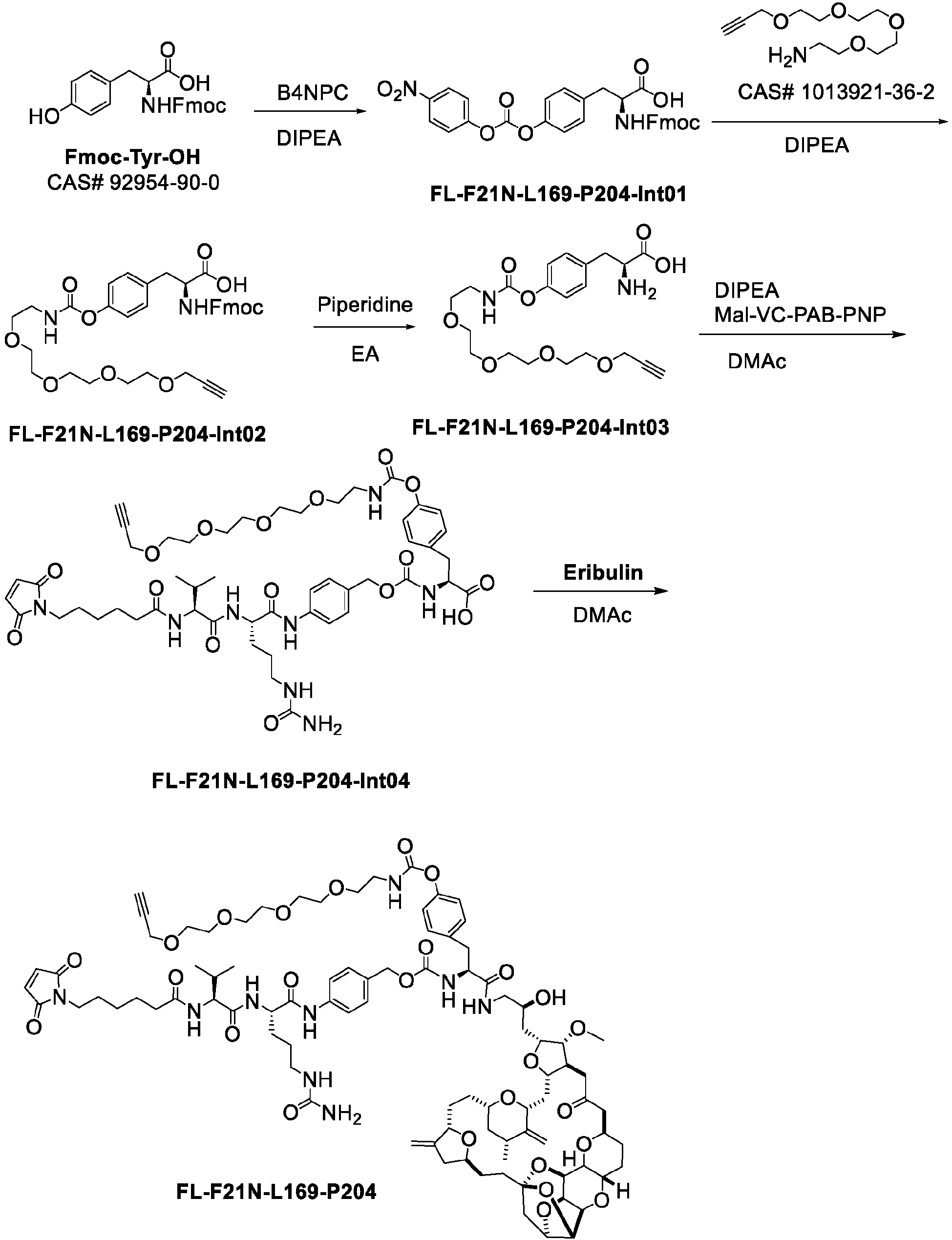

The synthesis pathway for FL-F21N-L172-P204 is planned as follows:

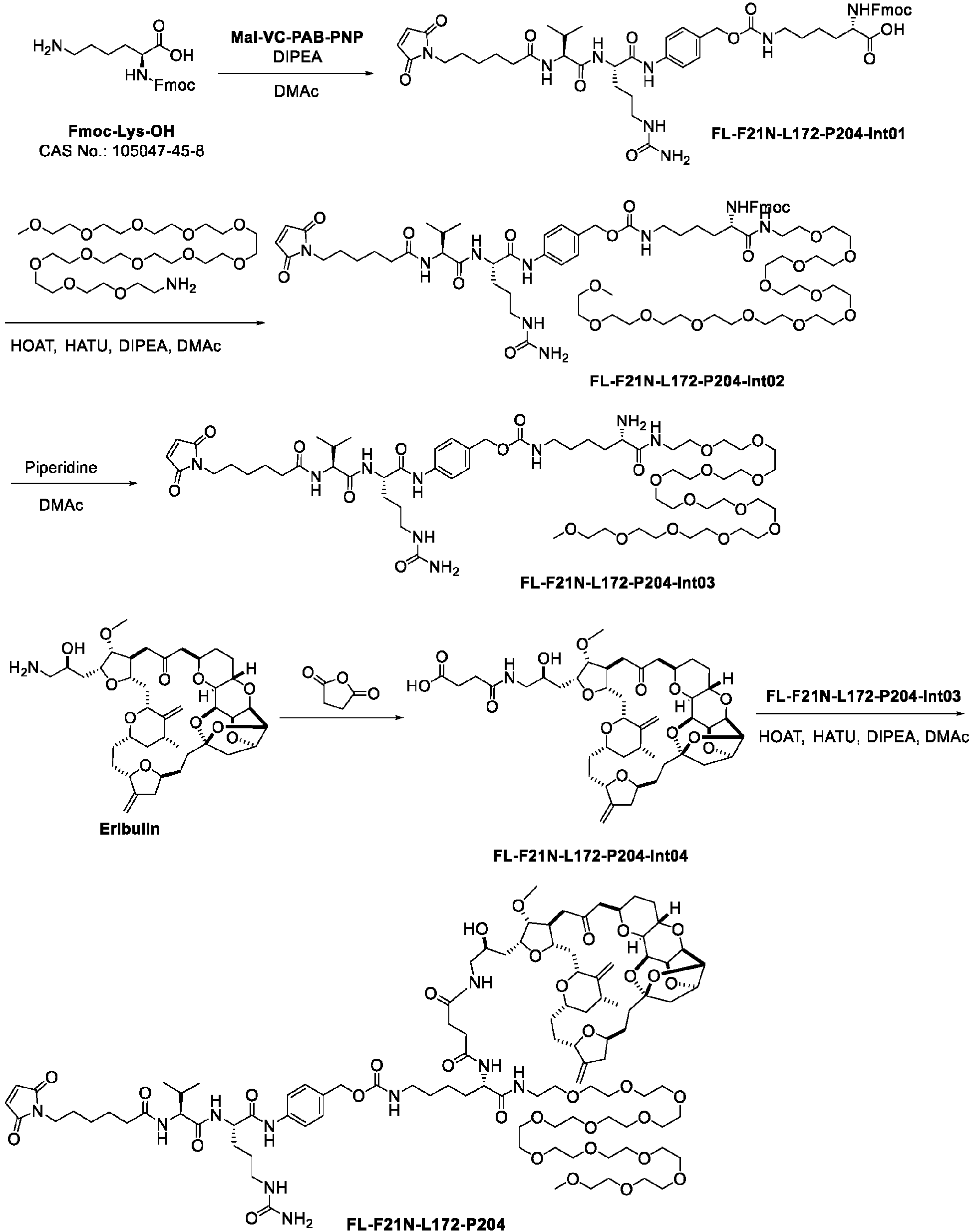

### Example 12: Synthesis of FL-F21N-L173-P204 (N-(PEG1-acid)-lys-amido-Mal-(PEG37)(Eribulin))

**Step 12-1:** mPEG37-acid (321 mg, 0.189 mmol, 1.0 equiv.) was dissolved in DMAc (3 mL). After adding HOAt (28.5 mg, 0.209 mmol, 1.1 equiv.) and HATU (80.0 mg, 0.209 mmol, 1.1 equiv.), DIPEA (73.7 mg, 0.57 mmol, 3.0 equiv.) was added thereto and the mixture was stirred at room temperature for 4 hours. Further, N-(PEG1-acid)-L-lys-amido-Mal TFA salt (100 mg, 0.189 mmol, 1.0 equiv.) was dissolved in DMAc (1 mL), and then slowly added into the solution of mPEG37-acid with stirring at room temperature for 30 minutes. The 1:1 mixture of ethyl acetate and n-heptane (40 mL) was added to precipitate crude FL-F21N-L173-P204-Int01. The crude was collected, washed once with the 1:1 mixture of ethyl acetate and n-heptane (20 mL), and vacuum-dried to give FL-F21N-L173-P204-Int01 with a yield of 86.3%.

**Step 12-2:** FL-F21N-L173-P204-Int01 (100 mg, 0.0479 mmol, 1.0 equiv.) was dissolved in DMAc (4 mL). After adding HOAt (7.1 mg, 0.0527 mmol, 1.1 equiv.) and HATU (20.0 mg, 0.0527 mmol, 1.1 equiv.), DIPEA (12.4 mg, 0.0958 mmol, 2.0 equiv.) was added thereto and the mixture was stirred at room temperature for 30 minutes. Next, Eribulin (35.0 mg, 0.0479 mmol, 1.0 equiv.) was added thereto and the mixture was stirred at room temperature for 30 minutes. After the completion of the reaction was confirmed, the reaction solution was dried under vacuum and then purified by HILIC so as to give FL-F21N-L173-P204 with a yield of 41.6%.

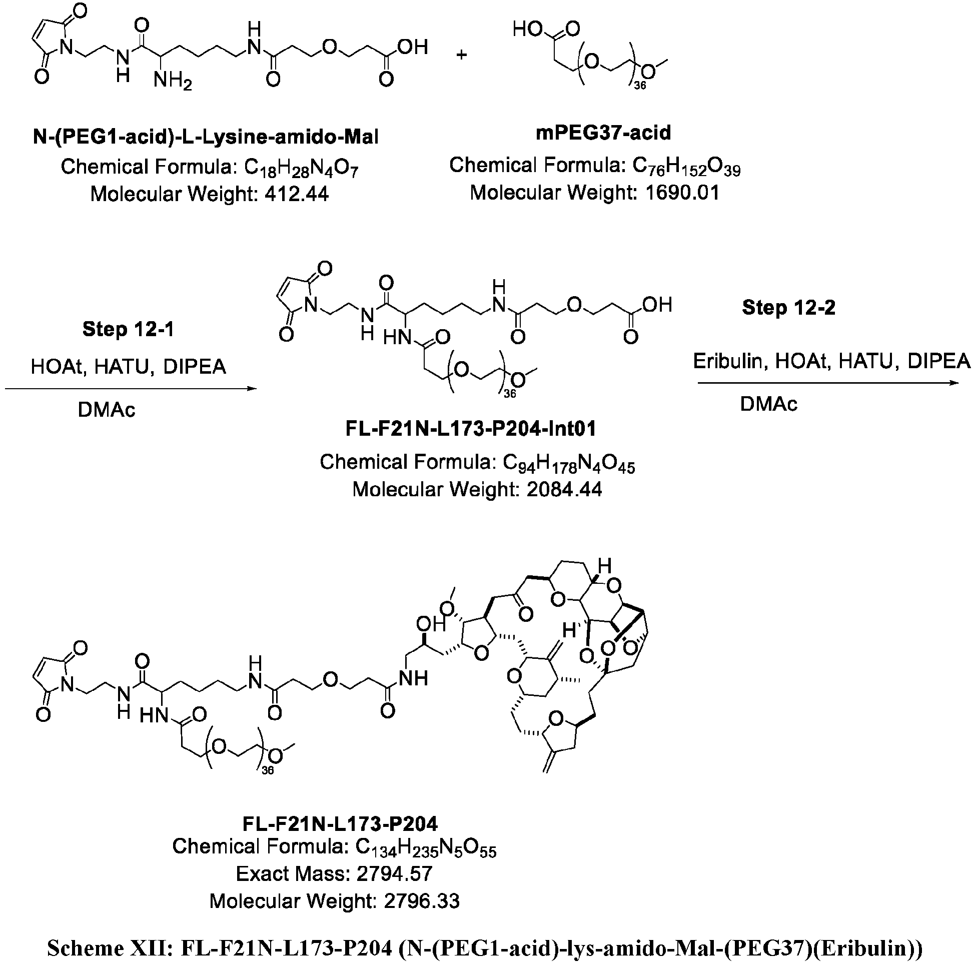

¹H-NMR (400MHz, DMSO-*d*₆) *δ* 7.94 (t, *J* = 5.9 Hz, 1H), 7.86 (d, *J* = 8.1 Hz, 1H), 7.76-7.74 (m, 2H), 6.99 (s, 2H), 5.05 (s, 1H), 4.99 (s, 1H), 4.82 (s, 1H), 4.75 (s, 1H), 4.64-4.62 (m, 1H), 4.58 (d, *J* = 5.0 Hz, 1H), 4.55 (t, *J* = 4.1 Hz, 1H), 4.32 (d, *J* = 4.2 Hz, 1H), 4.27-4.24 (m, 1H), 4.19-4.02 (m, 6H), 3.82-3.72 (m, 4H), 3.69-3.66 (m, 3H), 3.58-3.41 (m, 152H), 3.25 (s, 3H), 3.23 (s, 3H), 3.15-3.12 (m 2H), 3.05-2.97 (m, 4H), 2.83 (d, *J =* 10.0 Hz, 1H), 2.74-2.66 (m, 3H), 2.38-2.20 (m, 10H), 2.00-1.92 (m, 6H), 1.72-1.46 (m, 9H), 1.37-1.25 (m, 7H), 1.18-1.16 (m, 4H), 0.86-0.82 (m, 3H). ESI MS calculated [M+2Na]²⁺: 1420.2, observed: 1420.9.

### Example 13: Synthesis of FL-F21N-L166-P204 (Mal-Val-Cit-PAB-(PEG4-(Eribulin)₂)₂)

**Step 13-1:** FL-F21N-L106-P204-Int04 (200 mg, 0.14 mmol) was dissolved in DMAc (8 mL). Further, 2-aminopropane-1,3-diol (250 mg, 0.27 mmol) was dissolved in DMAc (2 mL), mixed with DIPEA (35 mg, 0.27 mmol), and then added into the solution of FL-F21N-L106-P204-Int04. The mixture was reacted at room temperature for 8 hours. After the reaction was complete, the reaction solution was dried under vacuum to remove DMAc so as to give crude FL-F21N-L166-P204-Int05. Then, the crude was washed twice with ethyl acetate/n-heptane (1:1, 40 mL*2) solution, dried, and purified by HILIC to give FL-F21N-L166-P204-Int05 with a yield of 71%.

**Step 13-2:** FL-F21N-L166-P204-Int05 (200 mg, 0.15 mmol) and B4NPC (290 mg, 0.95 mmol) were dissolved in DMAc (2.8 mL). The reaction solution was cooled down to 5-10°C and then DIPEA (123 mg, 0.95 mmol) was added thereto. After the reaction was complete, the reaction solution was concentrated to one-fourth of its original volume, then cooled down to 15°C, and mixed ethyl acetate/n-heptane (1:1, 40 mL) to precipitate crude FL-F21N-L166-P204-Int06. The crude was collected, washed twice with ethyl acetate/n-heptane (1:1, 40 mL*2) solution, and dried so as to give FL-F21N-L166-P204-Int06 with a yield of 84%.

**Step 13-3:** FL-F21N-L166-P204-Int06 (200 mg, 0.10 mmol) was dissolved in DMAc (8 mL). Further, Eribulin (289 mg, 0.4 mmol) was dissolved in DMAc (2 mL). DIPEA (51 mg, 0.40 mmol) was added thereto and the mixture was stirred at room temperature for 4 hours. After the reaction was complete, DMAc of the mixture was evaporated to one-fourth of its original volume and mixed with ethyl acetate/n-heptane (1:1, 40 mL) to precipitate crude FL-F21N-L1066-P204. Then, the crude was washed twice with ethyl acetate/n-heptane (1:1, 40 mL*2) solution, collected, dried, and purified by HILIC to give FL-F21N-L166-P204 with a yield of 56%.

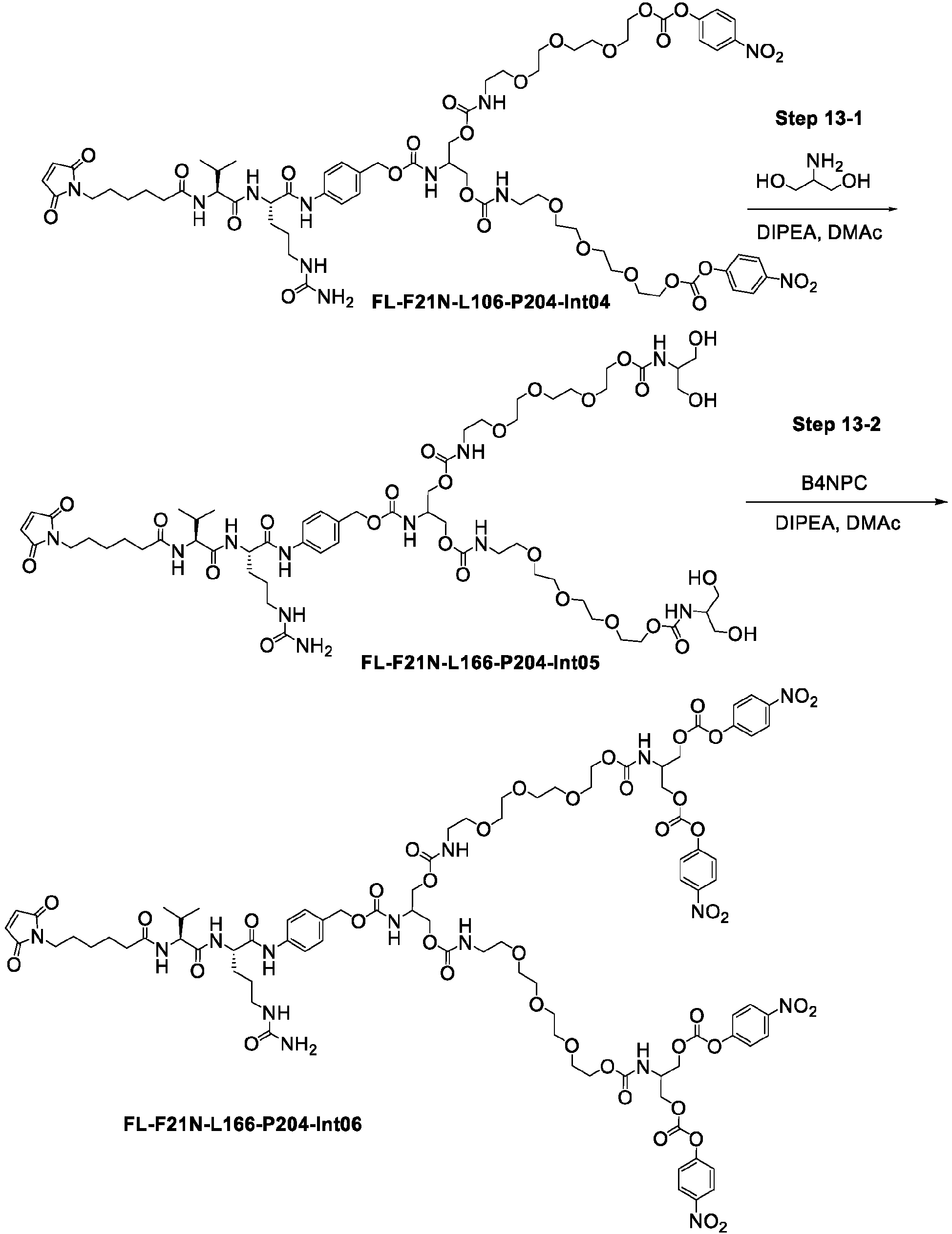

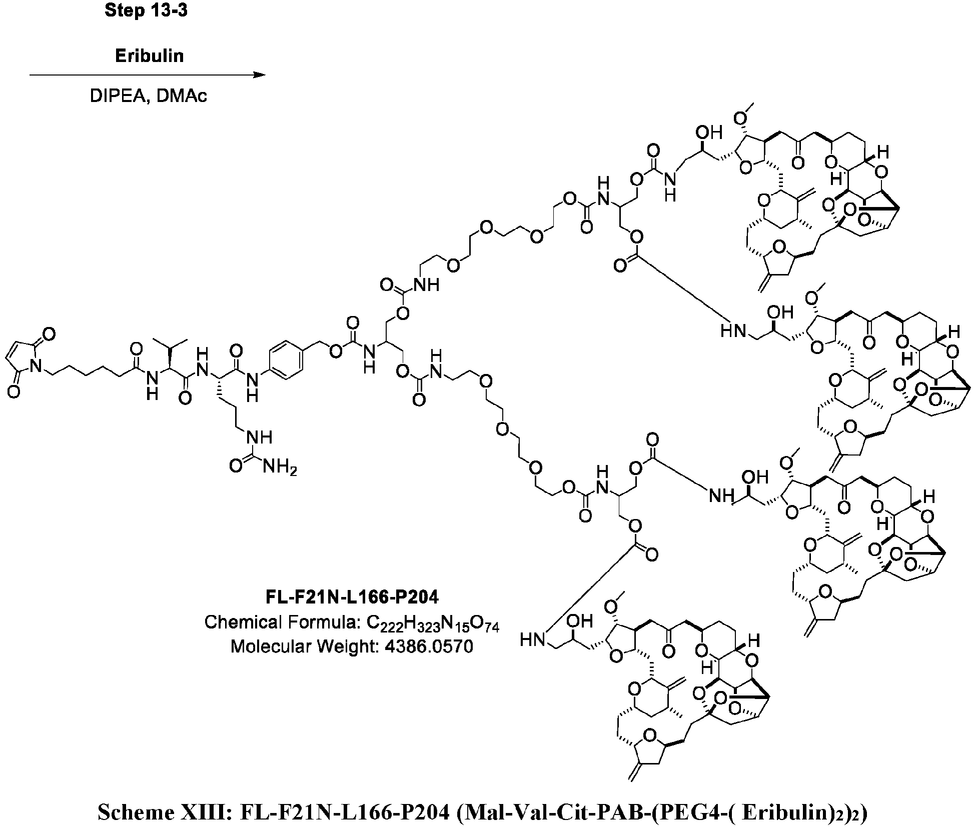

¹H NMR (400 MHz, DMSO-*d*₆) δ 9.96 (s, 1H), 8.10 (s, 1H), 8.05 (d, *J* = 7.4 Hz, 1H), 7.78 (d, *J* = 8.7 Hz, 1H), 7.60 (d, *J* = 8.6 Hz, 2H), 7.35-7.25 (m, 5H), 7.21-7.10 (m, 3H), 7.01-6.95 (m, 6H), 6.63 (s, 1H), 5.97, (t, J = 5.8 Hz, 1H), 5.39 (s, 2H), 5.33 (t, J=4.7 Hz, 1H), 5.06-4.96 (m, 12H), 4.86-4.82 (m, 4H), 4.78-4.73 (m, 4H), 4.65-4.63 (m, 5H), 4.57-4.55 (m, 4H), 4.52-4.50 (m, 5H), 4.30-4.27 (m, 2H), 4.26-4.24 (m, 3H), 4.21-4.15 (m, 6H), 4.12-4.10 (m, 12H), 4.05-3.96 (m, 14H), 3.91-3.87 (m, 5H), 3.83-3.69 (m, 15H), 3.58-3.48 (m, 29H), 3.44-3.36 (m, 6H), 3.28-3.25 (m, 16H), 3.18-3.10 (m, 6H), 2.97-2.94 (m, 22H), 2.89-2.83 (m, 5H), 2.75-2.67 (m, 5H), 2.58-2.54 (m, 5H), 2.34-2.14 (m, 23H), 2.05-1.98 (m, 6H), 1.95-1.90 (m, 16H), 1.76-1.62 (m, 24H), 1.58-1.42 (m, 22H), 1.21-1.14 (m, 4H), 1.04 (d, 6.4 Hz, 12H), 0.88-0.85 (m, 6H). ESIMS calculated [M+3H]³⁺: 1462.1, observed 1462.3.

### Example 14: Synthesis of FL-F21N-L175-P204 (MC-Phe-VC-PEG4-Eribulin)

**Step 14-1:** FL-F21N-L175-P204-Int01 (300 mg, 0.30 mmol) and DIPEA (90 uL, 0.52 mmol) were dissolved in DMAc (5.0 mL), and then mixed with Eribulin (185 mg, 0.25 mmol). After reacting at room temperature for 3 hours, the reaction was further mixed with diethylamine (0.26 mL) and kept at room temperature for additional 15 hours. After the reaction was complete, the reaction solution was dried under vacuum and then purified so as to give FL-F21N-L175-P204-Int02 with a yield of 89%.

**Step 14-2:** FL-F21N-L175-P204-Int02 (150 mg, 0.11 mmol), 4-Maleimidophenylacetic acid N-hydroxysuccinimide ester (45 mg, 0.14 mmol) and HOBt (19 mg, 0.14 mmol) were dissolved in DMAc (4.5 mL), and then mixed with DIPEA (0.038 mL, 0.22 mmol) to conduct the reaction at room temperature for 2 hours. After the reaction was complete, the reaction solution was dried under vacuum and then purified by pre-HPLC so as to give FL-F21N-L175-P204 with a yield of 37%.

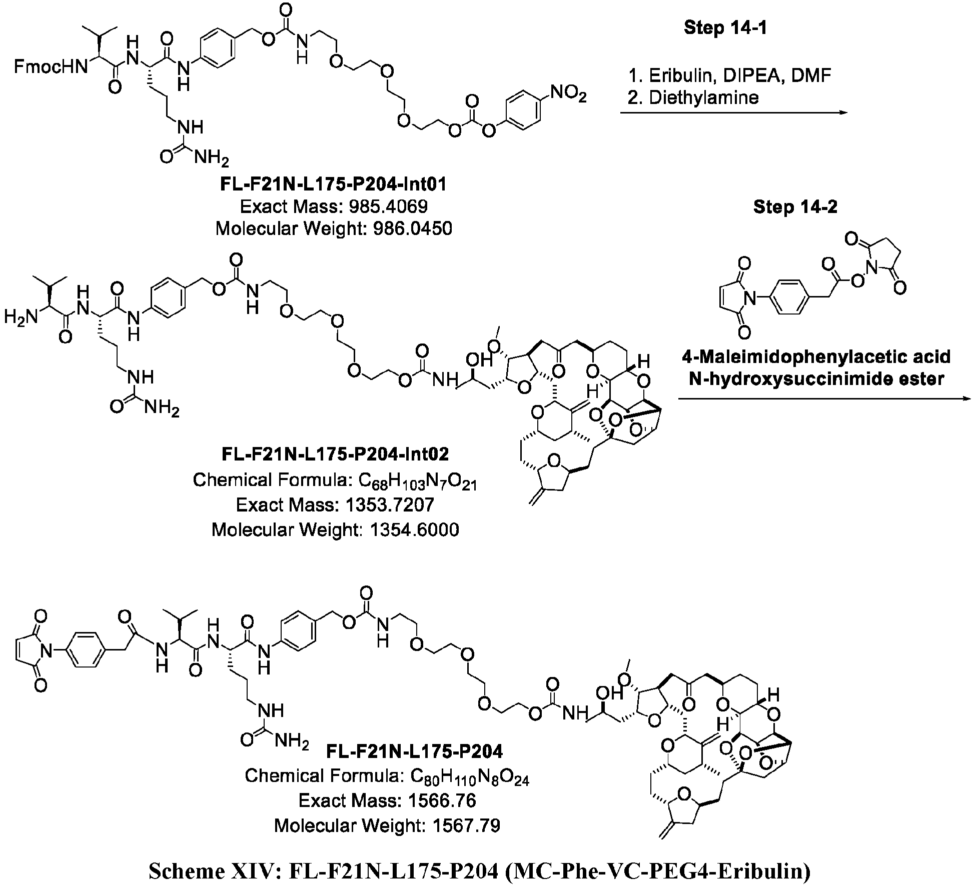

¹H-NMR (600 MHz, methanol*-d4*/*CDCl*₃) δ 7.53 (d, J = 8.3 Hz, 2H), 7.39 (d, J = 8.3 Hz, 2H), 7.30-7.24 (m, 4H), 6.91 (s, 2H), 5.10 (s, 1H), 5.03-4.96 (m, 3H), 4.86 (s, 1H), 4.80 (s, 1H), 4.70 (t, J = 4.5 Hz, 1H), 4.60 (t, J = 4.3 Hz, 1H), 4.51-4.47 (m, 1H), 4.42 (d, J = 11.1 Hz, 1H), 4.34-4.25 (m, 3H), 4.20-4.16 (m, 1H), 4.16-4.10 (m, 3H), 4.10-4.05 (m, 1H), 3.96 (t, J = 10.7 Hz, 1H), 3.89 - 3.71 (m, 5H), 3.70-3.58 (m, 13H), 3.53 (t, J = 5.0 Hz, 2H), 3.40 (s, 3H), 3.30-3.27 (m, 2H), 3.24-3.02 (m, 4H), 2.92-2.84 (m, 2H), 2.74-2.67 (m, 1H), 2.63-2.55 (m, 1H), 2.50-2.28 (m, 5H), 2.27-2.12 (m, 4H), 2.09-1.94 (m, 4H), 1.92 -1.79 (m, 4H), 1.77 -1.65 (m, 4H), 1.60 -1.26 (m, 8H), 1.09 (d, J = 6.5 Hz, 3H), 1.07-1.00 (m, 1H), 0.96-0.92 (m, 6H). ESI MS calculated [M+H]⁺: 1568, observed 1568.

### Example 15: Synthesis of FL-F21N-L176-P204 (BCN-VC-PEG4-Eribulin)

**Step 15-1:** FL-F21N-L176-P204-Int02 (148 mg, 0.11 mmol) and ((1R,8S,9r)-bicyclo[6.1.0]non-4-yn-9-yl)methyl (4-nitrophenyl) carbonate (41 mg, 0.13 mmol) were dissolved in DMF (4.4 mL), and then mixed with DIPEA (0.038 mL, 0.22 mmol) to conduct the reaction at room temperature for 20 hours. After the reaction was complete, the reaction solution was dried under vacuum and then purified by pre-HPLC so as to give FL-F21N-L175-P204 with a yield of 54%.

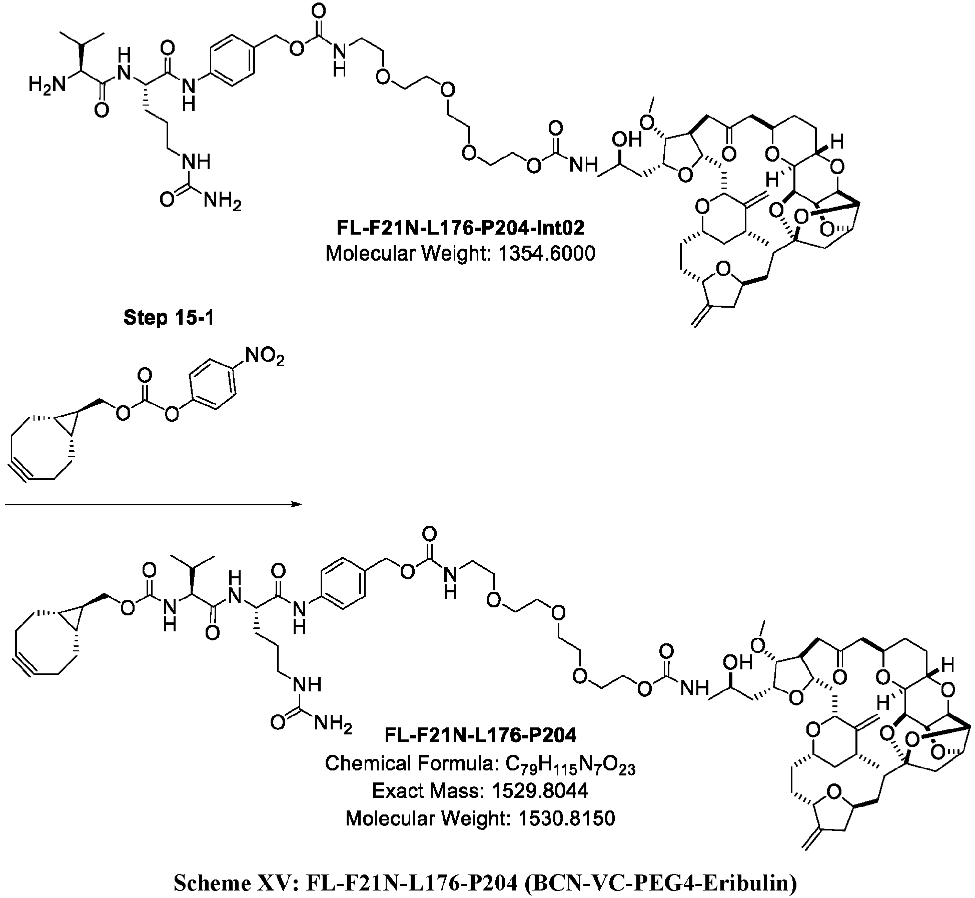

¹H-NMR (600 MHz, CD₃OD) δ 7.58 (d, J = 8.3 Hz, 2H), 7.32 (d, J = 8.3 Hz, 2H), 5.16-5.12 (m, 1H), 5.10-4.99 (m, 3H), 4.87 (s, 1H), 4.82 (s, 1H), 4.71 (t, J = 4.5 Hz, 1H), 4.61 (t, J = 4.3 Hz, 1H), 4.55-4.50 (m, 1H), 4.47 (d, J = 11.0 Hz, 1H), 4.32-4.27 (m, 2H), 4.20-4.07 (m, 7H), 4.00-3.94 (m, 2H), 3.89 -3.81 (m, 3H), 3.76-3.70 (m, 2H), 3.69-3.56 (m, 10 H), 3.55-3.50 (m, 2H), 3.41 (s, 3H), 3.34-3.32 (m, 1H), 3.30-3.28 (m, 2H), 3.25-3.08 (m, 4H), 2.94-2.84 (m, 2H), 2.75-2.64 (m, 2H), 2.50-2.28 (m, 5H), 2.27-1.95 (m, 14H), 1.95-1.66 (m, 8H), 1.65-1.28 (m, 11H), 1.10 (d, J = 6.5 Hz, 3H), 1.06-0.90 (m, 9H). ESI MS calculated [M+Na]⁺: 1553, observed 1553.

### Example 16: Synthesis of FL-F21N-L177-P204 (MC-Phe-VC-(PEG4-Eribulin)₂)

**Step 16-1:** Fmoc-VCP-NH-(PEG4-PNP)₂ (0.6 g, 1.0 eq.) was dissolved in DMAc (6 mL). The reaction solution was cooled down to 10°C and then Erubulin (0.6 g, 2.5 eq.) was added thereto with stirring at 10°C. After the completion of the reaction was confirmed, ethyl acetate/n-heptane (1:2, 45 mL) was added thereto with stirring for a few minutes and the liquid of the mixture was removed. Then, the residue was rinsed twice with diethyl ether/n-heptane (3: 5, 16 mL *2), collected, and dried to afford FL-F21N-L177-P204-Int01.

**Step 16-2:** FL-F21N-L177-P204-Int01 (1.07 g, 1.0 eq.) was dissolved in DMAc (8.6 mL). Further, piperidine (0.14 mL, 3.5 eq.) and DMAc (2.2 mL) were mixed and then added into the FL-F21N-L177-P204-Int01 (1.07 g, 1.0 eq.) solution with stirring at room temperature. After the completion of the reaction was confirmed, the reaction solution was dried under vacuum, and then mixed with diethyl ether/ethyl acetate (1:5, 30 mL) with stirring for 5-10 minutes. The supernatant was removed, and the solid was dried. Next, diethyl ether/ethyl acetate (1:5, 30 mL) was further added thereto with stirring for 5-10 minutes. After removing the supernatant, the solid was dried under vacuum so as to give FL-F21N-L177-P204-Int02 with a yield of 94.2%.

**Step 16-3:** 4-Maleimidophenylacetic acid N-hydroxysuccinimide ester (0.05 g, 3.5 eq.) was dissolved in dichloromethane (0.2 mL) and kept in an ice bath (below 10°C). Further, FL-F21N-L177-P204-Int02 (100 mg, 1.0 eq.) was dissolved in dichloromethane (0.05 mL), and then added into the reaction solution of 4-Maleimidophenylacetic acid N-hydroxysuccinimide ester. After the addition was complete, the reaction solution was continuously stirred at the ice bath. After the completion of the reaction was confirmed, dichloromethane was removed therefrom and the residue was purified (acetonitrile/water = 6/4) so as to give FL-F21N-L177-P204 with a yield of 32%.

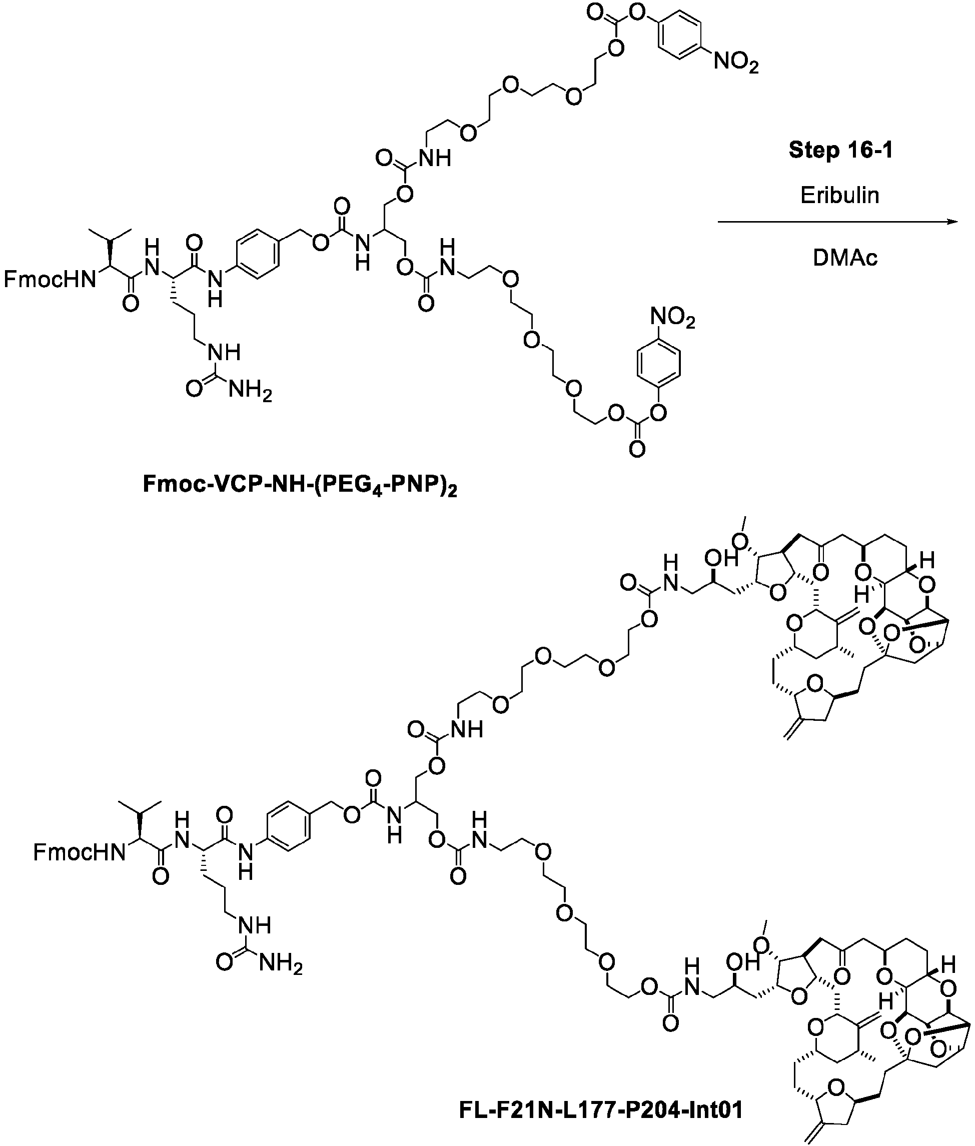

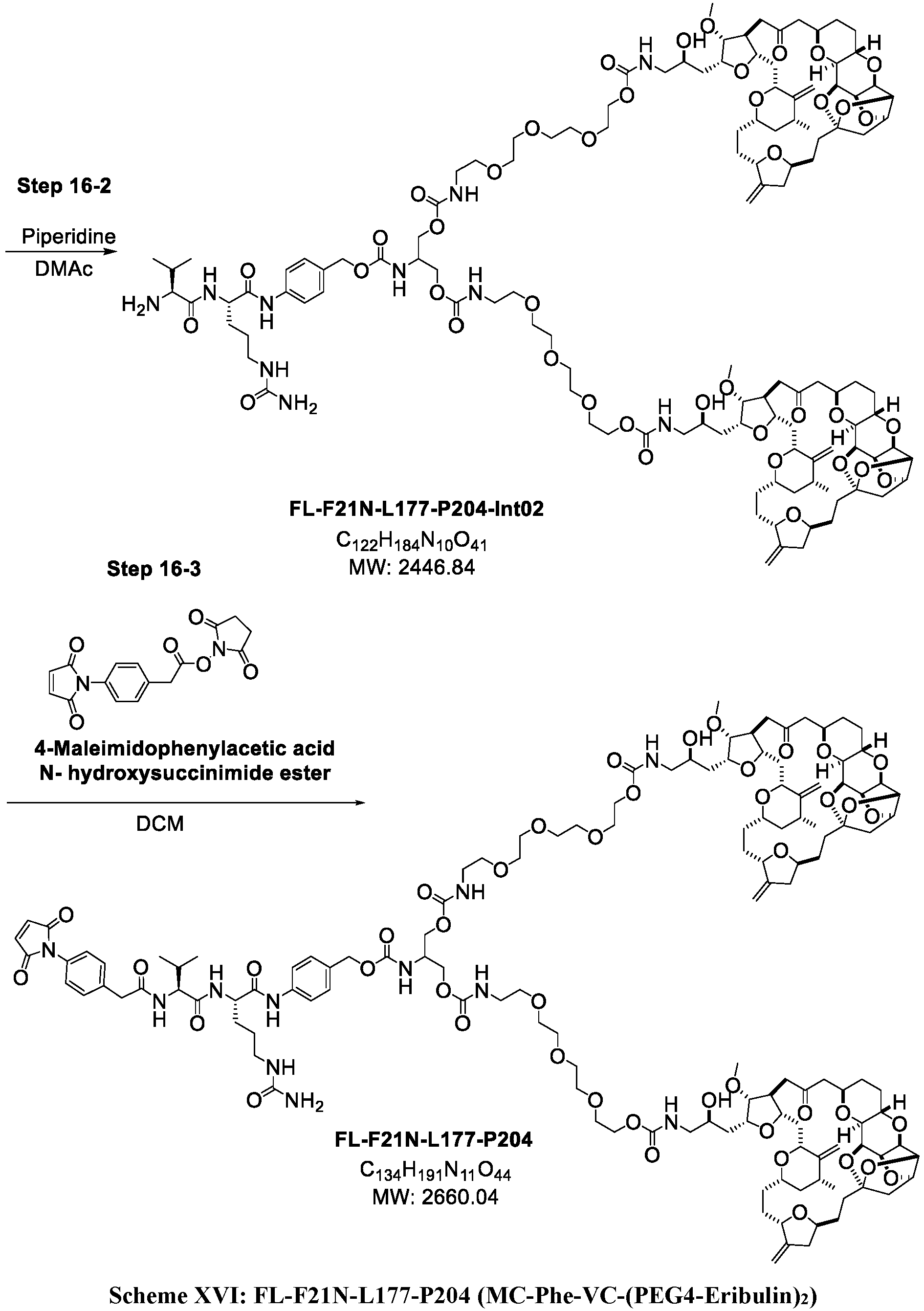

¹H-NMR (600 MHz, DMSO-*d6*) δ 10.01 (s, 1H), 8.19-8.15 (m, 2H), 7.59 (d, J = 8.3 Hz, 2H), 7.36 (d, J = 8.4 Hz, 2H), 7.31 (d, J = 7.6 Hz, 1H), 7.28 (d, J = 8.7 Hz, 2H), 7.23 (d, J = 8.3 Hz, 2H), 7.17 (s, 2H), 7.16 (s, 1H), 7.06-7.04 (m, 2H), 5.98-5.95 (m, 2H), 5.40 (s, 2H), 5.05 (s, 2H), 4.99 (s, 2H), 4.95 (s, 2H), 4.83 (s, 2H), 4.75 (s, 2H), 4.64-4.63 (m, 2H), 4.55 (t, J = 4.3 Hz, 2H), 4.51 (d, J = 5.2 Hz, 2H), 4.40-4.37 (m, 2H), 4.27-4.22 (m, 3H), 4.29-4.15 (m, 2H), 4.11-4.09 (m, 5H), 4.03-3.97 (m, 6H), 3.90-3.87 (m, 3H), 3.82-3.78 (m, 2H), 3.75-3.74 (m, 2H), 3.70-3.67 (m, 2H), 3.63-3.61 (m, 2H), 3.54-3.49 (m, 18H), 3.40-3.38 (m, 3H), 3.26-3.24 (m, 6H), 3.11-3.10 (m, 3H), 3.00-3.04 (m, 2H), 2.96-2.94 (m, 3H), 2.84 (d, J= 9.5 Hz, 2H), 2.76-2.67 (m, 5H), 2.61-2.54 (m, 4H), 2.33-2.11 (m, 13H), 2.03-1.92 (m, 12H), 1.72-1.14 (m, 32H), 1.03 (d, J = 6.4 Hz, 4H), 1.01-0.94 (m, 3H), 0.86-0.83 (m, 6H). ESI MS calculated [M+H]⁺: 2661.0, observed 2661.1.

Synthesis of FL-F21N-L167-P204 is similar to synthesis of Examples 10 and 16, and the synthesis pathway therefor is planned as follows:

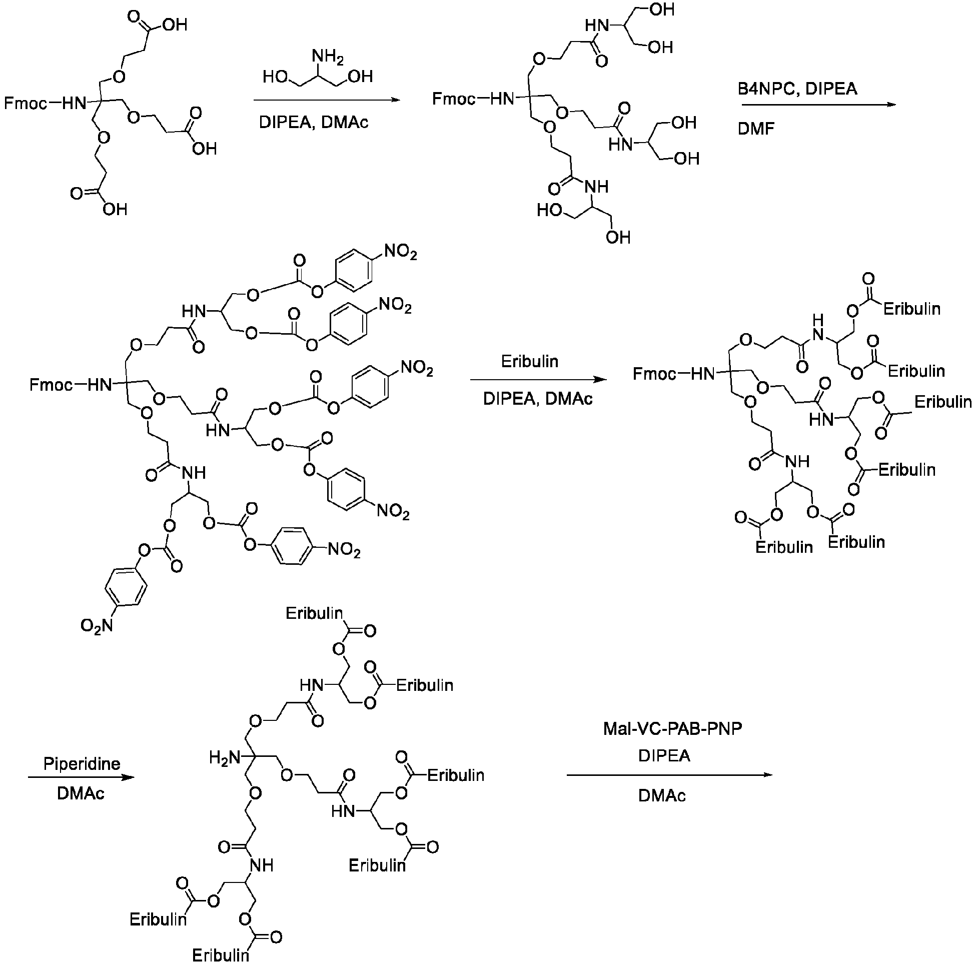

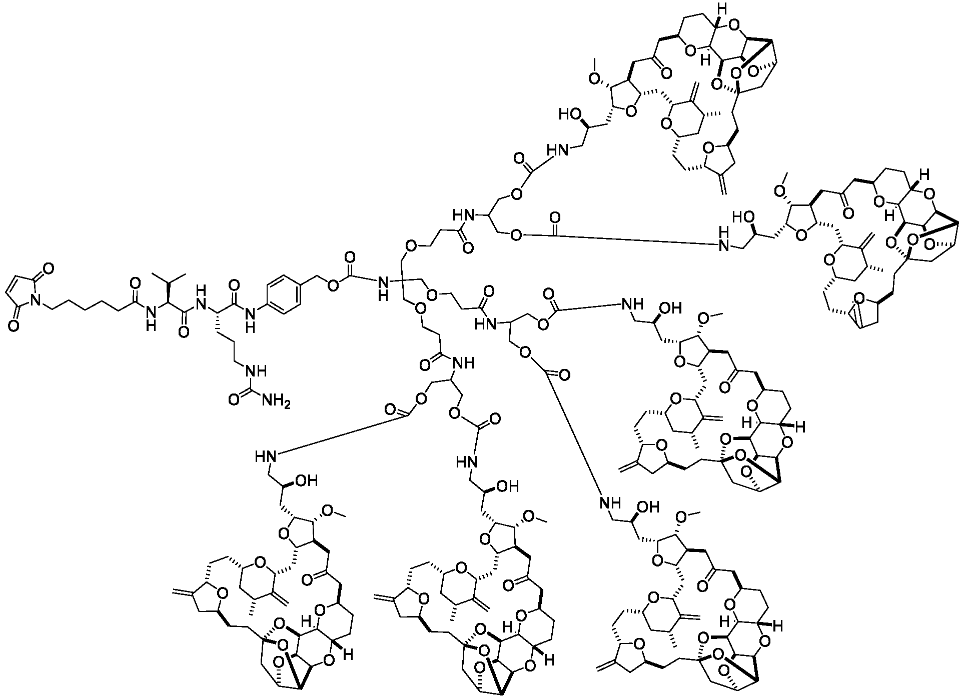

### Example 17: Synthesis of FL-F21N-L178-P204 (BCN-VC-(PEG4-Eribulin)₂)

**Step 17-1:** FL-F21N-L177-P204-Int02 (0.44 g) was dissolved in DMAc (4.4 mL) and stirred at 15-25°C for 5-10 min. Then, endo-BCN-O-PNB (86 mg) was added to the reaction mixture. After the reaction was complete, the mixture was added into ethyl acetate (6.6 mL) / *n*-Heptane (26.4 mL). After the addition was complete, the reaction mixture was sonicated for 5-10 min. The upper layer solvent was removed, and the resulting mixture was vacuum-dried. The sticky solid was precipitated by dichloromethane (1 mL) / diethyl ether (1 mL). The solid was vacuum-dried under high to afford yellow FL-F21N-L178-P204 0.33 g with a yield of 69.3%.

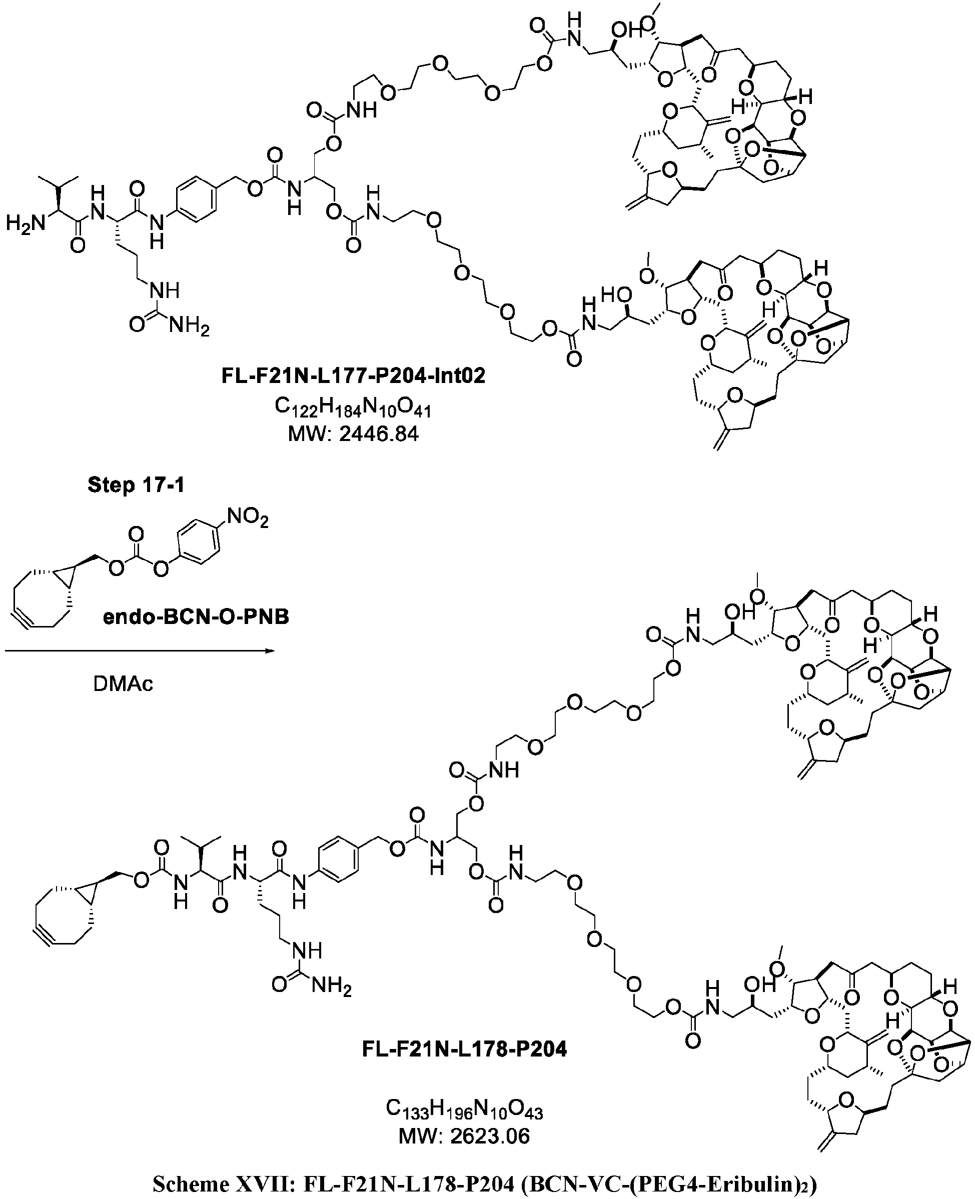

¹H-NMR (600 MHz, DMSO-*d6*) δ 10.03 (s, 1H), 8.02 (d, J = 7.7 Hz, 2H), 7.58 (d, J = 8.2 Hz, 2H), 7.31 (d, J = 7.6 Hz, 1H), 7.29 (d, J = 8.3 Hz, 1H), 7.19-7.14 (m, 1H), 7.12 (d, J = 8.9 Hz, 1H), 7.05 (t, J = 5.9 Hz, 1H), 5.96 (t, J = 5.8 Hz, 1H), 5.40 (s, 2H), 5.05 (d, J = 2.3 Hz, 1H), 5.00 (s, 1H), 4.95 (s, 1H), 4.83 (s, 1H), 4.75 (s, 1H), 4.64-4.62 (m, 1H), 4.55 (t, J = 4.3 Hz, 1H), 4.51 (d, J = 5.2 Hz, 1H), 4.43-4.40 (m, 1H), 4.26 (d, J = 10.9 Hz, 1H), 4.19-4.15 (m, 2H), 4.13-3.94 (m, 8H), 3.89-3.87 (m, 2H), 3.82-3.78 (m, 1H), 3.77-3.74 (m, 1H), 3.70-3.67 (m, 1H), 3.55-3.49 (m, 12H), 3.40-3.36 (m, 4H), 3.25 (s, 3H), 3.12-3.09 (m, 2H), 3.04-3.01 (m, 1H), 2.96-2.94 (m, 3H), 2.85-2.83 (m, 1H), 2.78- 2.67 (m, 3H), 2.58-2.54 (m, 1H), 2.33-2.11 (m, 10H), 2.03-1.88 (m, 8H), 1.75-1.14 (m, 19H), 1.09 (t, J = 7.0 Hz, 2H), 1.03 (d, J = 6.4 Hz, 3H), 1.01-0.95 (m, 2H), 0.87 (d, J = 6.4 Hz, 2H), 0.83 (d, J = 6.7 Hz, 2H). ESI MS calculated [M+H]⁺:2622.4, observed 2623.6.

### Example 18: Synthesis of FL-F21N-L179-P204 (Mal-Val-Cit-PAB-(PEG4-(PEG4-Eribulin)₂)₂)

**Step 18-1:** 2-(2-(2-(2-aminoethoxy)ethoxy)ethoxy)ethan-1-ol (1.0 g, 5.2 mmol) was dissolved in dichloromethane (10 mL) and DIPEA (0.7 g, 5.4 mmol) was added thereto. The reaction solution was cooled down to 0-5°C. Fmoc-Cl (1.4 mL, 5.4 mmol) was dissolved in dichloromethane (5 mL) and slowly added into the 2-(2-(2-(2-aminoethoxy)ethoxy)ethoxy)ethan- 1-ol solution. After the addition was complete, the reaction was stirred at 0-5°C for 1 hour. Once the reaction was complete, the reaction was quenched by adding H₂O (50 mL*2) at 0-5°C twice. The organic layer was dehydrated with anhydrous magnesium sulfate, then filtered, vacuum-dried, and purified by silica gel to give FmocNH-PEG4-OH with a yield of 19%.

**Step 18-2:** The FmocNH-PEG4-OH (50 mg, 0.12 mmol) was dissolved in dichloromethane (1.5 mL). B4NPC (109.8 mg, 0.36 mmol) and DIPEA (47 mg, 0.36 mmol) were added thereto. The mixture was stirred at room temperature for 46 hours. After the reaction was complete, the mixture was dried under vacuum and then purified by silica gel so as to give FmocNH-PEG4-PNP with a yield of 69%.

**Step 18-3:** The FmocNH-PEG4-PNP (487 mg, 0.84 mmol) was dissolved in DMAc (12 mL) and cooled down to 0-5°C. Eribulin (676 mg, 0.93 mmol) and DIPEA (168 mg, 1.30 mmol) were added thereto and the mixture was stirred at room temperature for 4 hours. After the reaction was complete, H₂O (39 mL) was added and the mixture was extracted with ethyl acetate twice (39 mL*2). The organic layer was collected, rinsed with H₂O (39 mL*3) three times, dehydrated with anhydrous magnesium sulfate, filtered, and concentrated so as to give crude Fmoc-Compound D.

**Step 18-4:** Fmoc-Compound **D** (50 mg, 0.04 mmol) was dissolved in ethyl acetate (1 mL). Piperidine (102 mg, 1.20 mmol) was added and the reaction solution was stirred at room temperature for 20 hours. The mixture was dried under vacuum and then purified by silica gel so as to give Compound **D** with a yield of 76%.

**Step 18-5:** Compound **D** (260 mg, 0.3 mmol) was dissolved in dichloromethane (5 mL). DIPEA (72 µL) and (9H-fluoren-9-yl)methyl (1,3-bis(((4-nitrophenoxy)carbonyl)oxy)propan-2-yl)carbamate (50 mg, 0.08 mmol) were added thereto. The mixture was stirred at room temperature for 20 hours. After the reaction was complete, ethyl acetate (55 mL) was added and the mixture was extracted with H₂O (11 mL*3) three times. The organic layer was dehydrated with anhydrous magnesium sulfate, filtered, and concentrated so as to give FL-F21N-L179-P204-Int01.

**Step 18-6:** FL-F21N-L179-P204-Int01 (100 mg, 0.04 mmol) was dissolved in ethyl acetate (1 mL). Piperidine (517 mg, 6.07 mmol) was added and the reaction solution was stirred at room temperature for 2 hours. The mixture was dried under vacuum and then purified by silica gel so as to give FL-F21N-L179-P204-Int02 with a yield of 71.9%.

**Step 18-7:** FL-F21N-L179-P204-Int02 (111 mg, 0.05 mmol) was dissolved in DMAc (1 mL). DIPEA (29 uL) and FL-F21N-L106-P204-Int04 (34 mg, 0.02 mmol) were added thereto. The reaction mixture was stirred at room temperature for 141 hours. After the reaction was complete, DMAc was evaporated under vacuum and the residue was mixed with ethyl acetate/n-heptane (1:1, 8.6 mL) to precipitate crude FL-F21N-L179-P204. Then, the crude was washed with ethyl acetate/n-heptane (1:1, 8.6 mL*2) solution twice, collected, dried, and purified by HILIC chromatography to give FL-F21N-L179-P204 with a yield of 60%.

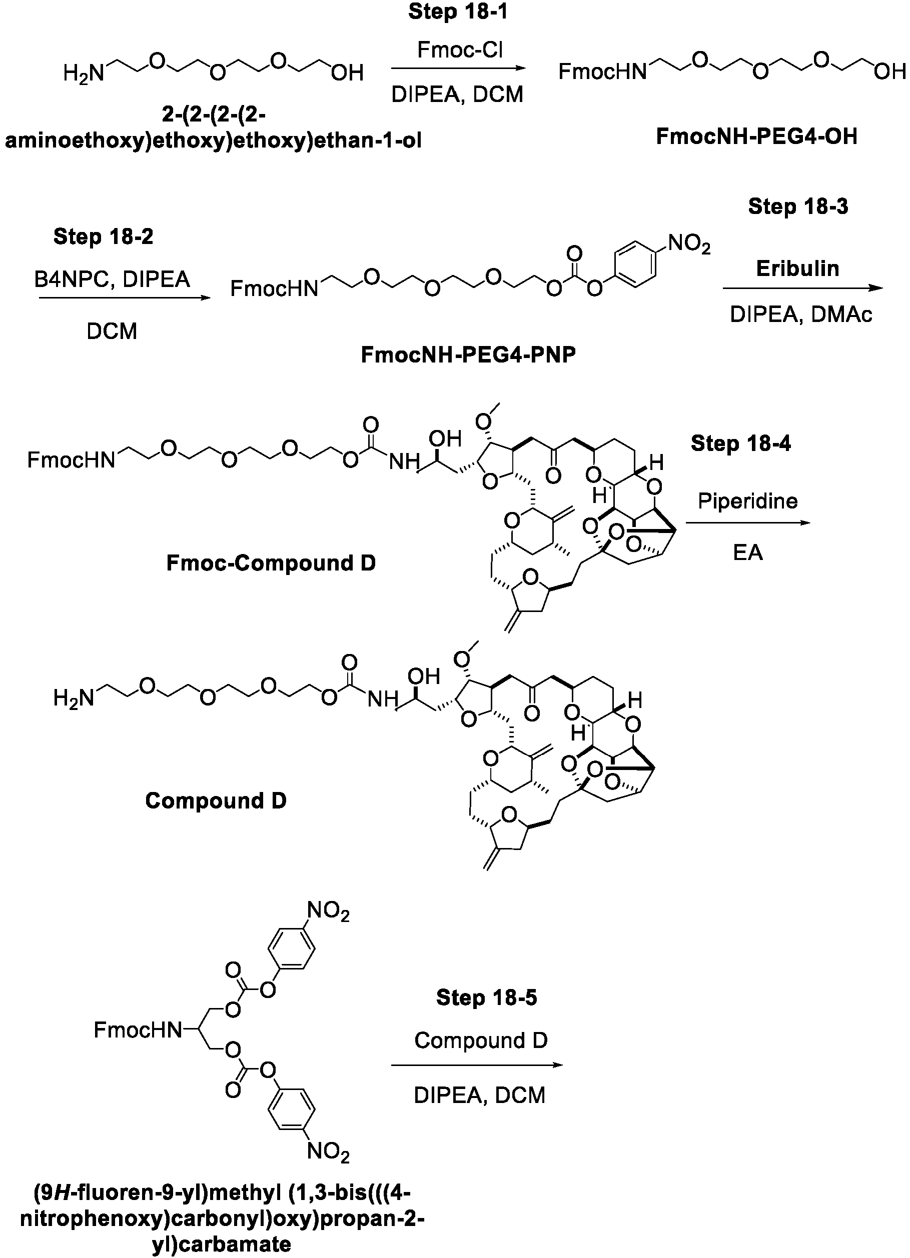

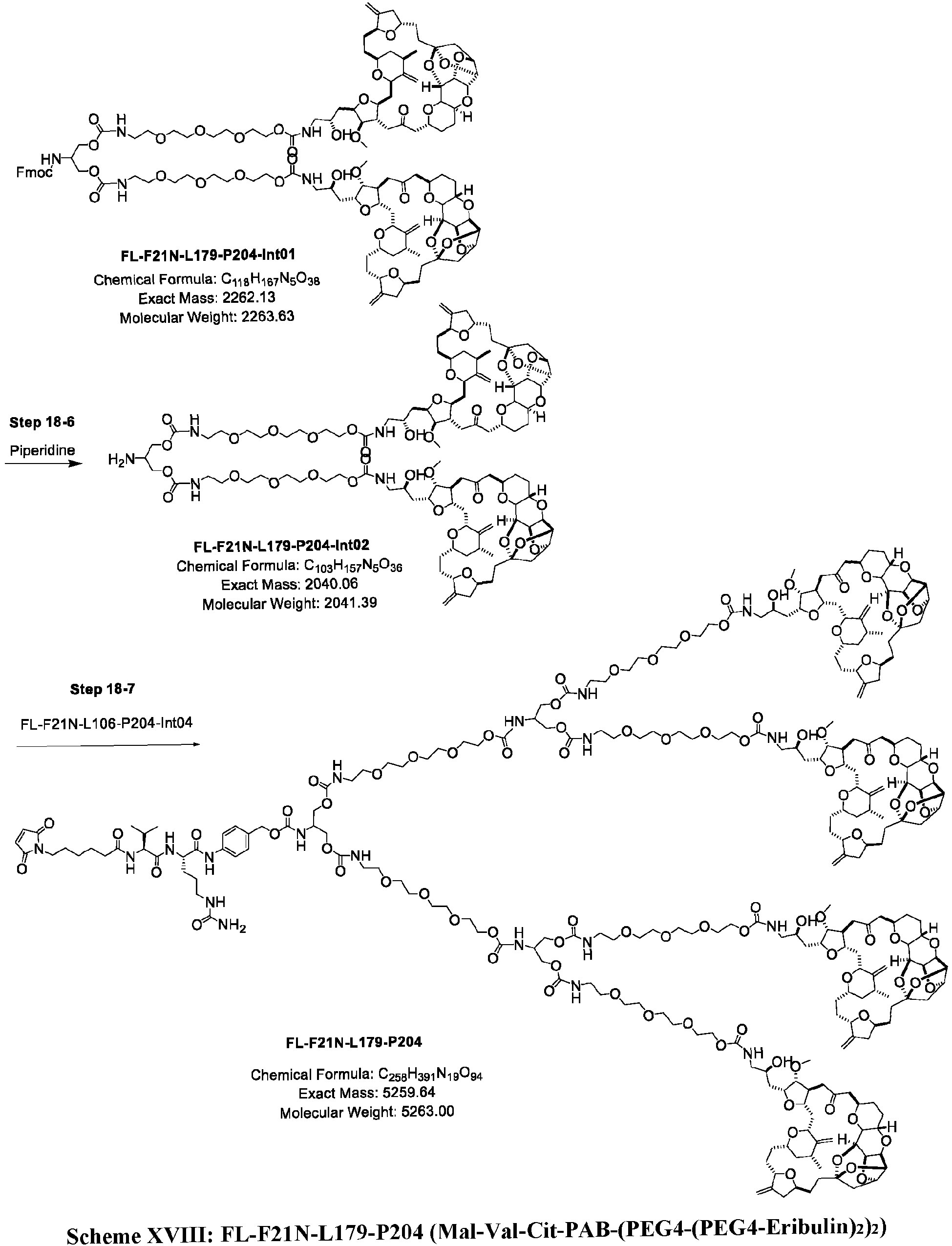

¹H NMR (400 MHz, CDCl₃) δ 9.29 (s, 1H), 7.61 (d, J = 8.5 Hz, 2H), 7.28 (d, J = 8.5 Hz, 2H), 6.68 (s, 2H), 5.09-5.01 (m, 7H), 5.01-4.96 (m, 4H), 4.89-4.86 (m, 4H), 4.82-4.78 (m, 4H) 4.69, (t, J = 4.5 Hz, 4H), 4.60 (t, J= 4.5 Hz, 4H), 4.37-4.29 (m, 15H), 4.20-3.79 (m, 77H), 3.68-3.65 (m, 13H), 3.57-3.50(m, 16H), 3.36-3.26 (m, 22H), 3.20-3.11 (m, 6H), 2.91-2.83 (m, 10H), 2.75-2.67 (m, 5H), 2.56-2.34 (m, 18H), 2.30-2.14 (m, 32H), 2.12-2.06 (m, 6H), 2.00-1.87 (m, 24H), 1.78-1.65 (m, 26H), 1.50-1.44 (m, 20H), 1.39-1.32 (m, 18H), 1.15-1.02 (m, 24H), 0.97-0.92 (m, 7H), 0.91-.080 (m, 14H). ESI MS calculated [M+4H]⁴⁺: 1315.9, observed 1316.4.

### Synthesis of ADCs (i.e. Moiety-Drug Conjugates in the Present Disclosure)

### Example A: Synthesis of FL-F21N-A701-L111-P204-D04 ADC (Trastuzumab-Mal-Val-Cit-PAB-PEG4-Eribulin)

The Trastuzumab, which has been replaced with a solution (i.e. conjugation buffer) of 10-50 mM NaPi or sodium phosphate or histidine or sodium succinate, 100-200 mM NaCl, 100-200 mM KCl, 2-20 mM EDTA, pH 7.0-7.5, was taken into a glass reaction bottle. 1.8-3.6 eq TCEP was added thereto to conduct reduction reaction. The reduction reaction was conducted in the reaction solution at 18-25 °C for 1.5-3.5 hours, and the estimated DAR was calculated by hydrophobic interaction chromatography (HIC)-HPLC. After the reduction reaction was complete, 5-15% DMSO or DMA or ACN and 4.0-6.0 eq FL-F21N-L111-P204 were added. After 0.5-2 hours of reaction, a sample was taken to confirm the DAR value. Afterwards, a buffer solution including 10-50 mM succinic acid or sodium citrate or sodium acetate or/and arginine, pH 4.5-6.0, was added to dilute the reaction solution 3-6 times and to terminate the conjugation reaction.

The diluted FL-F21N-A701-L111-P204-D04 ADC mixture was filtered by 0.2 µm filter, followed by diafiltration concentration and purification to a buffer solution including 10-50 mM succinic acid or sodium citrate or sodium acetate or/and arginine, pH 4.5-6.0, with 30-100 kDa filter membrane to remove FL-F21N-L111-P204, impurities related to FL-F21N-L111-P204 and residual solvent, e.g., DMSO, DMA or ACN. After the concentration was adjusted to 10-30 mg/mL, excipient (50-65% sucrose, and 0.02-0.4% tween 20 or tween 80) was added and the mixture was filtered by 0.2 µm filter to give FL-F21N-A701-L111-P204-D04 ADC DS. Finally, a sample was taken to confirm the concentration, the DAR value (HIC-HPLC or Reduced RPLC) and HMW (%). The product was stored under -20 to -80°C with a yield of 81-88%.

The average DAR of the representative batch of FL-F21N-A701-L111-P204-D04 ADC was 4.04 (HIC-HPLC) and 4.31(Reduced RPLC), with purity of 99.10% and HMW of 0.90%.

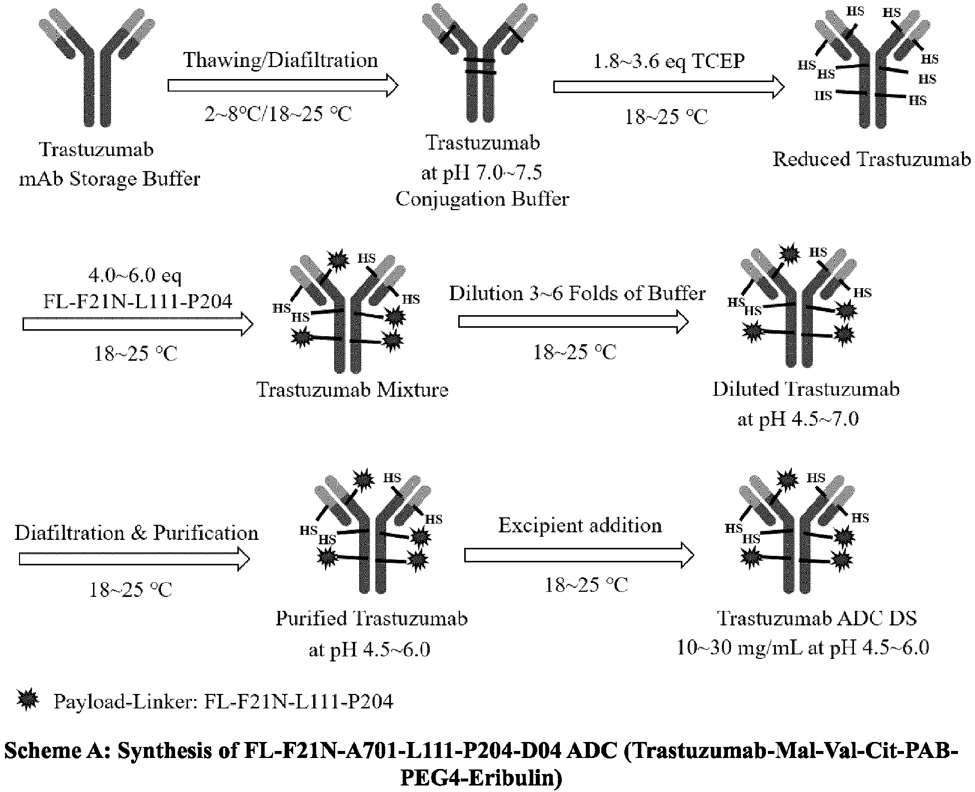

### Example B: Synthesis of FL-F21N-A701-L111-P204-D08 ADC (Trastuzumab-Mal-Val-Cit-PAB-PEG4-Eribulin)

The synthesis process of Example B is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "5.5-9.0 eq TCEP", "8.0-13.0 eq FL-F21N-L111-P204" and "FL-F21N-A701-L111-P204-D08 ADC", respectively. The product was stored with a yield of 90-97%.

The average DAR of the representative batch of FL-F21N-A701-L111-P204-D08 ADC was 7.93 (HIC-HPLC) and 7.84 (Reduced RPLC), with purity of 100% and HMW being not determined.

### Example C: Synthesis of FL-F21N-A701-L106-P204-D08 ADC (Trastuzumab-Mal-Val-Cit-PAB-(PEG4-Eribulin)₂)

The synthesis process of Example C is the same as the synthesis process of Example A, except that the terms "4.0-6.0 eq FL-F21N-L111-P204" and "FL-F21N-A701-L111-P204-D04 ADC" are replaced by "4.0-6.0 eq FL-F21N-L106-P204" and "FL-F21N-A701-L106-P204-D08 ADC", respectively. The product was stored with a yield of 76-86%.

The average DAR of the representative batch of FL-F21N-A701-L106-P204-D08 ADC was 6.72 (HIC-HPLC) and 7.81 (Reduced RPLC), with purity of 99.67% and HMW of 0.33%.

### Example D: Synthesis of FL-F21N-A701-L107-P204-D08 ADC (Trastuzumab-Mal-Val-Cit-PAB-(Eribulin)₂)

The synthesis process of Example D is the same as the synthesis process of Example A, except that the terms "4.0-6.0 eq FL-F21N-L111-P204" and "FL-F21N-A701-L111-P204-D04 ADC" are replaced by "4.0-6.0 eq FL-F21N-L107-P204" and "FL-F21N-A701-L107-P204-D08 ADC", respectively. The product was stored with a yield of 82-88%.

The average DAR of the representative batch of FL-F21N-A701-L107-P204-D08 ADC was 5.9 (HIC-HPLC) and 6.32 (Reduced RPLC), with purity of 94.92% and HMW of 5.08%.

### Example E: Synthesis of FL-F21N-A701-L162-P204-D04 ADC (Trastuzumab-Mal-Val-Cit-PAB-PEG₁₂-Eribulin)

The synthesis process of Example E is the same as the synthesis process of Example A, except that the terms "4.0-6.0 eq FL-F21N-L111-P204" and "FL-F21N-A701-L111-P204-D04 ADC" are replaced by "4.0-6.0 eq FL-F21N-L162-P204" and "FL-F21N-A701-L162-P204-D04 ADC", respectively. The product was stored with a yield of ~90%.

The average DAR of the representative batch of FL-F21N-A701-L162-P204-D04 ADC was 4.08 (HIC-HPLC) and 5.23 (Reduced RPLC), with purity of 98.88% and HMW of 0.57%.

### Example F: Synthesis of FL-F21N-A701-L162-P204-D08 ADC (Trastuzumab-Mal-Val-Cit-PAB-PEG₁₂-Eribulin)

The synthesis process of Example F is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "5.5-12.0 eq TCEP", "8.0-14.0 eq FL-F21N-L162-P204" and "FL-F21N-A701-L162-P204-D08 ADC", respectively. The product was stored with a yield of -88%.

The average DAR of the representative batch of FL-F21N-A701-L162-P204-D08 ADC was 7.69 (HIC-HPLC) and 7.38 (Reduced RPLC), with purity of 98.27% and HMW of 1.04%.

### Example G: Synthesis of FL-F21N-A701-L163-P204-D04 ADC (Trastuzumab-Mal-Val-Cit-PAB-PEG₂₄-Eribulin)

The synthesis process of Example G is the same as the synthesis process of Example A, except that the terms "4.0-6.0 eq FL-F21N-L111-P204" and "FL-F21N-A701-L111-P204-D04 ADC" are replaced by "4.0-7.0 eq FL-F21N-L163-P204" and "FL-F21N-A701-L163-P204-D04 ADC", respectively. The product was stored with a yield of ~94%.

The average DAR of the representative batch of FL-F21N-A701-L163-P204-D04 ADC was 4.02 (HIC-HPLC) and 3.66 (Reduced RPLC), with purity of 99.25% and HMW of 0.75%.

### Example H: Synthesis of FL-F21N-A701-L163-P204-D08 ADC (Trastuzumab-Mal-Val-Cit-PAB-PEG₂₄-Eribulin)

The synthesis process of Example H is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "5.5-10.0 eq TCEP", "8.0-16.0 eq FL-F21N-L163-P204" and "FL-F21N-A701-L163-P204-D08 ADC", respectively. The product was stored with a yield of 90%.

The average DAR of the representative batch of FL-F21N-A701-L163-P204-D08 ADC was 7.66 (HIC-HPLC) and 8.00 (Reduced RPLC), with purity of 98.94% and HMW of 1.06%.

### Example I: Synthesis of FL-F21N-A701-L164-P204-D08 ADC (Trastuzumab-SMCC-(PEG4-Eribulin)₂)

The synthesis process of Example I is the same as the synthesis process of Example A, except that the terms "4.0-6.0 eq FL-F21N-L111-P204" and "FL-F21N-A701-L111-P204-D04 ADC" are replaced by "4.0-7.0 eq FL-F21N-L164-P204" and "FL-F21N-A701-L164-P204-D08 ADC", respectively. The product was stored with a yield of ~87%.

The average DAR of the representative batch of FL-F21N-A701-L164-P204-D08 ADC was 6.26 (HIC-HPLC) and 6.68 (Reduced RPLC), with purity of 99.09% and HMW of 0.73%.

### Example J: Synthesis of FL-F21N-A701-L108-P204-D08 ADC (Trastuzumab-Mal-Val-Cit-PAB-(Eribulin)₃)

The synthesis process of Example J is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "5.5-10.0 eq TCEP", "5.0-12.0 eq FL-F21N-L108-P204" and "FL-F21N-A701-L108-P204-D08 ADC", respectively. The product was stored with a yield of 60-80%.

The average DAR of the representative batch of FL-F21N-A701-L108-P204-D08 ADC was 5.7 (HIC-HPLC) and 14.49 (Reduced RPLC).

### Example K: Synthesis of FL-F21N-A701-L165-P205-D08 ADC (Trastuzumab-SMCC-(PEG4-MMAE)₃)

The synthesis process of Example K is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "6.5-13.0 eq TCEP", "4.0-9.0 eq FL-F21N-L165-P205" and "FL-F21N-A701-L165-P205-D08 ADC", respectively. The product was stored with a yield of -91%.

The average DAR of the representative batch of FL-F21N-A701-L165-P205-D08 ADC was 5.93 (HIC-HPLC) and 10.62 (Reduced RPLC), with purity of 84.4% and HMW of 15.6%.

### Example L: Synthesis of FL-F21N-A701-L152-P204-D04 ADC (Trastuzumab-Mal-Val-Cit-PAB-Branched PEG₃₇-Eribulin)

The synthesis process of Example L is the same as the synthesis process of Example A, except that the terms "4.0-6.0 eq FL-F21N-L111-P204" and "FL-F21N-A701-L111-P204-D04 ADC" are replaced by "4.0-7.0 eq FL-F21N-L152-P204" and "FL-F21N-A701-L152-P204-D04 ADC", respectively. The product was stored with a yield of -81%.

The average DAR of the representative batch of FL-F21N-A701-L152-P204-D04 was 4.09 (HIC-HPLC) and 3.55 (Reduced RPLC), with purity of 99.09% and HMW of 0.91%.

### Example M: Synthesis of FL-F21N-A701-L152-P204-D08 ADC (Trastuzumab-Mal-Val-Cit-PAB-Branched PEG₃₇-Eribulin)

The synthesis process of Example M is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "6.5-12.0 eq TCEP", "9.0-16.0 eq FL-F21N-L152-P204" and "FL-F21N-A701-L152-P204-D08", respectively. The product was stored with a yield of ~95%.

The average DAR of the representative batch of FL-F21N-A701-L152-P204-D08 was 6.97 (HIC-HPLC) and 5.69 (Reduced RPLC), with purity of 99.27% and HMW of 0.73%.

### Example N: Synthesis of FL-F21N-A701-L177-P204-D08 ADC (Trastuzumab-Mal-Phe-Val-Cit-PAB-(PEG₄-Eribulin)₂)

The synthesis process of Example N is the same as the synthesis process of Example A, except that the terms "1.8-3.6 eq TCEP", "4.0-6.0 eq FL-F21N-L111-P204" and "FL-F21N-A701-L111-P204-D04 ADC" are replaced by "6.5-13.0 eq TCEP", "5.0-12.0 eq FL-F21N-L177-P204" and "FL-F21N-A701-L177-P204-D08 ADC", respectively. The product was stored with a yield of -94%.

The average DAR of the representative batch of FL-F21N-A701-L177-P204-D08 ADC was 7.94 (HIC-HPLC) and 15.76 (Reduced RPLC).

### Example O: Synthesis of FL-F21N-A701-L175-P204-D04 ADC (Trastuzumab-Mal-Phe-Val-Cit-PAB-PEG₄-Eribulin)

The synthesis process of Example O is the same as the synthesis process of Example A, except that the terms "4.0-6.0 eq FL-F21N-L111-P204" and "FL-F21N-A701-L111-P204-D04 ADC" are replaced by "4.5-8.0 eq FL-F21N-L175-P204" and "FL-F21N-A701-L175-P204-D04 ADC", respectively. The product was stored with a yield of ~94%.

The average DAR of the representative batch of FL-F21N-A701-L175-P204-D04 was 4.54 (HIC-HPLC) and 3.94 (Reduced RPLC), with purity of 99.21% and HMW of 0.79%.

### Example P: Synthesis of FL-F21N-A701-L175-P204-D08 ADC (Trastuzumab-Mal-Phe-Val-Cit-PAB--PEG₄-Eribulin

The synthesis process of Example P is the same as the synthesis process of Example A, except that the terms "1.8-3.6 eq TCEP", 4.0-6.0 eq FL-F21N-L111-P204" and "FL-F21N-A701-L111-P204-D04 ADC" are replaced by "6.5-12.0 eq TCEP", "6.0-12.0 eq FL-F21N-L175-P204" and "FL-F21N-A701-L175-P204-D08 ADC", respectively. The product was stored with a yield of -96%.

The average DAR of the representative batch of FL-F21N-A701-L175-P204-D08 was 7.78 (HIC-HPLC) and 7.65 (Reduced RPLC), with purity of 98.15% and HMW of 1.85%.

### Implementation Example 1: Water Solubility

ADMETlab 2.0 was used to calculate the Log P of the compound in the present disclosure. See Table 1, Entry 2-4, when the length of the PEG group in the compound increased, the corresponded Log P decreased and the water solubility was enhanced. In addition, when the number of drugs in the compound increased, the corresponded Log P increased and the water solubility decreased.

**Table 1: Prediction of LogP of the compounds in the present disclosure by ADMETlab 2.0**

| **Entry** | **Code** | **Name** | **Length of PEG** | **Number of arm** | **Log P** |
|---|---|---|---|---|---|
| 1 | P204 | Eribulin | N/A | N/A | 3.406 |
| 2 | FL-F21N-L111-P204 | MC-VCP-PEG4-Eribulin | 4 | 1 | 3.873 |
| 3 | FL-F21N-L162-P204 | MC-VCP-PEG12-Eribulin | 12 | 1 | 3.086 |
| 4 | FL-F21N-L163-P204 | MC-VCP-PEG24-Eribulin | 24 | 1 | 2.492 |
| 5 | FL-F21N-L106-P204 | MC-VCP-(PEG4-Eribulin)₂ | 4 | 2 | 5.684 |
| 6 | FL-F21N-L107-P204 | MC-VCP-(Eribulin)₂ | 0 | 2 | 7.092 |
| 7 | FL-F21N-L108-P204 | MC-VCP-(Eribulin)₃ | 0 | 3 | 9.350 |

In addition, ADMETlab 2.0 was also used to calculate the Log P of the cleaved products of the compounds in the present disclosure cleaved by Cathepsin B in cells. The results were shown in Table 2. The cleaved products of the compounds in the present disclosure with longer PEG group, e.g., Entry 2-4, were provided with better water solubility after cleaved by Cathepsin B, even better than Eribulin, e.g., Entry 1. With PEG group, e.g., Entry 5, the cleaved product was provided with better water solubility compared to the one without PEG group, e.g., Entry 6. The compounds provided with PEG groups in the present disclosure may enhance applicability to the water-soluble carrier (i.e. moiety), thereby modifying the characteristics of the moiety-compound conjugates.

**Table 2: Prediction of LogP of the compounds in the present disclosure after cleaved by Cathepsin B by ADMETlab 2.0**

| **Entry** | **Expected metabolite** | **Substrate** | **Length of PEG** | **Number of arm** | **Log P** |
|---|---|---|---|---|---|
| 1 | N/A | Eribulin | N/A | N/A | 3.406 |
| 2^{a} | P-PEG4-P204 | MC-VCP-PEG4-Eribulin | 4 | 1 | 3.548 |
| 3^{a} | P-PEG12-P204 | MC-VCP-PEG12-Eribulin | 12 | 1 | 2.687 |
| 4^{a} | P-PEG24-P204 | MC-VCP-PEG24-Eribulin | 24 | 1 | 1.986 |
| 5^{a} | P-(PEG4-P204)₂ | MC-VCP-(PEG4-Eribulin)₂ | 4 | 2 | 5.265 |
| 6^{a} | P-(P204)₂ | MC-VCP-(Eribulin)₂ | 0 | 2 | 6.607 |
| 7^{a} | P-(P204)₃ | MC-VCP-(Eribulin)₃ | 0 | 3 | 8.846 |

| | | | | | |
|---|---|---|---|---|---|
| # a: P is para-aminobenzyl group and P204 is Eribulin | | | | | |

**Table 3: Water solubility of the compounds of the present disclosure**

| **Entry** | **Code** | **Name** | **Length of PEG** | **Number of arm(s)** | **Solubility in DMSO (mg/mL)** | **Precipitation in % Water/DMSO (%)** |
|---|---|---|---|---|---|---|
| 1 | FL-F21N-L111-P204 | MC-VCP-PEG4-Enbulin | 4 | 1 | 10.1mg/70µL | 24.5 |
| 2 | FL-F21N-L162-P204 | MC-VCP-PEG12-Enbulin | 12 | 1 | 10.1mg/70µL | 31.5 |
| 3 | FL-F21N-L163-P204 | MC-VCP-PEG24-Enbulin | 24 | 1 | 10.1mg/70µL | 38.9 |
| 4 | FL-F21N-L106-P204 | MC-VCP-(PEG4-Enbulin)₂ | 4 | 2 | 10.1mg/90µL | 30.8 |
| 5 | FL-F21N-L107-P204 | MC-VCP-(Eribulin)₂ | 0 | 2 | 5.2mg/35µL | 19.5 |

The compounds in the present disclosure also underwent water solubility testing and the results were listed in Table 3. Briefly, the moiety-compound conjugates was dissolved with minimal DMSO and precipitated by the addition of water. As shown in Table 3, Entries 1 to 3 are conjugates with a PEG4 linker, a PEG12 linker and a PEG24 linker, respectively. Entry 1 was precipitated in 24.5% Water/DMSO, Entry 2 was precipitated in 31.5% Water/DMSO, and Entry 3 was precipitated in 38.9% Water/DMSO, indicating that the conjugates with a longer PEG arm were provided with better water solubility. However, Entry 5 and Entry 4 are conjugates with two-arm, with no PEG linker or two PEG4 linker, respectively, which were precipitated in 19.5% and 30.8% Water/DMSO. Thus, the results of water solubility of the conjugates are consistent with the inventors' hypothesis.

### Implementation Example 2: Cell Toxicity Testing

Additionally, a series of experiments were conducted, including anti-proliferation assays for linker-drug combinations (i.e. the compounds in the present disclosure), the drugs thereof, and the cleaved products thereof after cleavage by the Cathepsin B enzyme in cells. These assays were performed using HER2-expressing cell lines. MCF-7 and SK-BR-3 cell lines were seeded at a density of 1.5 × 10³ to 1 × 10⁴ cells/well in 90 µL of the corresponding culture medium, and cultured at 37°C in a CO₂ incubator (Forma/ThermoFisher (370) or BINDER (CB130)) with a range of dilutions (0-1000 nM) of the compounds in the present disclosure, the drugs thereof, and the cleaved products thereof. Cell density for each sample was recorded by measuring the absorbance of Cell Counting Kit-8 (CCK-8) at 450 nm. Data analysis was performed using GraphPad Prism 8.0.1, and the best-fit four-parameter dose-response curve was selected. Half Maximal Inhibitory Concentration (IC₅₀) values were determined via nonlinear regression; consistency between batches was assessed using One-way ANOVA. The relevant experimental results are detailed in Table 4. According to the results, the toxicity of Eribulin in HER2-expressing cell lines can reach IC₅₀ values of 0.244 and 0.337 nM. By conjugating Eribulin to a linker, such as FL-F21N-L111-P204, FL-F21N-L106-P204, FL-F21N-L107-P204, or the cleaved products thereof (Table 4, Entries 7 and 8) to form linker-drug combinations, their toxicities are significantly lower than that of the drug (P204, Eribulin), approximately 7.47-33.92% of the drug's toxicity. Such reduced toxicity of the linker-drug combinations (i.e, the compounds in the present disclosure) offers significant advantages in moiety-drug conjugate applications, including better biocompatibility, enhanced therapeutic efficacy, and greater dosing flexibility.

**Table 4: Cell toxicity testing of the compounds of the present disclosure**

| Entry | Code | No. of arm | Linker | Length of PEG | Drug | IC₅₀ (nM) | |
|---|---|---|---|---|---|---|---|
| | | | | | | SK-BR-3 (HER2 3+) | MCF-7 (HER2 1+) |
| 1 | P204 | -- | -- | -- | Eribulin | 0.244 | 0.337 |
| 2 | P209 | -- | -- | -- | Dxd | 5.512 | 9.531 |
| 3 | L155-P209 (Deruxtecan) | Single-arm | MC-GGFG | 0 | Dxd | 431.586 | 512.200 |
| 4 | L111-P204 | Single-arm | MC-VC-PAB | 4 | Eribulin | 30.848 | 45.505 |
| 5 | L106-P204 | Dual-arm | MC-VC-PAB | 4 | Eribulin | 12.998 | 26.256 |
| 6 | L107-P204 | Dual-arm | MC-VC-PAB | 0 | Eribulin | 9.240 | 14.175 |
| 7 | Cathepsin B-cleaved L111-P204 | | | 4 | Eribulin | 7.959 | 30.529 |
| 8 | Cathepsin B-cleaved L106-P204 | | | 4 | Eribulin | 17.928 | 27.981 |

To further advance the research, a set of the compounds in the present disclosure, FL-F21N-L111-P204 and FL-F21N-L106-P204, each containing single-arm and dual-arm respectively, was selected for plasma stability testing in both humans and rats. These were then compared in detail with commercially available Deruxtecan (Dxd) and VC-PAB-MMAE to evaluate their stability and safety in blood.

As shown in FIGs. 1A and 1B, the concentrations of the released drugs were calculated. These calculations were based on standard drugs (e.g., Eribulin, MMAE, Dxd) and they were accurately measured using liquid chromatography-tandem mass spectrometry (LC-MS/MS). FL-F21N-L111-P204 and FL-F21N-L106-P204 were observed to exhibit stable drug release trends in both human and rat plasma over a 13-day period. This trend provides an in-depth understanding of the drug release dynamics.

After culturing in human plasma for 13 days, the free drug percentage of FL-F21N-L111-P204 was only 0.11%, significantly lower than the corresponding percentages of VC-PAB-MMAE and Deruxtecan (0.56% and 5.19%, respectively). Meanwhile, the free drug percentage of FL-F21N-L106-P204 with dual-arm was 2.74%, lower than Deruxtecan of 5.19%. On the other hand, in rat plasma, after the same 13-day culture, the free drug percentage of FL-F21N-L111-P204 was 0.09%, much lower than that of VC-PAB-MMAE and Deruxtecan (5.03% and 0.70%, respectively); while FL-F21N-L106-P204 with dual-arm had a free drug percentage of 3.50%, lower than VC-PAB-MMAE of 5.03%. These experimental results provide crucial data supporting the efficacy and safety of the compounds in the present disclosure.

Furthermore, these innovative compounds in the present disclosure and Trastuzumab (anti-HER2 antibody) were applied in the manufacturing processes of ADCs, successfully producing ADCs with varying Drug-Antibody Ratios (DAR). These novel Anti-HER2-based ADCs were tested and compared them with commercially available ADCs to assess their efficacy in inhibiting cancer cells.

As shown in Table 5, Entry 1, the drug (P204, Eribulin) exhibited cytotoxic effects against both HER2-expressing cell lines (SK-BR-3, MCF-7) and HER2-negative cell lines (MDA-MB-231, MDA-MB-436). The experiment involved optimizing seeding densities (1.5 × 10³ to 1.5 × 10⁴ cells/well) for each cell line (SK-BR-3, MCF-7, MDA-MB-231, MDA-MB-436) in 90 µL of culture medium per well in a 96-well plate for 2-hour incubation. Next, 10 µL of a series of diluted ADC solutions (0-500 nM) were added to each well. The concentration of each ADC solution and measurements at four time points (24, 48, 72, and 96 hours) were conducted in triplicate. Cell density for each sample was recorded by measuring the absorbance of Cell Counting Kit-8 (CCK-8) at 450 nm. Data analysis was performed with GraphPad Prism 8.0.1, employing a four-parameter logistic curve-fit program.

**Table 5: Cell toxicity testing of the moiety-drug conjugates of the present disclosure**

| **Entry** | **Code** | **Antibody** | **No. of arm** | **Linker** | **Length of PEG** | **Drug** | **DAR** | **IC₅₀ (nM)** | | | |
|---|---|---|---|---|---|---|---|---|---|---|---|
| | | | | | | | | SK-BR-3 (HER2 3+) | MCF-7 (HER2 1+) | MDA-MB-231 (HER2-) | MDA-MB-436 (HER2-) |
| 1 | P204 | -- | -- | -- | -- | Eribulin | -- | 0.244 | 0.337 | 2.070 | 1.280 |
| 2 | P209 | -- | -- | -- | -- | Dxd | -- | 5.512 | 9.531 | 135 | 5.120 |
| 3 | A701-L111-P204-D04 | Trastuzumab | Single -arm | MC-VC-PAB | 4 | Eribulin | -4 | 0.012 | 3.832 | 148 | >250 |
| 4 | A701-L111-P204-D08 | Trastuzumab | Single -arm | MC-VC-PAB | 4 | Eribulin | -8 | 0.012 | 0.045 | >250 | 33 |
| 5 | A701-L106-P204-D08 | Trastuzumab | Dual-arm | MC-VC-PAB | 4 | Eribulin | -10 | 0.0216 | 0.0229 | 75.2 | 14.6 |
| 6 | A701-L107-P204-D08 | Trastuzumab | Dual-arm | MC-VC-PAB | 0 | Eribulin | -8 | 0.0172 | 0.0229 | -- | -- |
| 7 | Kadcyla (EU) | Trastuzumab | Single -arm | SMCC | -- | DM1 | 3.5 | 0.113 | 27.758 | 81.8 | 47.2 |
| 8 | Enhertu (EU) | Trastuzumab | Single -arm | GGFG | -- | Dxd | 7.6 | 0.222 | -- | 183 | >250 |

According to the experimental results, when the drug (P204, Eribulin) was conjugated with the anti-HER2 antibody Trastuzumab to form Eribulin-loaded Trastuzumab-based ADCs, there was a significant enhancement in cell growth inhibition activity against HER2-highly expressing cell lines (such as SK-BR-3) compared to using the drug (P204, Eribulin) alone.

The experimental results demonstrate that Eribulin-loaded Trastuzumab-based ADCs with high Drug-Antibody Ratios (DAR) (e.g., FL-A701-L111-P204-D08, FL-A701-L106-P204-D08 and FL-A701-L107-P204-D08) (Table 5, Entries 4-6) exhibit superior cytotoxic effects compared to commercially available drugs Kadcyla and Enhertu, regardless of whether they were tested on HER2-expressing cell lines (SK-BR-3, MCF-7) or HER2-negative cell lines (MDA-MB-231, MDA-MB-436) (Table 5, Entries 7-8). Moreover, Eribulin-loaded Trastuzumab-based ADCs with high DAR values (e.g., FL-A701-L111-P204-D08, FL-A701-L106-P204-D08 and FL-A701-L107-P204-D08) are capable of delivering sufficient effective drugs to cancer cells, demonstrating cell cytotoxicity even in cell lines with lower HER2 expression levels (e.g., MCF-7). For HER2-highly expressing cell lines (e.g., SK-BR-3), even ADCs with low DAR values (e.g., FL- A701-L111-P204-D04) (Table 5, Entry 3) provide a sufficient amount of effective drug to induce cell death. The comprehensive analysis indicates that the moiety-drug conjugate of the present disclosure can achieve a higher DAR value (approximately 8), thus significantly expanding its application range and efficacy for HER2-expressing tumors.

These research results show that the newly developed linker-drug combinations of the present disclosure exhibit higher stability and safety. The linker design significantly reduces the cytotoxicity of the drug (P204, Eribulin). Furthermore, when the linker-drug combination is used in moiety-drug conjugates, it significantly enhances the therapeutic efficacy against target genes.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

All references cited herein are incorporated herein by reference to the full extent allowed by law. The discussion of those references is intended merely to summarize the assertions made by their authors. No admission is made that any reference (or a portion of any reference) is relevant prior art. Applicants reserve the right to challenge the accuracy and pertinence of any cited reference

## Claims

1. A compound as represented by Formula (I), 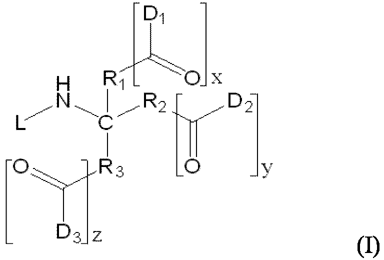 wherein,
L is a linker;
each of D₁, D₂, and D₃ independently is a drug;
each of x, y, and z independently is 0 or 1, with a provision that at least one of x, y, and z is 1; each of R₁, R₂, and R₃ independently is a bond, hydrogen, 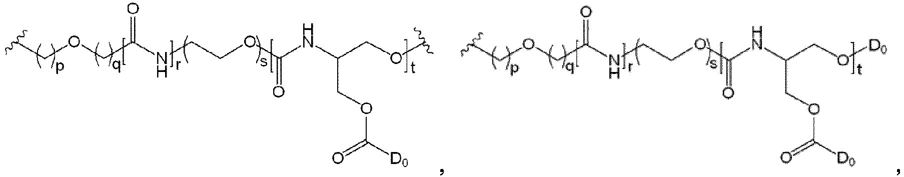 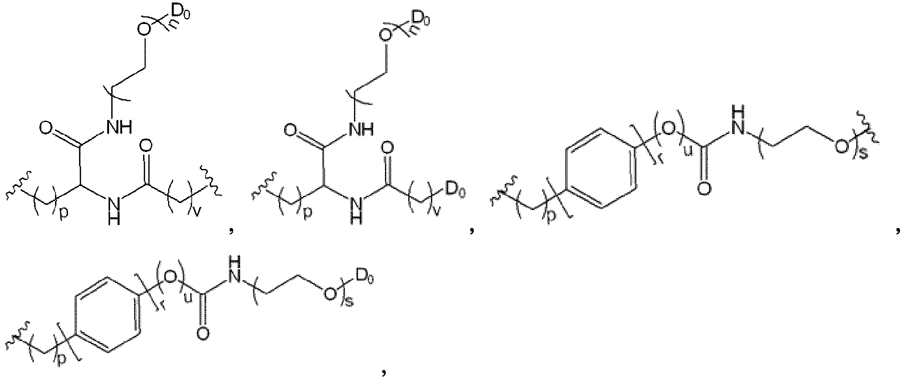 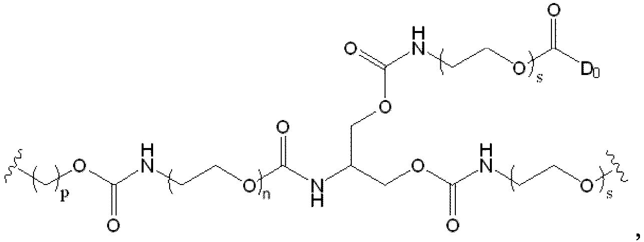 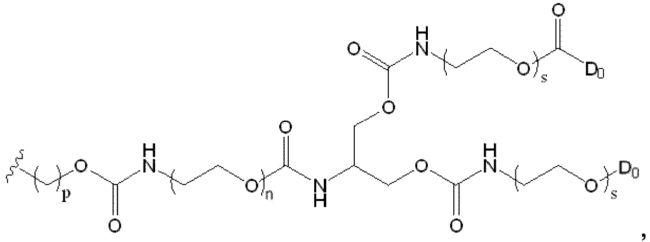 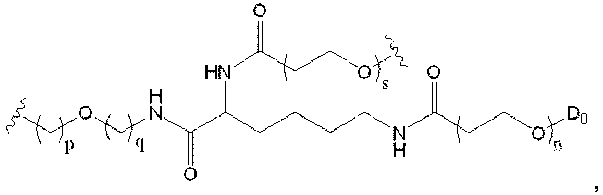
or 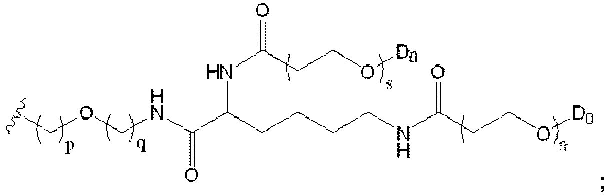 wherein p is 1, 2, or 3; q is 0, 1, or 2;
r is 0 or 1; s is an integer between 0 and 37; t is 0 or 1; u is 0 or 1; v is 1, 2, or 3; and n is an integer between 1 and 37; or
x and y are 0 and R₁ and R₂ are together to form =O and R₃ is 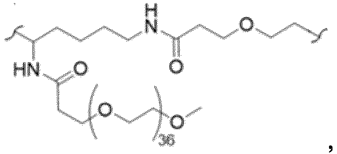
D₀ is methyl, propargyl, or a drug;
with a provision that at least one of R₁, R₂, and R₃ is not hydrogen;
if q, r, s, and t are 0, x+y+z>1;
if x is 0, R₁ is hydrogen, 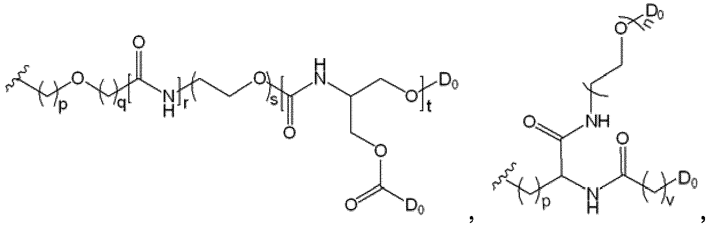 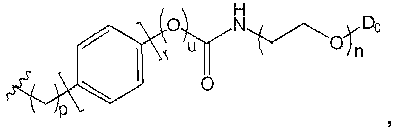 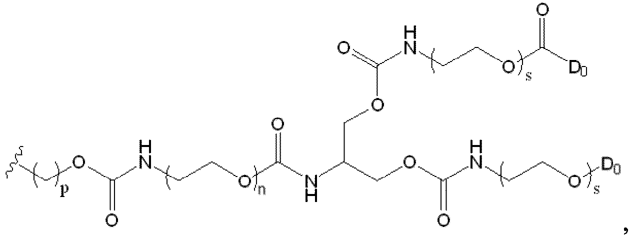
or 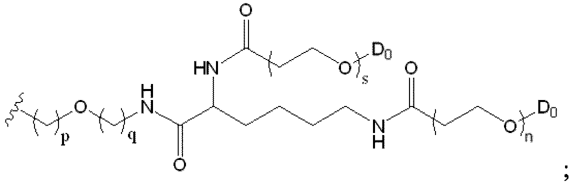
if y is 0, R₂ is hydrogen, 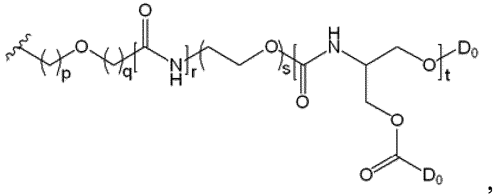 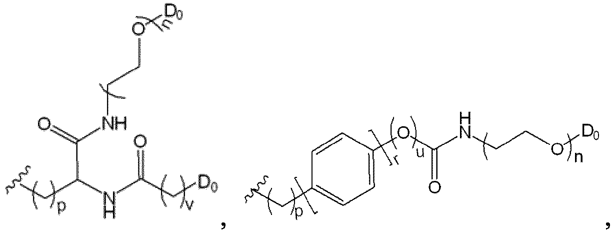 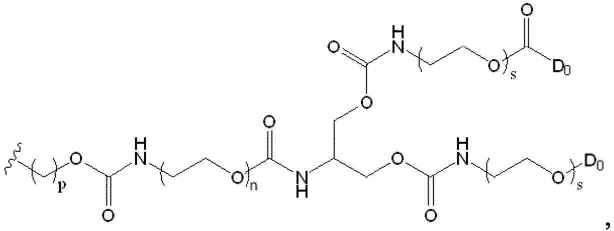 or 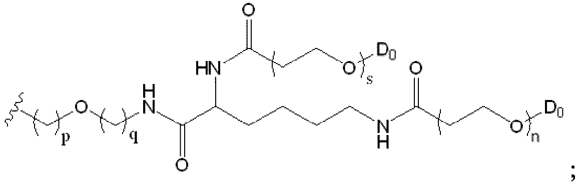
if z is 0, R₃ is hydrogen, 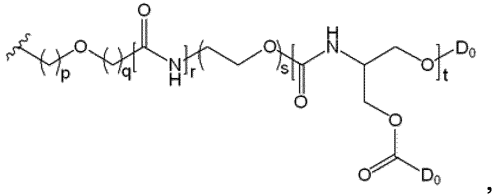 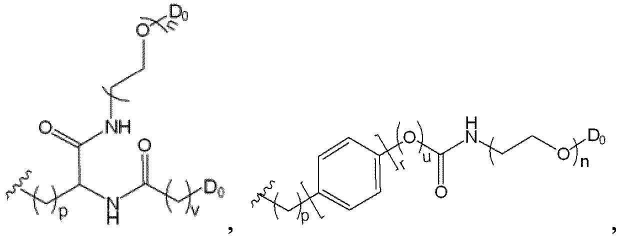 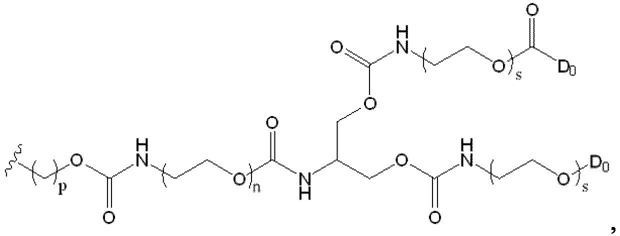 or 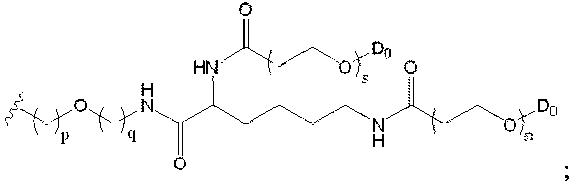
if x+y+z=1 and each of R₁, R₂, and R₃ independently is 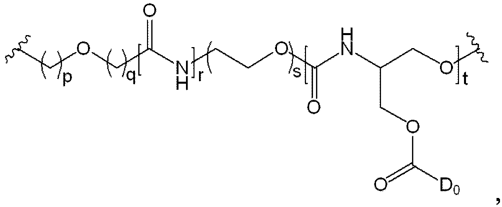 p is not 1;
if r, s, and t are 0 and each of R₁, R₂, and R₃ independently is 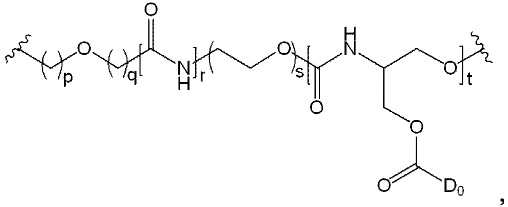 x+y+z>1.

2. The compound of claim 1, wherein L is selected from the group consisting of 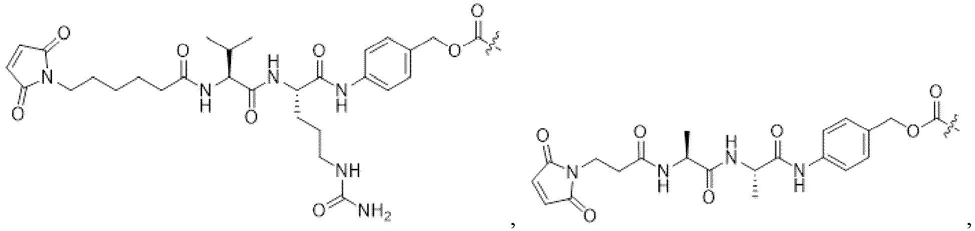 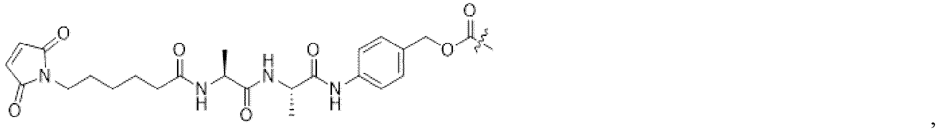 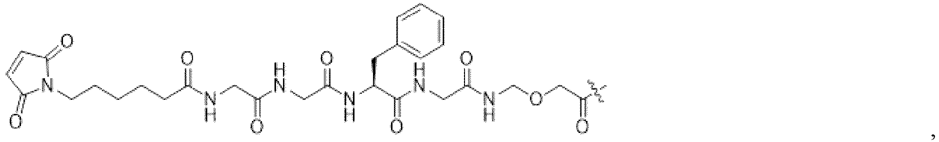 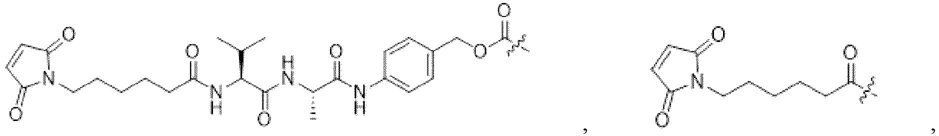 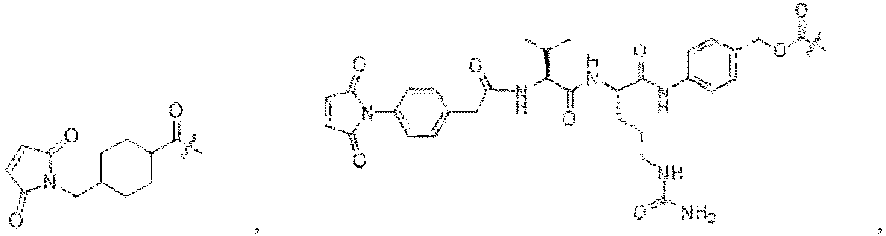 and

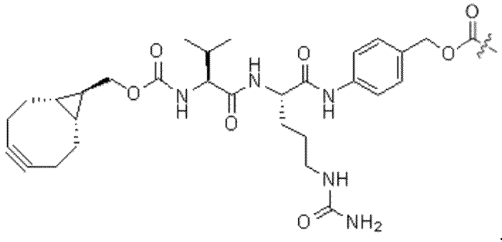

3. The compound of claim 1, wherein each of R₁, R₂, and R₃ independently is a bond, hydrogen, 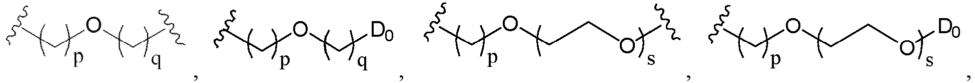 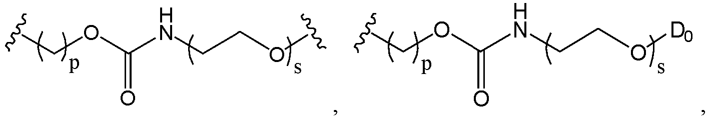 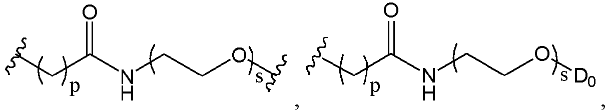 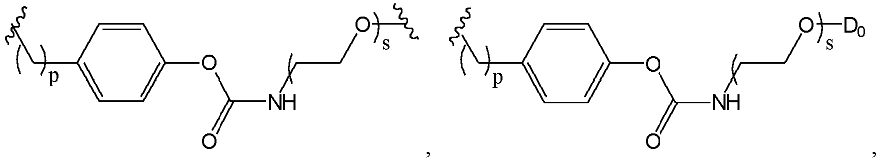 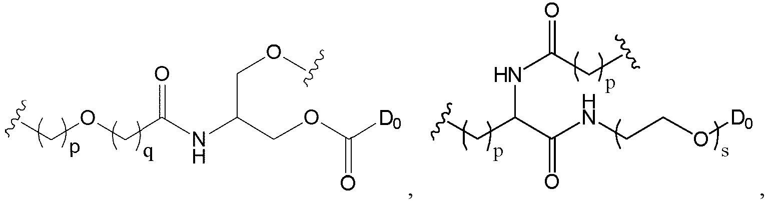 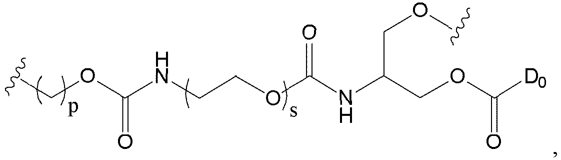 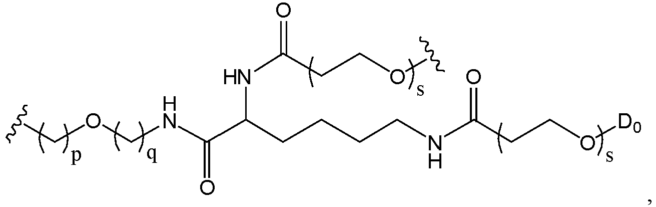 or

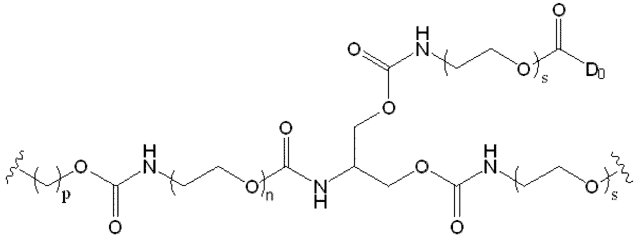

4. The compound of claim 1, wherein x+y+z=1.

5. The compound of claim 4, wherein R₁ is 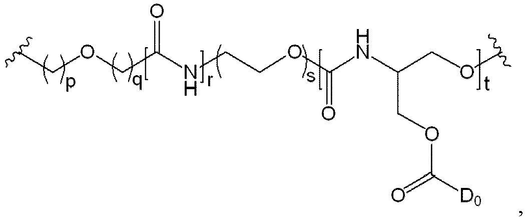 and both of R₂ and R₃ are H.

6. The compound of claim 5, wherein each of D₁, D₂, and D₃ independently is MMAE, MMAF, Exatecan, or Eribulin.

7. The compound of claim 4, wherein R₁ is a bond; R₂ is and R₃ is H.

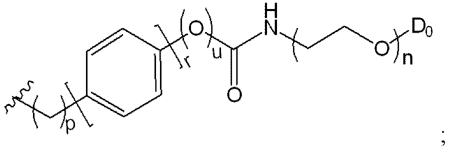

8. The compound of claim 4, wherein both of R₂ and R₃ are H; and R₁ is

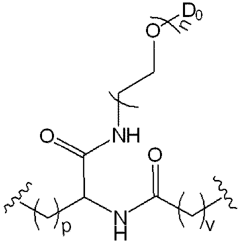

9. The compound of claim 4, wherein both of R₂ and R₃ are H; and R₁ is

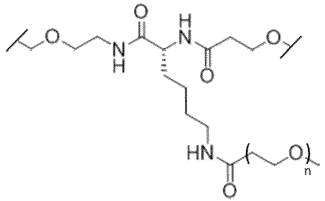

10. The compound of claim 1, whereinx+y+z>1.

11. The compound of claim 10, wherein x+y+z=2.

12. The compound of claim 11, wherein both of R₁ and R₂ are 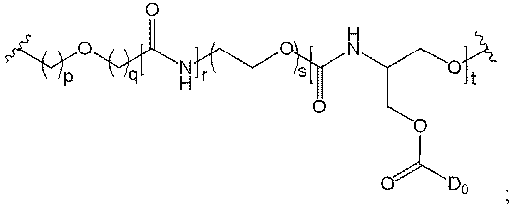 and R₃ is H.

13. The compound of claim 10, wherein x+y+z=3.

14. The compound of claim 13, wherein all of R₁, R₂, and R₃ are

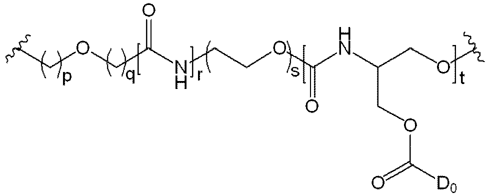

15. The compound of claim 1, selected from the group consisting of: 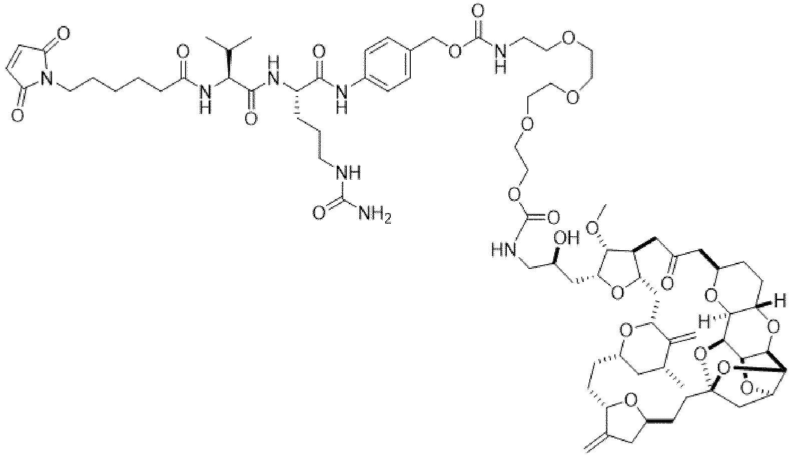 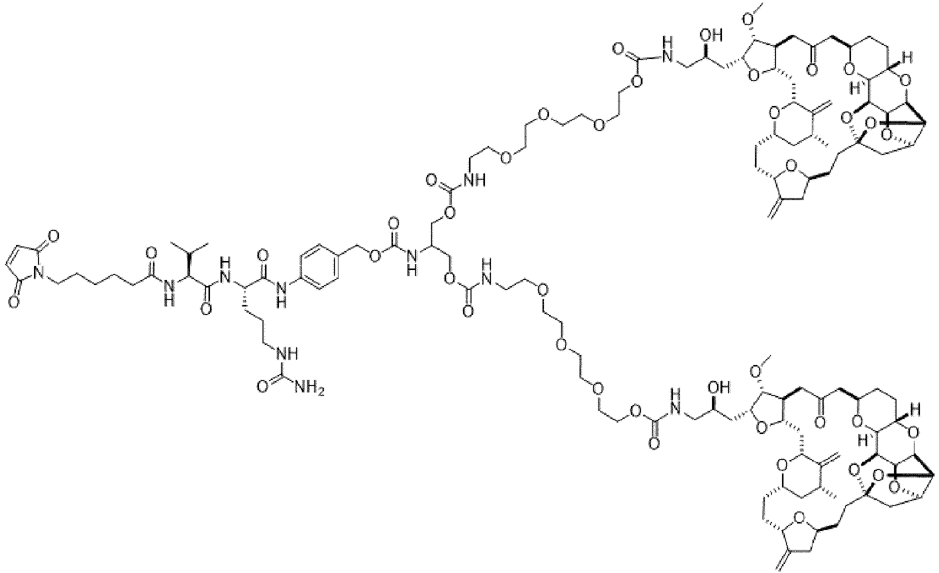 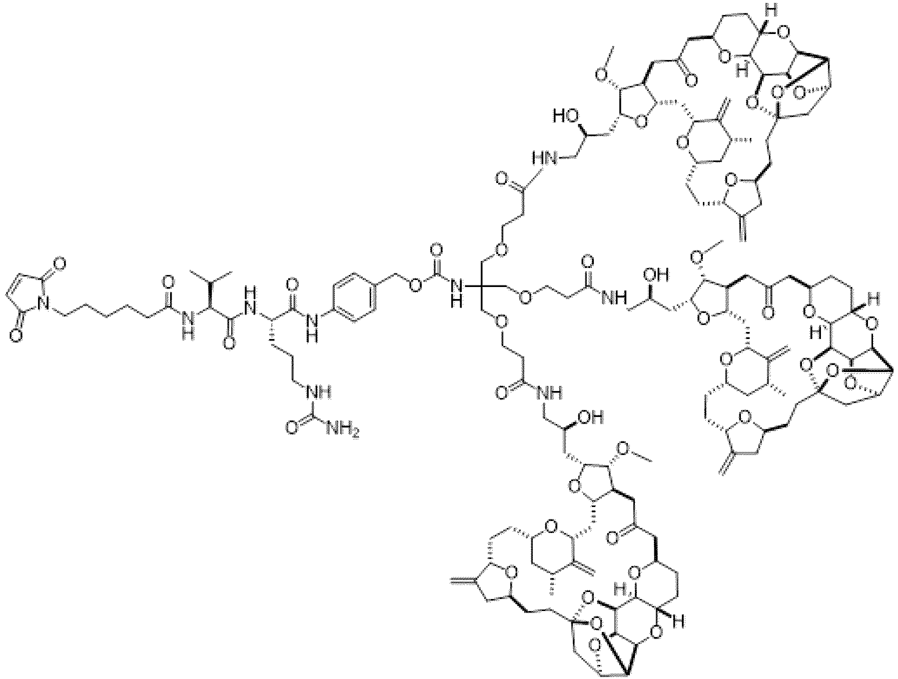 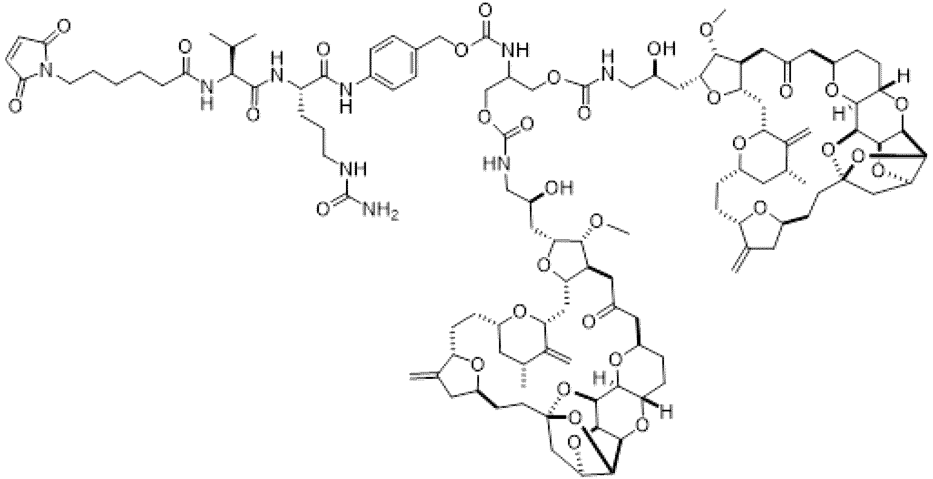 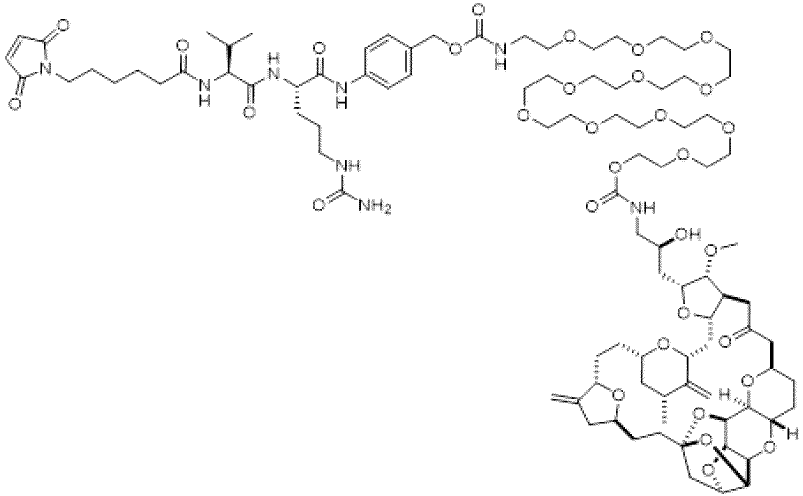 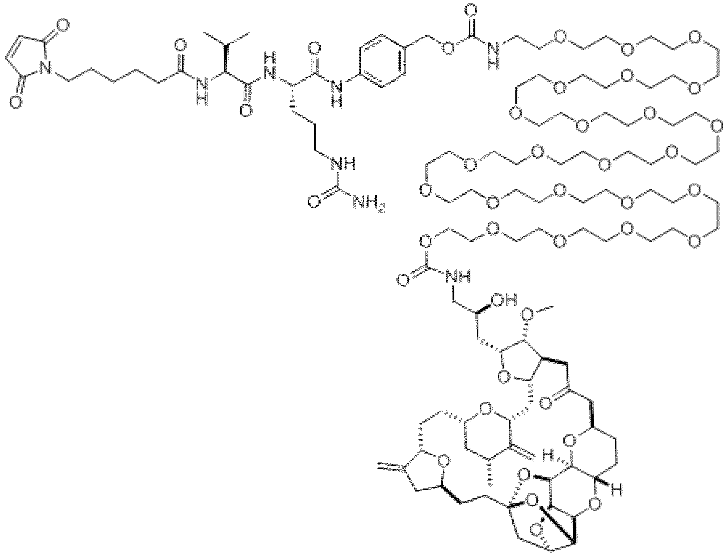 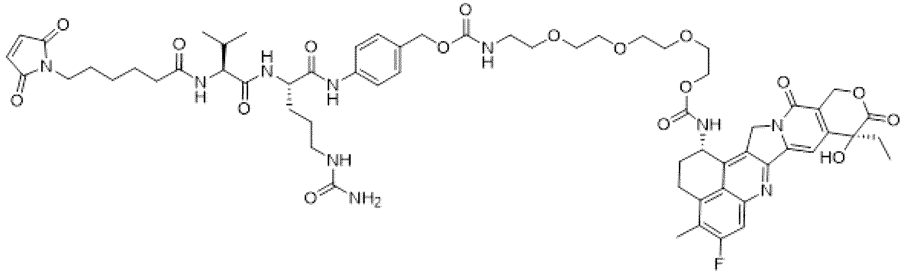 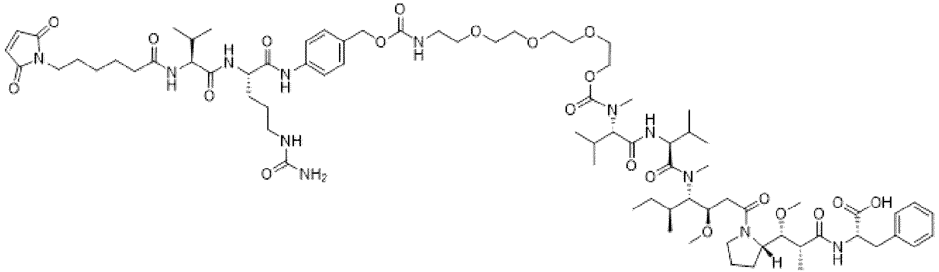 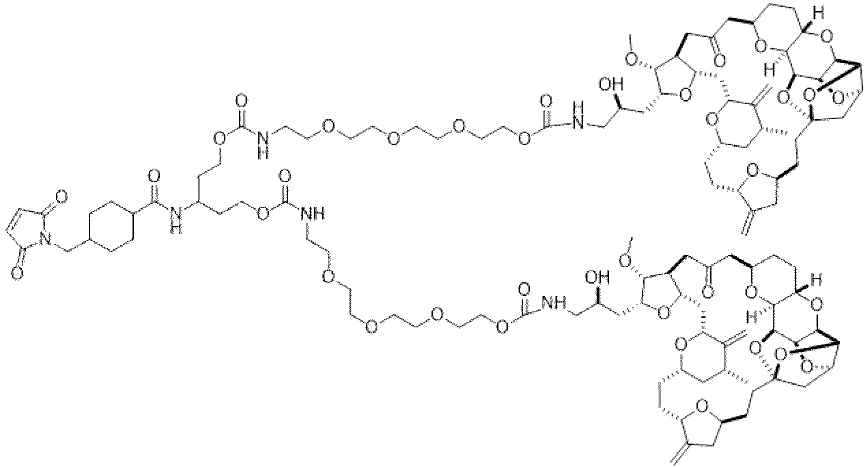 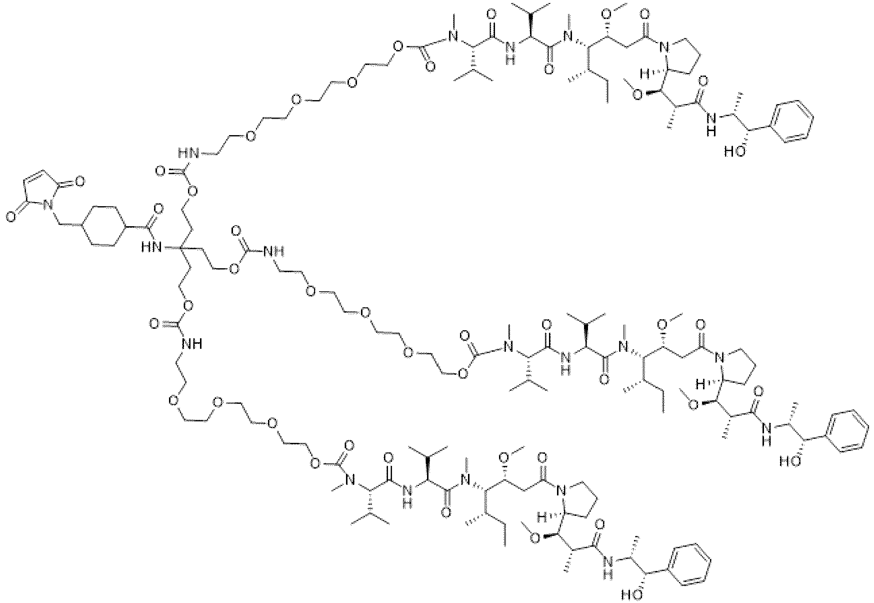 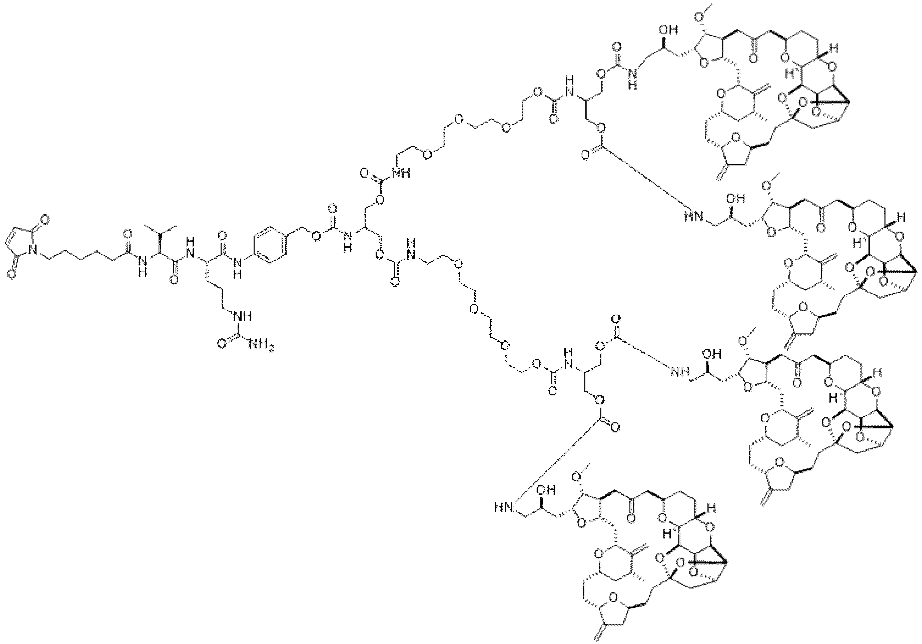 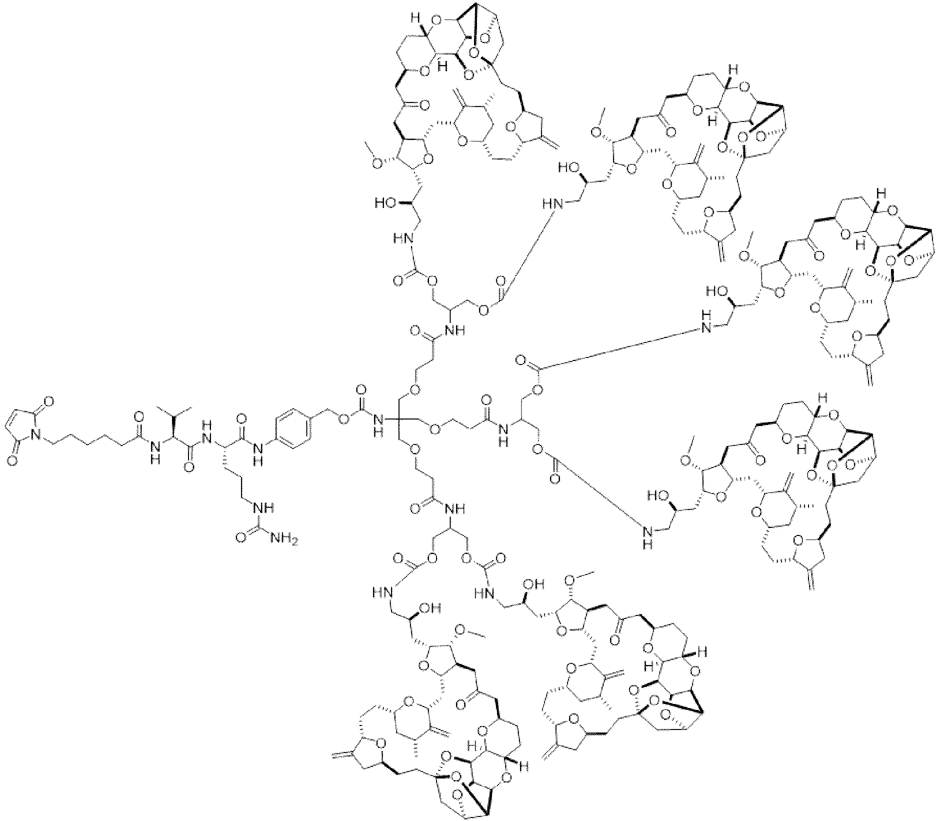 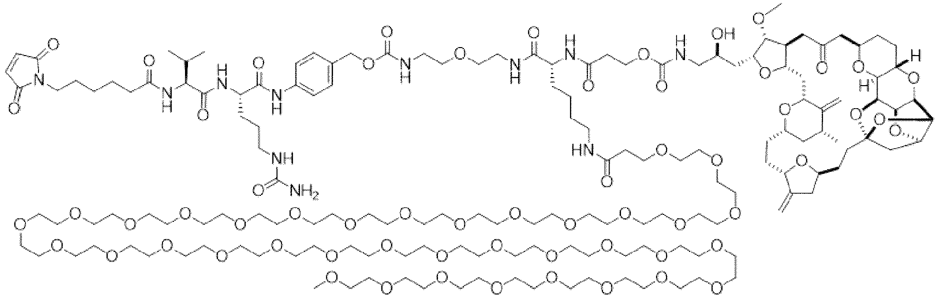 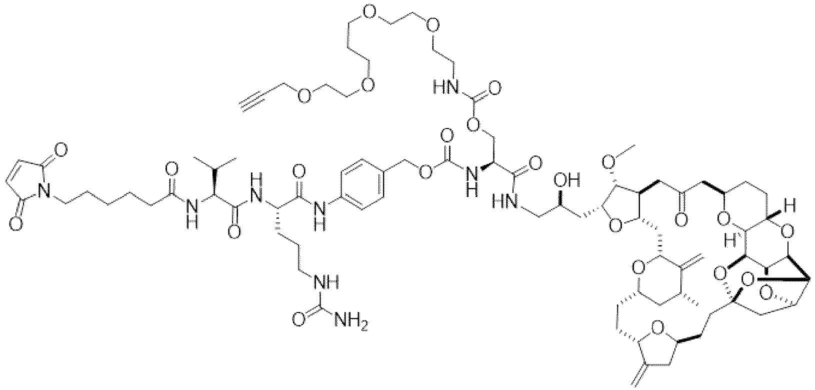 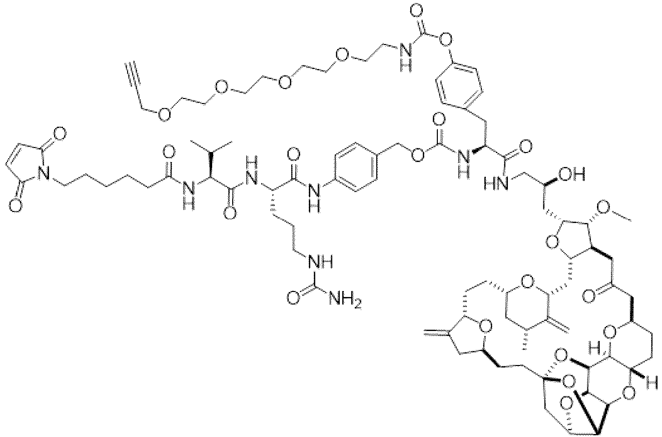 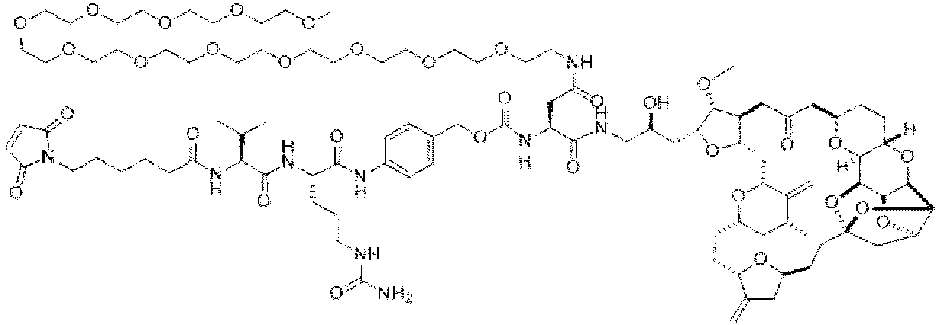 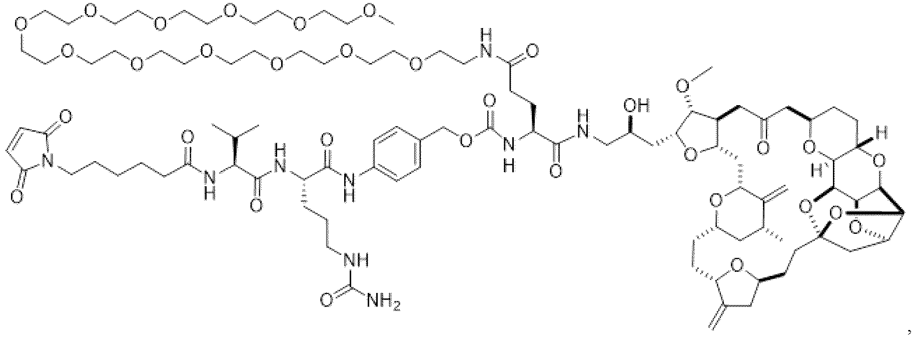 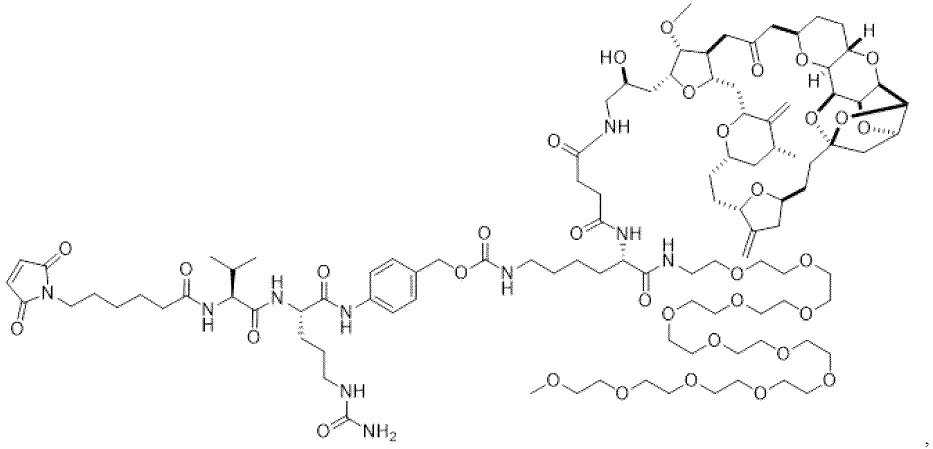 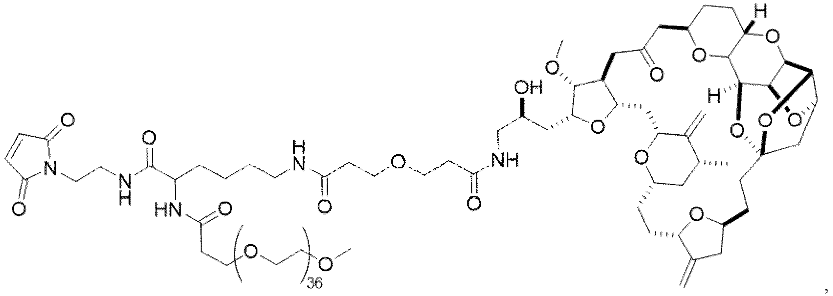 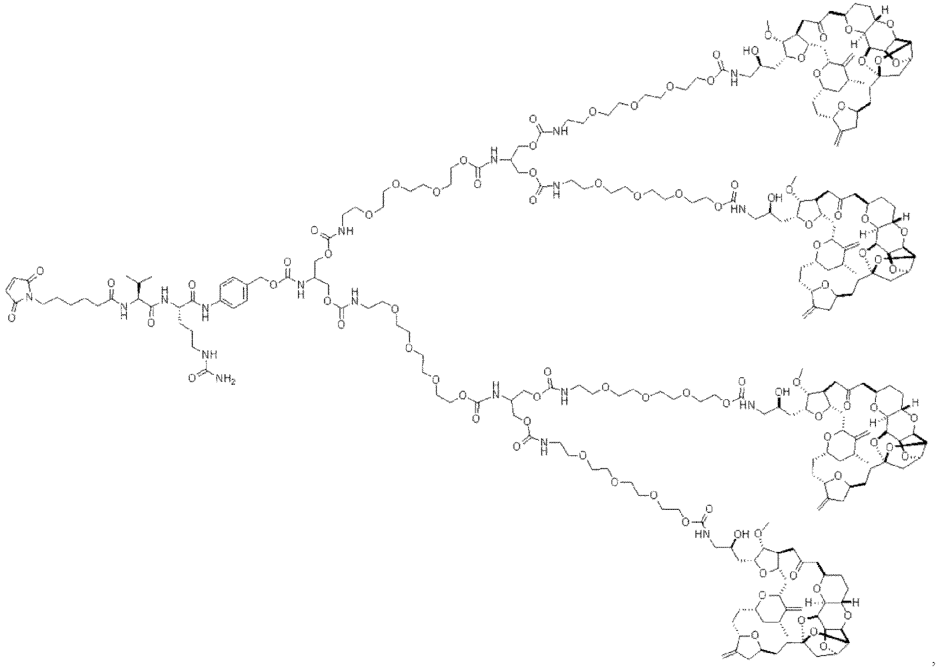 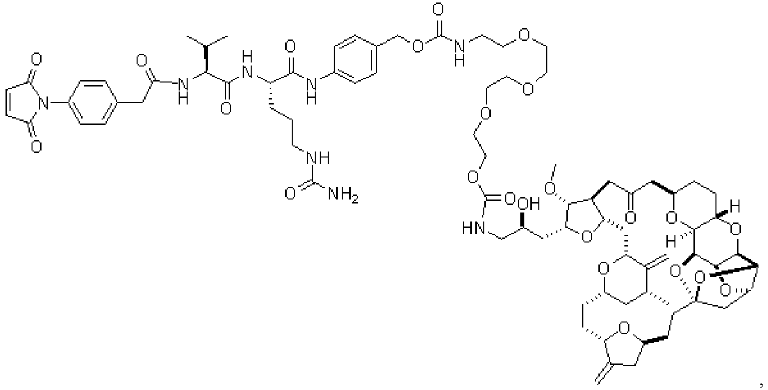 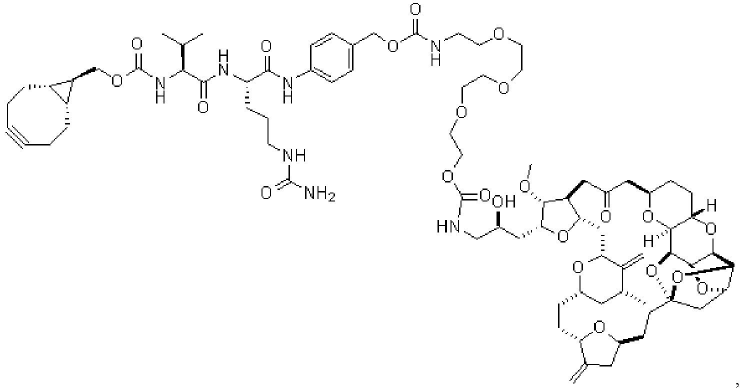 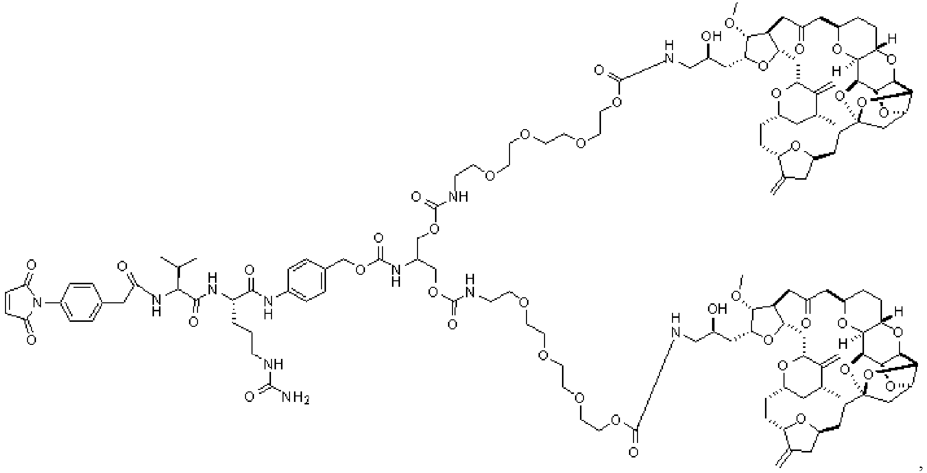 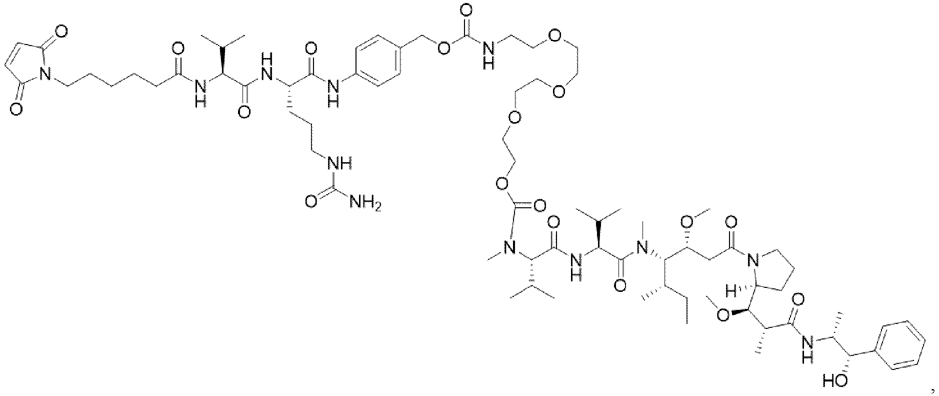 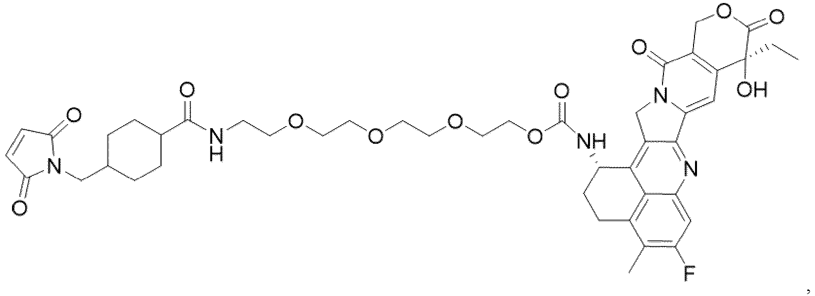 and

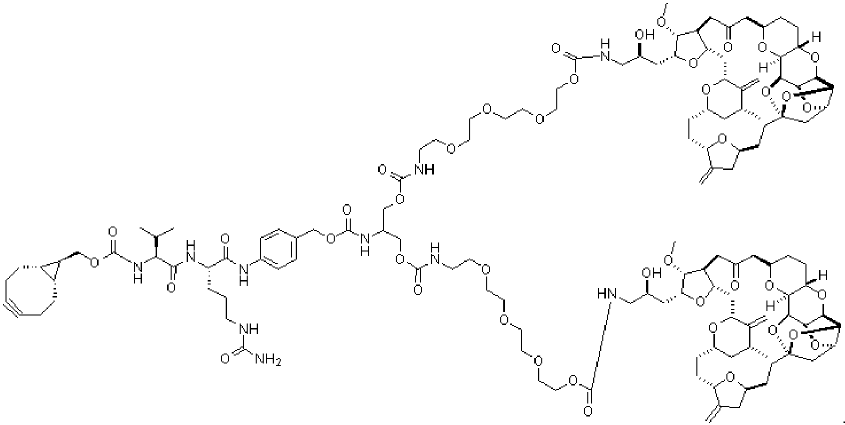

16. A conjugate comprising as represented by Formula (II), wherein 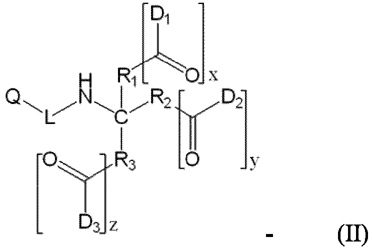
Q is a moiety selected from the group of a peptide, a polypeptide and a protein that specifically binds to an antigen;
L, D₁, D₂, D₃, x, y, z, R₁, R₂, and R₃ are as defined in claim 1.

17. The conjugate of claim 16, wherein L is conjugated to Q via cysteine residues on Q.

18. The conjugate of claim 16, wherein Q is an antibody selected from a monoclonal antibody, an antigen-binding fragment, a chimeric antibody, and a humanized antibody.

19. The conjugate of claim 18, wherein the antigen-binding fragment is an Fab, F(ab')₂, Fv or scFv fragment.

20. A pharmaceutical composition comprising the conjugate of claim 16, or a pharmaceutically acceptable salt thereof; and a pharmaceutically acceptable diluent, carrier or excipient.
